(19) Europäisches Patentamt
European Patent Office
Office européen des brevets

(11) Veröffentlichungsnummer : **0 254 866 B1**

(12) # EUROPÄISCHE PATENTSCHRIFT

(45) Veröffentlichungstag der Patentschrift : **13.03.91 Patentblatt 91/11**

(21) Anmeldenummer : **87108948.8**

(22) Anmeldetag : **23.06.87**

(51) Int. Cl.$^5$ : **C07D 403/12**, C07D 405/12, C07D 409/12, C07D 413/12, C07D 417/12, A01N 43/42, A01N 43/56, A01N 43/647, A01N 43/74, A01N 43/80, A01N 43/82

(54) **Chinolinderivate, Verfahren zu ihrer Herstellung, diese enthaltende Mikrobizide und ihre Verwendung zur Bekämpfung von Bakterien und Pilzen.**

Die Akte enthält technische Angaben, die nach dem Eingang der Anmeldung eingereicht wurden und die nicht in dieser Patentschrift enthalten sind.

(30) Priorität : **27.06.86 DE 3621540**

(43) Veröffentlichungstag der Anmeldung : **03.02.88 Patentblatt 88/05**

(45) Bekanntmachung des Hinweises auf die Patenterteilung : **13.03.91 Patentblatt 91/11**

(84) Benannte Vertragsstaaten : **AT BE CH DE ES FR GB GR IT LI NL SE**

(56) Entgegenhaltungen :
**DE-A- 3 225 169**
**DE-C- 2 005 959**
**FR-E- 95 521**
**GB-A- 1 141 697**
**GB-A- 1 148 405**
**ZA-A- 6 804 576**

(56) Entgegenhaltungen :
**Chemical Abstracts, Bd. 82, Nr. 9, 3 März 1975, Columbus, Ohio, USA;Massarani, E.; Nardi, D.; Tajana, A.; Leonardi, A.; Degen, L. "7-Nitro-8-acyloxyquinolines with antimicrobial and antifungal activity"; Seite 599, Spalte 1, Zusammenfassung Nr. 57538d**
**Chemical Abstracts, Bd. 79, Nr. 5, 6 August 1973, Columbus, Ohio, USA; Kharizanova, T.; Simova, V. "Antibacterial and antimycotic action of 8-hydroxyquinolines synthesized at the Bulgarian Scientific-Research Chemico-pharmaceutical Institute"; Seite 89, Spalte 2, Zusammenfassung Nr. 27787e**

(73) Patentinhaber : **BASF Aktiengesellschaft**
**Carl-Bosch-Strasse 38**
**W-6700 Ludwigshafen (DE)**

(72) Erfinder : **Hamprecht, Gerhard, Dr.**
**Rote-Turm-Strasse 28**
**W-6940 Weinheim (DE)**
Erfinder : **Theobald, Hans, Dr.**
**Queichstrasse 6**
**W-6703 Limburgerhof (DE)**
Erfinder : **Spiegler, Wolfgang, Dr.**
**Westpreussenstrasse 5**
**W-6520 Worms 27 (DE)**
Erfinder : **Richarz, Winfried, Dr.**
**Koenigsberger Strasse 5**
**W-6081 Stockstadt (DE)**
Erfinder : **Ammermann, Eberhard, Dr.**
**Sachsenstrasse 3**
**W-6700 Ludwigshafen (DE)**
Erfinder : **Pommer, Ernst-Heinrich, Dr.**
**Berliner Platz 7**
**W-6703 Limburgerhof (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 254 866 B1

## Beschreibung

Die Erfindung betrifft neue Chinolinderivate, Verfahren zu ihrer Herstellung, Mikrobizide, die diese Verbindungen als Wirkstoffe enthalten, sowie ihre Anwendung zur Bekämpfung von Bakterien und Pilzen.

Aus den Druckschriften DE-A 3 225 169, DE-A 2 005 959, GB 1 141 697, GB 1 148 405 und EP-A 98 486 sind 8-α-Furoyloxy-, 8-α-Thenoyloxy- und – im Falle der DE-A 3 225 169 und der EP-A 98 486 – 8-Azolylcarbonyloxy-Chinoline vom Typ der Verbindungen I bekannt.

Desweiteren werden in der FR 95 521 5-Methyl-7-halogen-8-α-furoyloxy- und in der S. African 68 04 576 5-Nitro-8-α-furoyloxychinoline beschrieben.

Aus Arzneim.-Forsch. 24, 1545 (1974) sind das 7-Nitro-8-α-furoyloxy- und das 7-Nitro-8-α-thenoyloxychinolin bekannt.

In den genannten Druckschriften wird auch die Verwendung der Verbindungen zur Bekämpfung von Pilzen und z.T. von Bakterien beschrieben.

Ferner sind aus der Literatur Tr. Nauchnoizsled. Khim.-Farm. Inst. 7, 423 (1972) dihalogenierte 8-Hydroxychinoline mit mikrobizider Wirkung bekannt.

Die genannten Verbindungen können jedoch hinsichtlich ihrer fungiziden und mikrobiziden Wirkungen nicht immer voll befriedigen.

Der Erfindung lag die Aufgabe zugrunde, nach mikrobiziden Verbindungen zu suchen, die den bekannten in dieser Hinsicht überlegen sind.

Es wurde nun gefunden, daß substituierte Chinolinderivate der Formel I

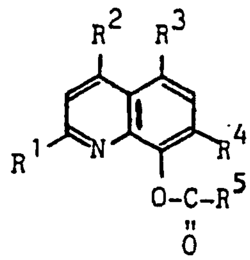

I,

in der

$R^1$ Wasserstoff oder Methyl,
$R^2$ Wasserstoff oder Halogen,
$R^3$ Wasserstoff, Halogen oder Nitro,
$R^4$ Wasserstoff, Halogen oder Nitro und
$R^5$ einen gegebenenfalls durch einen $C_1$-$C_4$-Alkylrest, einen $C_1$-$C_4$-Halogenalkylrest, eine Nitrogruppe oder ein Halogenatom einfach oder unabhängig voneinander zweifach substituierten Thiophen-, Pyrrol-, Oxazol-, Thiazol-, Imidazol-, Isoxazol-, Isothiazol-, Pyrazol-, 1,2,3-, 1,2,4-, 1,2,5- oder 1,3,4-Thiadiazol-, 1,2,3-, 1,2,4-, 1,2,5- oder 1,3,4-Oxadiazol-, 1,2,3- oder 1,2,4-Triazol- oder einen halogen- oder mono- oder dimethylsubstituierten Furan-Rest bedeuten, mit der Maßgabe, daß
$R^3$ und $R^4$ nicht gleichzeitig für Wasserstoff oder Halogen stehen und das 5- Chlor-, das 2-Methyl- und das 5-Nitro-8-(1′,2′,3′-thiadiazol-4′-carbonyloxy)- -chinolin, das 5-Chlor-8-(4′-methyl-1′,2′,3′-thiadiazol-5′-carbonyloxy)- -chinolin, das 7-Nitro-8-(alpha-thienoyl-oxy)-chinolin, das 5-Nitro-8-(2′-halogenfuroyl-5′-oxy)-chinolin und das 2-Methyl-8-(isoxazol-5′-carbonyl-oxy)-chinolin ausgeschlossen bleiben, ausgezeichnet mikrobizid wirksam sind.

Der Heterocyclus $R^5$ kann an jeder möglichen Stelle mit dem restlichen Molekül verknüpft sein. $R^5$ bedeutet einen ggf. durch einen $C_1$-$C_4$-Halogenalkylrest, eine Nitrogruppe oder ein Halogenatom einfach oder unabhängig voneinander zweifach substituierten Thiophen-(2)- oder -(3)-Rest, Pyrrol-(1)-, -(2)- oder -(3)-Rest, Oxazol-(2)-, -(4)- oder -(5)-Rest, Thiazol-(2)-, -(4)- oder -(5)-Rest, Imidazol-(1)-, -(2)-, -(4)- oder -(5)-Rest, Isoxazol-(3)-, -(4)- oder -(5)-Rest, Isothiazol-(3)-, -(4)oder -(5)-Rest, Pyrazol-(1)-, -(3)-, -(4)- oder -(5)-Rest, 1,3,4-Thiadiazol-(2)-Rest, 1,3,4-Oxadiazol-(2)-Rest, 1,2,4-Thiadiazol-(3)- oder -(5)-Rest, 1,2,4-Oxadiazol-(3)- oder -(5)-Rest, 1,2,4-Triazol-(1)-, -(3)oder -(5)-Rest, 1,2,3-Thiadiazol-(4)- oder -(5)-Rest, 1,2,3-Oxadiazol-(4)- oder -(5)-Rest, 1,2,3-Triazol-(1)-, -(4)- oder -(5)-Rest, 1,2,5-Thiadiazol-(3)-Rest oder 1,2,5-Oxadiazol-(3)-Rest, oder einen halogen- oder mono- oder dimethylsubstituierten Furan-(2)- oder -(3)-Rest. Halogen bedeutet vorzugsweise Chlor oder Brom.

Bevorzugt werden Verbindungen der Formel I, in denen $R^1$ Wasserstoff, $R^2$
Wasserstoff oder Halogen, $R^3$ Wasserstoff, Halogen oder Nitro, $R^4$ Wasserstoff, Halogen oder Nitro und

$R^5$ einen gegebenenfalls durch Chlormethyl substituierten Isoxazol-(3)- oder -(5)-Rest, einen gegebenenfalls durch Methyl substituierten Oxazol-(5)-Rest, einen Imidazol-(1)-Rest oder einen gegebenenfalls durch Methyl substituierten 1,2,3-Thiadiazol-(4)- oder -(5)-Rest bedeutet.

Die Herstellung der Verbindungen der Formel I erfolgt beispielsweise dadurch, daß man eine Verbindung der Formel II

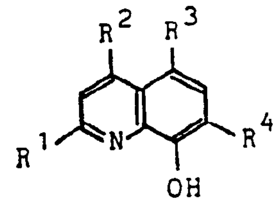

II,

oder eines ihrer Alkalimetall- oder Erdalkalimetallsalze mit einem Carbonsäurederivat der Formel III

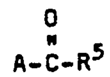

III,

in der

A für eine nucleophil verdrängbare Abgangsgruppe steht, umsetzt.

In der Formel III bedeutet A beispielsweise Halogen, wie Chlor oder Brom, einen Alkoxycarbonyloxyrest, wie Methoxycarbonyloxy und Ethoxycarbonyloxy, den Benzyloxycarbonyloxyrest, einen Azolylrest, wie den Imidazolyl- oder den Triazolylrest oder einen Alkoxyrest wie den methoxy- oder Ethoxyrest.

Nach einer Variante können die Chinolinderivate der Formel I, in denen $R^5$ einen 1-Azolylrest darstellt, auch dadurch erhalten werden, daß man eine Verbindung der Formel II, mit einem Carbonyl-bisazol der Formel

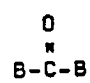

IV,

in der B für die in Anspruch 1 für $R^5$ genannten 1-Azolreste steht, umsetzt.

Für den Fall der Verwendung des Carbonyl-bisimidazols und des 8-Hydroxychinolins läßt sich die Umsetzung durch folgendes Schema wiedergeben :

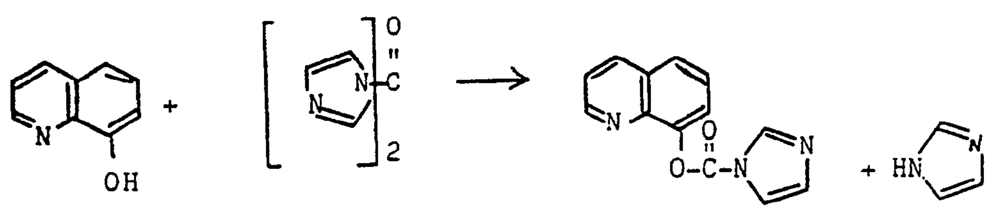

Obwohl die Reaktion auch in Abwesenheit von Lösungsmitteln durchgeführt werden kann, ist es zweckmäßig, die Umsetzung in einem inerten Lösungs- oder Verdünnungsmittel vorzunehmen. Als Lösungsmittel kommen z.B. in Frage :

Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Iodbenzol, o-, m-, p-Dichlorbenzol, o-, p-, m-Dibrombenzol, o-, m-, p-Chlortoluol, 1,2,4-Trichlorbenzol ; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β"-Dichlordiethylether ; Nitrokohlenwasserstoffe, nie Nitromethan, Nitroethan, Nitrobenzol, o-, m-, p-Chlornitrobenzol, o-Nitrotoluol ; Nitrile wie Acetonitril, Butyronitril, Isobutyronitril, Benzonitril, m-Chlorbenzonitril ; aliphatische oder cycloaliphatische

Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethyl pentan, Octan ; Ester, z.B. Ethylacetat, Acetessigester, Isobutylacetat ; Amide, z.B. Formamid, Methylformamid, Dimethylformamid ; Ketone, z.B. Aceton, Methylethylketon, gegebenenfalls auch Wasser und entsprechende Gemische. Zweckmäßig verwendet man das Lösungsmittel in einer Menge von 100 bis 2.000 Gew.%, vorzugsweise von 200 bis 900 Gew.%, bezogen auf Ausgangsstoff II.

Falls A Halogen ist, empfiehlt es sich – obwohl es nicht notwendig ist –, die Umsetzung in Gegenwart eines Säureakzeptors durchzuführen. Als solche können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Als basische verbindungen kommen z.B. in Frage : Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Bariumoxid, Magnesiumhydroxid, Magnesiumoxid, Bariumhydroxid, Calciumcarbonat, Magnesiumcarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-methylpyrrolidin, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, alpha-Picolin, gamma-Picolin, Isochinolin, Pyrinidin, Acridin, N,N,N",N"-Tetramethylethylendiamin, N,N,N",N"-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Man kann jedoch auch den bei der Reaktion beispielsweise entstehenden Halogenwasserstoff durch Einleiten eines Inertgases, beispielsweise Stickstoff, entfernen.

Zweckmäßig wird das Verfahren zur Herstellung der neuen Verbindungen so durchgeführt, daß man den Ausgangsstoff II, gegebenenfalls in einem der vorgenannten Verdünnungsmittel, vorlegt und dann den Ausgangsstoff III oder IV und gegebenenfalls einen Säureakzeptor gleichzeitig oder nacheinander zugibt. Man kenn jedoch auch den Ausgangsstoff III oder IV in einen der vorgenannten Verdünnungsmittel vorlegen und dann den Ausgangsstoff II und gegebenenfalls einen Säureakzeptor, gleichzeitig oder in beliebiger Reihenfolge, über zwei getrennte Zuführungen zugeben.

Die Umsetzung ist in vielen Fällen nach der Zugabe der Komponenten bereits abgeschlossen, andernfalls rührt man zu ihrer Beendigung noch 10 Minuten bis 14 Stunden bei –10 bis 120°C, vorzugsweise 0 bis 100°C, insbesondere 20 bis 80°C nach.

Aus dem Reaktionsgemisch wird die Verbindung I in üblicher Weise, z.B. durch Abdestillieren von Lösungsmittel oder überschüssigem Ausgangsstoff III oder direkt durch Absaugen isoliert. Der verbleibende Rückstand wird in diesem Fall zur Entfernung saurer oder basischer Verunreinigungen mit Wasser bzw. verdünnten Alkali oder Säure gewaschen und getrocknet. Im Fall von mit Wasser nicht mischbaren Verdünnungsmitteln kann man auch direkt das Reaktionsgemisch mit Wasser bzw. mit verdünnten Alkali oder Säure extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation weiter gereinigt werden.

Die in den Herstell- und Anwendungsbeispielen genannten Teile und Prozente beziehen sich auf des Gewicht.

Herstell-Beispiele :

Beispiel 1

Zu einer Mischung von 13,5 Teilen 7-Brom-5-nitro-8-oxychinolin in 135 Teilen Essigester wurden bei 15 bis 25°C unter Rühren innerhalb 5 Minuten 5,1 Teile Triethylamin gegeben und 5 minuten gerührt. Innerhalb 10 Minuten wurden dann 7,4 Teile 1,2,3-Thiadiazol-4-carbonsäurechlorid unter Rühren bei 20 bis 25°C zugeführt und 2 Stunden bei 50°C gerührt. Die Reaktionsmischung wurde im Vakuum zur Trockne eingedampft und der Rückstand in 400 Teilen 0,5 n Salzsäure während 5 Minuten digeriert. Das Ungelöste wurde abgesaugt, mit Wasser gewaschen und während 10 Minuten in 200 Teilen 1 n Natronlauge gerührt. Nach dem Absaugen, Waschen mit Wasser und Trocknen erhielt man 13 Teile 7-Brom-5-nitro-8-(1",2",3"-thiadiazol-4"-carbonyl)-oxychinolin vom Fp. 192 bis 194°C.

Beispiel 2

6,52 Teile 2-Methylpyridin wurden bei 25°C unter Rühren innerhalb 5 Minuten zu einer Mischung von 15,7 Teilen 7-Chlor-5-nitro-8-oxychinolin in 190 Teilen 1,2-Dichlorethan gegeben und 5 Minuten gerührt. Innerhalb 10 minuten wurden dann bei 20 bis 25°C 9,2 Teile Isoxazol-5-carbonsäurechlorid unter Rühren zugegeben und 3 Stunden bei 55°C gerührt. Die Reaktionsmischung wurde im Vakuum zur Trockne eingedampft und der Rückstand in 200 Teilen 0,5 n Salzsäure während 5 Minuten gerührt. Der Niederschlag wurde abgesaugt, mit Wasser gewaschen und in 200 Teilen verdünnter Natriumcarbonatlösung während 10 Minuten gerührt. Nach dem Absaugen, Waschen mit 60°C warmem Wasser und Trocknen erhielt man 17,5 Teile 7-Chlor-5-nitro-8-(isoxazol-5″-carbonyl)-oxychinolin vom Fp. 132 bis 134°C.

In analoger weise sind die folgenden Beispiele herstellbar :

| Nr. | $R^1$ | $R^2$ | $R^3$ | $R^4$ | $R^5$ | Fp. [°C] |
|---|---|---|---|---|---|---|
| 4 | H | H | H | Cl | Thienyl-2 | |
| 5 | H | H | H | Cl | Thienyl-3 | |
| 6 | H | H | H | Cl | 2-Chlor- thienyl-3 | |
| 7 | H | H | H | Cl | 5-Chlor- thienyl-2 | |
| 8 | H | H | H | Cl | 5-Nitro- thienyl-2 | |
| 9 | H | H | H | Cl | 4-Methyl-thienyl-2 | |
| 10 | H | H | H | Cl | 3-Methyl-thienyl-2 | |
| 11 | H | H | H | Cl | 2-Methyl-thienyl-3 | |
| 12 | H | H | H | Cl | 3-Methyl-thienyl-4 | |
| 13 | H | H | H | Cl | 2,5-Dimethyl-thienyl-4 | |
| 14 | H | H | H | Cl | 2,3-Dichlor-thienyl-4 | |
| 15 | H | H | H | Cl | 5-Methyl-thieñyl-2 | |
| 16 | H | H | H | Cl | Pyrryl-2 | |
| 17 | H | H | H | Cl | Pyrryl-3 | |
| 18 | H | H | H | Cl | 3-Methyl-pyrryl-2 | |
| 19 | H | H | H | Cl | 2-Methyl-pyrryl-3 | |
| 20 | H | H | H | Cl | 5-Chlor-pyrryl-2 | |
| 21 | H | H | H | Cl | Oxazolyl-2 | |
| 22 | H | H | H | Cl | Oxazolyl-4 | |
| 23 | H | H | H | Cl | Oxazolyl-5 | |
| 24 | H | H | H | Cl | 1,2,3-Thiadiazolyl-4 | 121-123 |
| 25 | H | H | H | Cl | 4-Methyl-oxazolyl-5 | |
| 26 | H | H | H | Cl | 2-Methyl-oxazolyl-5 | |
| 27 | H | H | H | Cl | Thiazolyl-2 | |
| 28 | H | H | H | Cl | Thiazolyl-4 | |
| 29 | H | H | H | Cl | Thiazolyl-5 | |
| 30 | H | H | H | Cl | 4-Methyl-thiazolyl-5 | |
| 31 | H | H | H | Cl | 2-Methyl-thiazolyl-5 | |
| 32 | H | H | H | Cl | Imidazolyl-4 | |
| 33 | H | H | H | Cl | Imidazolyl-5 | |
| 34 | H | H | H | Cl | 4-Methyl-imidazolyl-5 | |
| 35 | H | H | H | Cl | 1-Methyl-imidazolyl-5 | |
| 36 | H | H | H | Cl | 4-Nitro-imidazolyl-1 | |
| 37 | H | H | H | Cl | 2-Methyl-4-nitro-imidazolyl-1 | |
| 38 | H | H | H | Cl | 4,5-Dichlorimidazolyl-1 | |
| 39 | H | H | H | Cl | 1-Methyl-pyrryl-2 | |
| 40 | H | H | H | Cl | Isoxazolyl-3 | 93- 96 |
| 41 | H | H | H | Cl | Isoxazolyl-4 | |
| 42 | H | H | H | Cl | Isoxazolyl-5 | 101-104 |
| 43 | H | H | H | Cl | 5-Chlormethyl-isoxazolyl-3 | 151-152 |
| 44 | H | H | H | Cl | Isothiazolyl-3 | |
| 45 | H | H | H | Cl | Isothiazolyl-4 | |

| Nr. | $R^1$ | $R^2$ | $R^3$ | $R^4$ | $R^5$ | Fp. [°C] |
|---|---|---|---|---|---|---|
| 46 | H | H | H | Cl | Isothiazolyl-5 | |
| 47 | H | H | H | Cl | 4-Methyl-isothiazolyl-5 | |
| 48 | H | H | H | Cl | Pyrazolyl-4 | |
| 49 | H | H | H | Cl | Pyrazolyl-5 | |
| 50 | H | H | H | Cl | 4-Chlor-pyrazolyl-5 | |
| 51 | H | H | H | Cl | 1-Methyl-pyrazolyl-5 | |
| 52 | H | H | H | Cl | 1,2,3-Thiadiazolyl-5 | |
| 53 | H | H | H | Cl | 1,2,3-Oxadiazolyl-5 | |
| 54 | H | H | H | Cl | 1,2,3-Oxadiazolyl-4 | |
| 55 | H | H | H | Cl | 5-Methyl-1,2,3-oxadiazolyl-4 | |
| 56 | H | H | H | Cl | 4-Methyl-1,2,3-oxadiazolyl-5 | |
| 57 | H | H | H | Cl | 1,3,4-Thiadiazolyl-2 | |
| 58 | H | H | H | Cl | 2-Methyl-1,3,4-thiadiazolyl-5 | |
| 59 | H | H | H | Cl | 1,2,4-Thiadiazolyl-3 | |
| 60 | H | H | H | Cl | 1,2,4-Thiadiazolyl-5 | |
| 61 | H | H | H | Cl | 3-Methyl-1,2,4-thiadiazolyl-5 | |
| 62 | H | H | H | Cl | 1,2,4-Oxadiazolyl-3 | |
| 63 | H | H | H | Cl | 1,2,4-Oxadiazolyl-5 | |
| 64 | H | H | H | Cl | 1,2,4-Triazolyl-5 | |
| 65 | H | H | H | Cl | 1,2,4-Triazolyl-3 | |
| 66 | H | H | H | Cl | 1,3,4-Oxadiazolyl-5 | |
| 67 | H | H | H | Cl | 2-Methyl-1,3,4-oxadiazolyl-5 | |
| 68 | H | H | H | Cl | 2-Chlor-1,3,4-oxadiazolyl-5 | |
| 69 | H | H | H | Cl | 1,2,5-Oxadiazolyl-3 | |
| 70 | H | H | H | Cl | 3-Methyl-1,2,5-oxadiazolyl-4 | |
| 71 | H | H | H | Cl | 1,2,5-Thiadiazolyl-3 | |
| 72 | H | H | H | Cl | 3-Methyl-1,2,5-thiadiazolyl-4 | |
| 73 | H | H | H | Cl | 2-Methyl-furyl-5 | |
| 74 | H | H | H | Cl | 2,5-Dimethylfuryl-4 | 111-114 |
| 75 | H | H | H | Cl | 2-Chlor-furyl-5 | |
| 76 | H | H | H | Cl | 5-Methyl-oxazolyl-4 | |
| 77 | H | H | H | Cl | 4-Methyl-oxazolyl-2 | |
| 78 | H | H | H | Cl | 2-Bromfuryl-5 | |
| 79 | H | H | H | Cl | 2-Methyl-furyl-4 | |
| 80 | H | H | H | Cl | 5-Methyl-isoxazolyl-3 | |
| 81 | H | H | H | Cl | 4-Methyl-isoxazolyl-3 | |
| 82 | H | H | H | Cl | 5-Methyl-isothiazolyl-3 | |
| 83 | H | H | H | Cl | 4-Methyl-isothiazolyl-3 | |
| 84 | H | H | H | Cl | 5-Methyl-1,2,3-thiadiazolyl-4 | |
| 85 | H | H | H | Cl | 4-Methyl-1,2,3-thiadiazolyl-5 | 162-164 |
| 86 | H | H | H | Cl | Pyrryl-1 | |
| 87 | H | H | H | Cl | Imidazolyl-1 | |

| Nr. | $R^1$ | $R^2$ | $R^3$ | $R^4$ | $R^5$ | Fp. [°C] |
|---|---|---|---|---|---|---|
| 88 | H | H | H | Cl | Pyrazolyl-1 | |
| 89 | H | H | H | Cl | 1,3,4-Triazolyl-1 | |
| 90 | H | H | H | Cl | 1,2,4-Triazolyl-1 | |
| 91 | H | H | H | Br | Thienyl-2 | |
| 92 | H | H | H | Br | Thienyl-3 | |
| 93 | H | H | H | Br | 2-Chlor- thienyl-3 | |
| 94 | H | H | H | Br | 5-Chlor- thienyl-2 | |
| 95 | H | H | H | Br | 5-Nitro- thienyl-2 | |
| 96 | H | H | H | Br | 4-Methyl-thienyl-2 | |
| 97 | H | H | H | Br | 3-Methyl-thienyl-2 | |
| 98 | H | H | H | Br | 2-Methyl-thienyl-3 | |
| 99 | H | H | H | Br | 3-Methyl-thienyl-4 | |
| 100 | H | H | H | Br | 2,5-Dimethyl-thienyl-4 | |
| 101 | H | H | H | Br | 2,3-Dichlor-thienyl-4 | |
| 102 | H | H | H | Br | 5-Methyl-thienyl-2 | |
| 103 | H | H | H | Br | Pyrryl-2 | |
| 104 | H | H | H | Br | Pyrryl-3 | |
| 105 | H | H | H | Br | 3-Methyl-pyrryl-2 | |
| 106 | H | H | H | Br | 2-Methyl-pyrryl-3 | |
| 107 | H | H | H | Br | 5-Chlor-pyrryl-2 | |
| 108 | H | H | H | Br | Oxazolyl-2 | |
| 109 | H | H | H | Br | Oxazolyl-4 | |
| 110 | H | H | H | Br | Oxazolyl-5 | |
| 111 | H | H | H | Br | 1,2,3-Thiadiazolyl-4 | 127-130 |
| 112 | H | H | H | Br | 4-Methyl-oxazolyl-5 | |
| 113 | H | H | H | Br | 2-Methyl-oxazolyl-5 | |
| 114 | H | H | H | Br | Thiazolyl-2 | |
| 115 | H | H | H | Br | Thiazolyl-4 | |
| 116 | H | H | H | Br | Thiazolyl-5 | |
| 117 | H | H | H | Br | 4-Methyl-thiazolyl-5 | |
| 118 | H | H | H | Br | 2-Methyl-thiazolyl-5 | |
| 119 | H | H | H | Br | Imidazolyl-4 | |
| 120 | H | H | H | Br | Imidazolyl-5 | |
| 121 | H | H | H | Br | 4-Methyl-imidazolyl-5 | |
| 122 | H | H | H | Br | 1-Methyl-imidazolyl-5 | |
| 123 | H | H | H | Br | 4-Nitro-imidazolyl-1 | |
| 124 | H | H | H | Br | 2-Methyl-4-nitro-imidazolyl-1 | |
| 125 | H | H | H | Br | 4,5-Dichlorimidazolyl-1 | |
| 126 | H | H | H | Br | 1-Methyl-pyrryl-2 | |
| 127 | H | H | H | Br | Isoxazolyl-3 | 126-128 |
| 128 | H | H | H | Br | Isoxazolyl-4 | |
| 129 | H | H | H | Br | Isoxazolyl-5 | |

| Nr. | $R^1$ | $R^2$ | $R^3$ | $R^4$ | $R^5$ | Fp. [°C] |
|---|---|---|---|---|---|---|
| 130 | H | H | H | Br | 5-Chlormethyl-isoxazolyl-3 | 148-151 |
| 131 | H | H | H | Br | Isothiazolyl-3 | |
| 132 | H | H | H | Br | Isothiazolyl-4 | |
| 133 | H | H | H | Br | Isothiazolyl-5 | |
| 134 | H | H | H | Br | 4-Methyl-isothiazolyl-5 | |
| 135 | H | H | H | Br | Pyrazolyl-4 | |
| 136 | H | H | H | Br | Pyrazolyl-5 | |
| 137 | H | H | H | Br | 4-Chlor-pyrazolyl-5 | |
| 138 | H | H | H | Br | 1-Methyl-pyrazolyl-5 | |
| 139 | H | H | H | Br | 1,2,3-Thiadiazolyl-5 | |
| 140 | H | H | H | Br | 1,2,3-Oxadiazolyl-5 | |
| 141 | H | H | H | Br | 1,2,3-Oxadiazolyl-4 | |
| 142 | H | H | H | Br | 5-Methyl-1,2,3-oxadiazolyl-4 | |
| 143 | H | H | H | Br | 4-Methyl-1,2,3-oxadiazolyl-5 | |
| 144 | H | H | H | Br | 1,3,4-Thiadiazolyl-2 | |
| 145 | H | H | H | Br | 2-Methyl-1,3,4-thiadiazolyl-5 | |
| 146 | H | H | H | Br | 1,2,4-Thiadiazolyl-3 | |
| 147 | H | H | H | Br | 1,2,4-Thiadiazolyl-5 | |
| 148 | H | H | H | Br | 3-Methyl-1,2,4-thiadiazolyl-5 | |
| 149 | H | H | H | Br | 1,2,4-Oxadiazolyl-3 | |
| 150 | H | H | H | Br | 1,2,4-Oxadiazolyl-5 | |
| 151 | H | H | H | Br | 1,2,4-Triazolyl-5 | |
| 152 | H | H | H | Br | 1,2,4-Triazolyl-3 | |
| 153 | H | H | H | Br | 1,3,4-Oxadiazolyl-5 | |
| 154 | H | H | H | Br | 2-Methyl-1,3,4-oxadiazolyl-5 | |
| 155 | H | H | H | Br | 2-Chlor-1,3,4-oxadiazolyl-5 | |
| 156 | H | H | H | Br | 1,2,5-Oxadiazolyl-3 | |
| 157 | H | H | H | Br | 3-Methyl-1,2,5-oxadiazolyl-4 | |
| 158 | H | H | H | Br | 1,2,5-Thiadiazolyl-3 | |
| 159 | H | H | H | Br | 3-Methyl-1,2,5-thiadiazolyl-4 | |
| 160 | H | H | H | Br | 2-Methyl-furyl-5 | |
| 161 | H | H | H | Br | 2,5-Dimethylfuryl-4 | 159-161 |
| 162 | H | H | H | Br | 2-Chlor-furyl-5 | |
| 163 | H | H | H | Br | 5-Methyl-oxazolyl-4 | |
| 164 | H | H | H | Br | 4-Methyl-oxazolyl-2 | |
| 165 | H | H | H | Br | 2-Bromfuryl-5 | |
| 166 | H | H | H | Br | 2-Methyl-furyl-4 | |
| 167 | H | H | H | Br | 5-Methyl-isoxazolyl-3 | |
| 168 | H | H | H | Br | 4-Methyl-isoxazolyl-3 | |
| 169 | H | H | H | Br | 5-Methyl-isothiazolyl-3 | |
| 170 | H | H | H | Br | 4-Methyl-isothiazolyl-3 | |
| 171 | H | H | H | Br | 5-Methyl-1,2,3-thiadiazolyl-4 | |

| Nr. | $R^1$ | $R^2$ | $R^3$ | $R^4$ | $R^5$ | Fp. [°C] |
|---|---|---|---|---|---|---|
| 172 | H | H | H | Br | 4-Methyl-1,2,3-thiadiazolyl-5 | 184-185 |
| 173 | H | H | H | Br | Pyrryl-1 | |
| 174 | H | H | H | Br | Imidazolyl-1 | |
| 175 | H | H | H | Br | Pyrazolyl-1 | |
| 176 | H | H | H | Br | 1,3,4-Triazolyl-1 | |
| 177 | H | H | H | Br | 1,2,4-Triazolyl-1 | |
| 178 | H | H | H | $NO_2$ | 2-Chlor- thienyl-3 | |
| 179 | H | H | H | $NO_2$ | 5-Chlor- thienyl-2 | |
| 180 | H | H | H | $NO_2$ | 5-Nitro- thienyl-2 | |
| 181 | H | H | H | $NO_2$ | 4-Methyl-thienyl-2 | |
| 182 | H | H | H | $NO_2$ | 3-Methyl-thienyl-2 | |
| 183 | H | H | H | $NO_2$ | 2-Methyl-thienyl-3 | |
| 184 | H | H | H | $NO_2$ | 3-Methyl-thienyl-4 | |
| 185 | H | H | H | $NO_2$ | 2,5-Dimethyl-thienyl-4 | |
| 186 | H | H | H | $NO_2$ | 2,3-Dichlor-thienyl-4 | |
| 187 | H | H | H | $NO_2$ | 5-Methyl-thienyl-2 | |
| 188 | H | H | H | $NO_2$ | Pyrryl-2 | |
| 189 | H | H | H | $NO_2$ | Pyrryl-3 | |
| 190 | H | H | H | $NO_2$ | 3-Methyl-pyrryl-2 | |
| 191 | H | H | H | $NO_2$ | 2-Methyl-pyrryl-3 | |
| 192 | H | H | H | $NO_2$ | 5-Chlor-pyrryl-2 | |
| 193 | H | H | H | $NO_2$ | Oxazolyl-2 | |
| 194 | H | H | H | $NO_2$ | Oxazolyl-4 | |
| 195 | H | H | H | $NO_2$ | Oxazolyl-5 | |
| 196 | H | H | H | $NO_2$ | 1,2,3-Thiadiazolyl-4 | 198 Zers. |
| 197 | H | H | H | $NO_2$ | 4-Methyl-oxazolyl-5 | |
| 198 | H | H | H | $NO_2$ | 2-Methyl-oxazolyl-5 | |
| 199 | H | H | H | $NO_2$ | Thiazolyl-2 | |
| 200 | H | H | H | $NO_2$ | Thiazolyl-4 | |
| 201 | H | H | H | $NO_2$ | Thiazolyl-5 | |
| 202 | H | H | H | $NO_2$ | 4-Methyl-thiazolyl-5 | |
| 203 | H | H | H | $NO_2$ | 2-Methyl-thiazolyl-5 | |
| 204 | H | H | H | $NO_2$ | Imidazolyl-4 | |
| 205 | H | H | H | $NO_2$ | Imidazolyl-5 | |
| 206 | H | H | H | $NO_2$ | 4-Methyl-imidazolyl-5 | |
| 207 | H | H | H | $NO_2$ | 1-Methyl-imidazolyl-5 | |
| 208 | H | H | H | $NO_2$ | 4-Nitro-imidazolyl-1 | |
| 209 | H | H | H | $NO_2$ | 2-Methyl-4-nitro-imidazolyl-1 | |
| 210 | H | H | H | $NO_2$ | 4,5-Dichlorimidazolyl-1 | |
| 211 | H | H | H | $NO_2$ | 1-Methyl-pyrryl-2 | |

| Nr. | $R^1$ | $R^2$ | $R^3$ | $R^4$ | $R^5$ | Fp. [°C] |
|---|---|---|---|---|---|---|
| 212 | H | H | H | $NO_2$ | Isoxazolyl-3 | 153-154 |
| 213 | H | H | H | $NO_2$ | Isoxazolyl-4 | |
| 214 | H | H | H | $NO_2$ | Isoxazolyl-5 | 168-170 |
| 215 | H | H | H | $NO_2$ | 5-Chlormethyl-isoxazolyl-3 | 129-131 |
| 216 | H | H | H | $NO_2$ | Isothiazolyl-3 | |
| 217 | H | H | H | $NO_2$ | Isothiazolyl-4 | |
| 218 | H | H | H | $NO_2$ | Isothiazolyl-5 | |
| 219 | H | H | H | $NO_2$ | 4-Methyl-isothiazolyl-5 | |
| 220 | H | H | H | $NO_2$ | Pyrazolyl-4 | |
| 221 | H | H | H | $NO_2$ | Pyrazolyl-5 | |
| 222 | H | H | H | $NO_2$ | 4-Chlor-pyrazolyl-5 | |
| 223 | H | H | H | $NO_2$ | 1-Methyl-pyrazolyl-5 | |
| 224 | H | H | H | $NO_2$ | 1,2,3-Thiadiazolyl-5 | |
| 225 | H | H | H | $NO_2$ | 1,2,3-Oxadiazolyl-5 | |
| 226 | H | H | H | $NO_2$ | 1,2,3-Oxadiazolyl-4 | |
| 227 | H | H | H | $NO_2$ | 5-Methyl-1,2,3-oxadiazolyl-4 | |
| 228 | H | H | H | $NO_2$ | 4-Methyl-1,2,3-oxadiazolyl-5 | |
| 229 | H | H | H | $NO_2$ | 1,3,4-Thiadiazolyl-2 | |
| 230 | H | H | H | $NO_2$ | 2-Methyl-1,3,4-thiadiazolyl-5 | |
| 231 | H | H | H | $NO_2$ | 1,2,4-Thiadiazolyl-3 | |
| 232 | H | H | H | $NO_2$ | 1,2,4-Thiadiazolyl-5 | |
| 233 | H | H | H | $NO_2$ | 3-Methyl-1,2,4-thiadiazolyl-5 | |
| 234 | H | H | H | $NO_2$ | 1,2,4-Oxadiazolyl-3 | |
| 235 | H | H | H | $NO_2$ | 1,2,4-Oxadiazolyl-5 | |
| 236 | H | H | H | $NO_2$ | 1,2,4-Triazolyl-5 | |
| 237 | H | H | H | $NO_2$ | 1,2,4-Triazolyl-3 | |
| 238 | H | H | H | $NO_2$ | 1,3,4-Oxadiazolyl-5 | |
| 239 | H | H | H | $NO_2$ | 2-Methyl-1,3,4-oxadiazolyl-5 | |
| 240 | H | H | H | $NO_2$ | 2-Chlor-1,3,4-oxadiazolyl-5 | |
| 241 | H | H | H | $NO_2$ | 1,2,5-Oxadiazolyl-3 | |
| 242 | H | H | H | $NO_2$ | 3-Methyl-1,2,5-oxadiazolyl-4 | |
| 243 | H | H | H | $NO_2$ | 1,2,5-Thiadiazolyl-3 | |
| 244 | H | H | H | $NO_2$ | 3-Methyl-1,2,5-thiadiazolyl-4 | |
| 245 | H | H | H | $NO_2$ | 2-Methyl-furyl-5 | |
| 246 | H | H | H | $NO_2$ | 2,5-Dimethylfuryl-4 | 156-157 |
| 247 | H | H | H | $NO_2$ | 2-Chlor-furyl-5 | |
| 248 | H | H | H | $NO_2$ | 5-Methyl-oxazolyl-4 | |
| 249 | H | H | H | $NO_2$ | 4-Methyl-oxazolyl-2 | |
| 250 | H | H | H | $NO_2$ | 2-Bromfuryl-5 | |
| 251 | H | H | H | $NO_2$ | 2-Methyl-furyl-4 | |
| 252 | H | H | H | $NO_2$ | 5-Methyl-isoxazolyl-3 | |
| 253 | H | H | H | $NO_2$ | 4-Methyl-isoxazolyl-3 | |

| Nr. | $R^1$ | $R^2$ | $R^3$ | $R^4$ | $R^5$ | Fp. [°C] |
|---|---|---|---|---|---|---|
| 254 | H | H | H | $NO_2$ | 5-Methyl-isothiazolyl-3 | |
| 255 | H | H | H | $NO_2$ | 4-Methyl-isothiazolyl-3 | |
| 256 | H | H | H | $NO_2$ | 4-Methyl-1,2,3-thiadiazolyl-5 | 154-155 |
| 257 | H | H | H | $NO_2$ | 5-Methyl-1,2,3-thiadiazolyl-4 | |
| 258 | H | H | H | $NO_2$ | Pyrryl-1 | |
| 259 | H | H | H | $NO_2$ | Imidazolyl-1 | |
| 260 | H | H | H | $NO_2$ | Pyrazolyl-1 | |
| 261 | H | H | H | $NO_2$ | 1,3,4-Triazolyl-1 | |
| 262 | H | H | H | $NO_2$ | 1,2,4-Triazolyl-1 | |
| 263 | H | H | $NO_2$ | H | Thienyl-2 | |
| 264 | H | H | $NO_2$ | H | Thienyl-3 | |
| 265 | H | H | $NO_2$ | H | 2-Chlor-thienyl-3 | |
| 266 | H | H | $NO_2$ | H | 5-Chlor-thienyl-2 | |
| 267 | H | H | $NO_2$ | H | 5-Nitro-thienyl-2 | |
| 268 | H | H | $NO_2$ | H | 4-Methyl-thienyl-2 | |
| 269 | H | H | $NO_2$ | H | 3-Methyl-thienyl-2 | |
| 270 | H | H | $NO_2$ | H | 2-Methyl-thienyl-3 | |
| 271 | H | H | $NO_2$ | H | 3-Methyl-thienyl-4 | |
| 272 | H | H | $NO_2$ | H | 2,5-Dimethyl-thienyl-4 | |
| 273 | H | H | $NO_2$ | H | 2,3-Dichlor- thienyl-4 | |
| 274 | H | H | $NO_2$ | H | 5-Methyl- thienyl-2 | |
| 275 | H | H | $NO_2$ | H | Pyrryl-2 | |
| 276 | H | H | $NO_2$ | H | Pyrryl-3 | |
| 277 | H | H | $NO_2$ | H | 3-Methyl-pyrryl-2 | |
| 278 | H | H | $NO_2$ | H | 2-Methyl-pyrryl-3 | |
| 279 | H | H | $NO_2$ | H | 5-Chlor-pyrryl-2 | |
| 280 | H | H | $NO_2$ | H | Oxazolyl-2 | |
| 281 | H | H | $NO_2$ | H | Oxazolyl-4 | |
| 282 | H | H | $NO_2$ | H | Oxazolyl-5 | |
| 283 | H | H | $NO_2$ | H | 4-Methyl-oxazolyl-5 | |
| 284 | H | H | $NO_2$ | H | 2-Methyl-oxazolyl-5 | |
| 285 | H | H | $NO_2$ | H | Thiazolyl-2 | |
| 286 | H | H | $NO_2$ | H | Thiazolyl-4 | |
| 287 | H | H | $NO_2$ | H | Thiazolyl-5 | |
| 288 | H | H | $NO_2$ | H | 4-Methyl-thiazolyl-5 | |
| 289 | H | H | $NO_2$ | H | 2-Methyl-thiazolyl-5 | |
| 290 | H | H | $NO_2$ | H | Imidazolyl-4 | |
| 291 | H | H | $NO_2$ | H | Imidazolyl-5 | |
| 292 | H | H | $NO_2$ | H | 4-Methyl-imidazolyl-5 | |
| 293 | H | H | $NO_2$ | H | 1-Methyl-imidazolyl-5 | |
| 294 | H | H | $NO_2$ | H | 4-Nitro-imidazolyl-1 | |
| 295 | H | H | $NO_2$ | H | 2-Methyl-4-nitro-imidazolyl-1 | |

| Nr. | $R^1$ | $R^2$ | $R^3$ | $R^4$ | $R^5$ | Fp. [°C] |
|---|---|---|---|---|---|---|
| 296 | H | H | $NO_2$ | H | 4,5-Dichlorimidazolyl-1 | |
| 297 | H | H | $NO_2$ | H | 1-Methyl-pyrryl-2 | |
| 298 | H | H | $NO_2$ | H | Isoxazolyl-3 | 150-152 |
| 299 | H | H | $NO_2$ | H | Isoxazolyl-4 | |
| 300 | H | H | $NO_2$ | H | Isoxazolyl-5 | 167-169 |
| 301 | H | H | $NO_2$ | H | 5-Chlormethyl-isoxazolyl-3 | 129-131 |
| 302 | H | H | $NO_2$ | H | Isothiazolyl-3 | |
| 303 | H | H | $NO_2$ | H | Isothiazolyl-4 | |
| 304 | H | H | $NO_2$ | H | Isothiazolyl-5 | |
| 305 | H | H | $NO_2$ | H | 4-Methyl-isothiazolyl-5 | |
| 306 | H | H | $NO_2$ | H | Pyrazolyl-4 | |
| 307 | H | H | $NO_2$ | H | Pyrazolyl-5 | |
| 308 | H | H | $NO_2$ | H | 4-Chlor-pyrazolyl-5 | |
| 309 | H | H | $NO_2$ | H | 1-Methyl-pyrazolyl-5 | |
| 310 | H | H | $NO_2$ | H | 1,2,3-Thiadiazolyl-5 | |
| 311 | H | H | $NO_2$ | H | 1,2,3-Oxadiazolyl-5 | |
| 312 | H | H | $NO_2$ | H | 1,2,3-Oxadiazolyl-4 | |
| 313 | H | H | $NO_2$ | H | 5-Methyl-1,2,3-oxadiazolyl-4 | |
| 314 | H | H | $NO_2$ | H | 4-Methyl-1,2,3-oxadiazolyl-5 | |
| 315 | H | H | $NO_2$ | H | 1,3,4-Thiadiazolyl-2 | |
| 316 | H | H | $NO_2$ | H | 2-Methyl-1,3,4-thiadiazolyl-5 | |
| 317 | H | H | $NO_2$ | H | 1,2,4-Thiadiazolyl-3 | |
| 318 | H | H | $NO_2$ | H | 1,2,4-Thiadiazolyl-5 | |
| 319 | H | H | $NO_2$ | H | 3-Methyl-1,2,4-thiadiazolyl-5 | |
| 320 | H | H | $NO_2$ | H | 1,2,4-Oxadiazolyl-3 | |
| 321 | H | H | $NO_2$ | H | 1,2,4-Oxadiazolyl-5 | |
| 322 | H | H | $NO_2$ | H | 1,2,4-Triazolyl-5 | |
| 323 | H | H | $NO_2$ | H | 1,2,4-Triazolyl-3 | |
| 324 | H | H | $NO_2$ | H | 1,3,4-Oxadiazolyl-5 | |
| 325 | H | H | $NO_2$ | H | 2-Methyl-1,3,4-oxadiazolyl-5 | |
| 326 | H | H | $NO_2$ | H | 2-Chlor-1,3,4-oxadiazolyl-5 | |
| 327 | H | H | $NO_2$ | H | 1,2,5-Oxadiazolyl-3 | |
| 328 | H | H | $NO_2$ | H | 3-Methyl-1,2,5-oxadiazolyl-4 | |
| 329 | H | H | $NO_2$ | H | 1,2,5-Thiadiazolyl-3 | |
| 330 | H | H | $NO_2$ | H | 3-Methyl-1,2,5-thiadiazolyl-4 | |
| 331 | H | H | $NO_2$ | H | 2-Methyl-furyl-5 | |
| 332 | H | H | $NO_2$ | H | 2,5-Dimethylfuryl | 158-160 |
| 333 | H | H | $NO_2$ | H | 5-Methyl-oxazolyl-4 | |
| 334 | H | H | $NO_2$ | H | 4-Methyl-oxazolyl-2 | |
| 336 | H | H | $NO_2$ | H | 2-Methyl-furyl-4 | |
| 337 | H | H | $NO_2$ | H | 5-Methyl-isoxazolyl-3 | |

| Nr. | $R^1$ | $R^2$ | $R^3$ | $R^4$ | $R^5$ | Fp. [°C] |
|---|---|---|---|---|---|---|
| 338 | H | H | $NO_2$ | H | 4-Methyl-isoxazolyl-3 | |
| 339 | H | H | $NO_2$ | H | 5-Methyl-isothiazolyl-3 | |
| 340 | H | H | $NO_2$ | H | 4-Methyl-isothiazolyl-3 | |
| 341 | H | H | $NO_2$ | H | 5-Methyl-1,2,3-thiadiazolyl-4 | |
| 342 | H | H | $NO_2$ | H | 4-Methyl-1,2,3-thiadiazolyl-5 | 145-147 |
| 343 | H | H | $NO_2$ | H | Pyrryl-1 | |
| 344 | H | H | $NO_2$ | H | Imidazolyl-1 | |
| 345 | H | H | $NO_2$ | H | Pyrazolyl-1 | |
| 346 | H | H | $NO_2$ | H | 1,3,4-Triazolyl-1 | |
| 347 | H | H | $NO_2$ | H | 1,2,4-Triazolyl-1 | |
| 348 | H | H | $NO_2$ | Cl | Thienyl-2 | |
| 349 | H | H | $NO_2$ | Cl | Thienyl-3 | |
| 350 | H | H | $NO_2$ | Cl | 2-Chlor-thienyl-3 | |
| 351 | H | H | $NO_2$ | Cl | 5-Chlor-thienyl-2 | |
| 352 | H | H | $NO_2$ | Cl | 5-Nitro-thienyl-2 | |
| 353 | H | H | $NO_2$ | Cl | 4-Methyl-thienyl-2 | |
| 354 | H | H | $NO_2$ | Cl | 3-Methyl-thienyl-2 | |
| 355 | H | H | $NO_2$ | Cl | 2-Methyl-thienyl-3 | |
| 356 | H | H | $NO_2$ | Cl | 3-Methyl-thienyl-4 | |
| 357 | H | H | $NO_2$ | Cl | 2,5-Dimethyl-thienyl-4 | |
| 358 | H | H | $NO_2$ | Cl | 2,3-Dichlor- thienyl-4 | |
| 359 | H | H | $NO_2$ | Cl | 5-Methyl- thienyl-2 | |
| 360 | H | H | $NO_2$ | Cl | Pyrryl-2 | |
| 361 | H | H | $NO_2$ | Cl | Pyrryl-3 | |
| 362 | H | H | $NO_2$ | Cl | 3-Methyl-pyrryl-2 | |
| 363 | H | H | $NO_2$ | Cl | 2-Methyl-pyrryl-3 | |
| 364 | H | H | $NO_2$ | Cl | 5-Chlor-pyrryl-2 | |
| 365 | H | H | $NO_2$ | Cl | Oxazolyl-2 | |
| 366 | H | H | $NO_2$ | Cl | Oxazolyl-4 | |
| 367 | H | H | $NO_2$ | Cl | Oxazolyl-5 | |
| 368 | H | H | $NO_2$ | Cl | 1,2,3-Thiadiazolyl-4 | 205-207 |
| 369 | H | H | $NO_2$ | Cl | 4-Methyl-oxazolyl-5 | |
| 370 | H | H | $NO_2$ | Cl | 2-Methyl-oxazolyl-5 | |
| 371 | H | H | $NO_2$ | Cl | Thiazolyl-2 | |
| 372 | H | H | $NO_2$ | Cl | Thiazolyl-4 | |
| 373 | H | H | $NO_2$ | Cl | Thiazolyl-5 | |
| 374 | H | H | $NO_2$ | Cl | 4-Methyl-thiazolyl-5 | |
| 375 | H | H | $NO_2$ | Cl | 2-Methyl-thiazolyl-5 | |
| 376 | H | H | $NO_2$ | Cl | Imidazolyl-4 | |
| 377 | H | H | $NO_2$ | Cl | Imidazolyl-5 | |
| 378 | H | H | $NO_2$ | Cl | 4-Methyl-imidazolyl-5 | |
| 379 | H | H | $NO_2$ | Cl | 1-Methyl-imidazolyl-5 | |

| Nr. | $R^1$ | $R^2$ | $R^3$ | $R^4$ | $R^5$ | Fp. [°C] |
|---|---|---|---|---|---|---|
| 380 | H | H | $NO_2$ | Cl | 4-Nitro-imidazolyl-1 | |
| 381 | H | H | $NO_2$ | Cl | 2-Methyl-4-nitro-imidazolyl-1 | |
| 382 | H | H | $NO_2$ | Cl | 4,5-Dichlorimidazolyl-1 | |
| 383 | H | H | $NO_2$ | Cl | 1-Methyl-pyrryl-2 | |
| 384 | H | H | $NO_2$ | Cl | Isoxazolyl-3 | 132-134 |
| 385 | H | H | $NO_2$ | Cl | Isoxazolyl-4 | |
| 386 | H | H | $NO_2$ | Cl | 5-Chlormethyl-isoxazolyl-3 | 138-141 |
| 387 | H | H | $NO_2$ | Cl | Isothiazolyl-3 | |
| 388 | H | H | $NO_2$ | Cl | Isothiazolyl-4 | |
| 389 | H | H | $NO_2$ | Cl | Isothiazolyl-5 | |
| 390 | H | H | $NO_2$ | Cl | 4-Methyl-isothiazolyl-5 | |
| 391 | H | H | $NO_2$ | Cl | Pyrazolyl-4 | |
| 392 | H | H | $NO_2$ | Cl | Pyrazolyl-5 | |
| 393 | H | H | $NO_2$ | Cl | 4-Chlor-pyrazolyl-5 | |
| 394 | H | H | $NO_2$ | Cl | 1-Methyl-pyrazolyl-5 | |
| 395 | H | H | $NO_2$ | Cl | 1,2,3-Thiadiazolyl-5 | |
| 396 | H | H | $NO_2$ | Cl | 1,2,3-Oxadiazolyl-5 | |
| 397 | H | H | $NO_2$ | Cl | 1,2,3-Oxadiazolyl-4 | |
| 398 | H | H | $NO_2$ | Cl | 5-Methyl-1,2,3-oxadiazolyl-4 | |
| 399 | H | H | $NO_2$ | Cl | 4-Metnyl-1,2,3-oxadiazolyl-5 | |
| 400 | H | H | $NO_2$ | Cl | 1,3,4-Thiadiazolyl-2 | |
| 401 | H | H | $NO_2$ | Cl | 2-Methyl-1,3,4-thiadiazolyl-5 | |
| 402 | H | H | $NO_2$ | Cl | 1,2,4-Thiadiazolyl-3 | |
| 403 | H | H | $NO_2$ | Cl | 1,2,4-Thiadiazolyl-5 | |
| 404 | H | H | $NO_2$ | Cl | 3-Methyl-1,2,4-thiadiazolyl-5 | |
| 405 | H | H | $NO_2$ | Cl | 1,2,4-Oxadiazolyl-3 | |
| 406 | H | H | $NO_2$ | Cl | 1,2,4-Oxadiazolyl-5 | |
| 407 | H | H | $NO_2$ | Cl | 1,2,4-Triazolyl-5 | |
| 408 | H | H | $NO_2$ | Cl | 1,2,4-Triazolyl-3 | |
| 409 | H | H | $NO_2$ | Cl | 1,3,4-Oxadiazolyl-5 | |
| 410 | H | H | $NO_2$ | Cl | 2-Methyl-1,3,4-oxadiazolyl-5 | |
| 411 | H | H | $NO_2$ | Cl | 2-Chlor-1,3,4-oxadiazolyl-5 | |
| 412 | H | H | $NO_2$ | Cl | 1,2,5-Oxadiazolyl-3 | |
| 413 | H | H | $NO_2$ | Cl | 3-Methyl-1,2,5-oxadiazolyl-4 | |
| 414 | H | H | $NO_2$ | Cl | 1,2,5-Thiadiazolyl-3 | |
| 415 | H | H | $NO_2$ | Cl | 3-Methyl-1,2,5-thiadiazolyl-4 | |
| 416 | H | H | $NO_2$ | Cl | 2-Methyl-furyl-5 | |
| 417 | H | H | $NO_2$ | Cl | 2,5-Dimethylfuryl | 116-118 |
| 418 | H | H | $NO_2$ | Cl | 2-Chlor-furyl-5 | |
| 419 | H | H | $NO_2$ | Cl | 5-Methyl-oxazolyl-4 | |
| 420 | H | H | $NO_2$ | Cl | 4-Methyl-oxazolyl-2 | |
| 421 | H | H | $NO_2$ | Cl | 2-Bromfuryl-5 | |

| Nr. | $R^1$ | $R^2$ | $R^3$ | $R^4$ | $R^5$ | Fp. [°C] |
|---|---|---|---|---|---|---|
| 422 | H | H | $NO_2$ | Cl | 2-Methyl-furyl-4 | |
| 423 | H | H | $NO_2$ | Cl | 5-Methyl-isoxazolyl-3 | |
| 424 | H | H | $NO_2$ | Cl | 4-Methyl-isoxazolyl-3 | |
| 425 | H | H | $NO_2$ | Cl | 5-Methyl-isothiazolyl-3 | |
| 426 | H | H | $NO_2$ | Cl | 4-Methyl-isothiazolyl-3 | |
| 427 | H | H | $NO_2$ | Cl | 4-Methyl-1,2,3-thiadiazolyl-5 | 230-234 |
| 428 | H | H | $NO_2$ | Cl | 5-Methyl-1,2,3-thiadiazolyl-4 | 145-148 |
| 429 | H | H | $NO_2$ | Cl | Pyrryl-1 | |
| 430 | H | H | $NO_2$ | Cl | Imidazolyl-1 | |
| 431 | H | H | $NO_2$ | Cl | Pyrazolyl-1 | |
| 432 | H | H | $NO_2$ | Cl | 1,3,4-Triazolyl-1 | |
| 433 | H | H | $NO_2$ | Cl | 1,2,4-Triazolyl-1 | |
| 434 | H | H | $NO_2$ | Br | Thienyl-2 | |
| 435 | H | H | $NO_2$ | Br | Thienyl-3 | |
| 436 | H | H | $NO_2$ | Br | 2-Chlor-thienyl-3 | |
| 437 | H | H | $NO_2$ | Br | 5-Chlor-thienyl-2 | |
| 438 | H | H | $NO_2$ | Br | 5-Nitro-thienyl-2 | |
| 439 | H | H | $NO_2$ | Br | 4-Methyl-thienyl-2 | |
| 440 | H | H | $NO_2$ | Br | 3-Methyl-thienyl-2 | |
| 441 | H | H | $NO_2$ | Br | 2-Methyl-thienyl-3 | |
| 442 | H | H | $NO_2$ | Br | 3-Methyl-thienyl-4 | |
| 443 | H | H | $NO_2$ | Br | 2,5-Dimethyl-thienyl-4 | |
| 444 | H | H | $NO_2$ | Br | 2,3-Dichlor-thienyl-4 | |
| 445 | H | H | $NO_2$ | Br | 5-Methyl-thienyl-2 | |
| 446 | H | H | $NO_2$ | Br | Pyrryl-2 | |
| 447 | H | H | $NO_2$ | Br | Pyrryl-3 | |
| 448 | H | H | $NO_2$ | Br | 3-Methyl-pyrryl-2 | |
| 449 | H | H | $NO_2$ | Br | 2-Methyl-pyrryl-3 | |
| 450 | H | H | $NO_2$ | Br | 5-Chlor-pyrryl-2 | |
| 451 | H | H | $NO_2$ | Br | Oxazolyl-2 | |
| 452 | H | H | $NO_2$ | Br | Oxazolyl-4 | |
| 453 | H | H | $NO_2$ | Br | Oxazolyl-5 | |
| 454 | H | H | $NO_2$ | Br | 4-Methyl-oxazolyl-5 | |
| 455 | H | H | $NO_2$ | Br | 2-Methyl-oxazolyl-5 | |
| 456 | H | H | $NO_2$ | Br | Thiazolyl-2 | |
| 457 | H | H | $NO_2$ | Br | Thiazolyl-4 | |
| 458 | H | H | $NO_2$ | Br | Thiazolyl-5 | |
| 459 | H | H | $NO_2$ | Br | 4-Methyl-thiazolyl-5 | |
| 460 | H | H | $NO_2$ | Br | 2-Methyl-thiazolyl-5 | |
| 461 | H | H | $NO_2$ | Br | Imidazolyl-4 | |
| 462 | H | H | $NO_2$ | Br | Imidazolyl-5 | |
| 463 | H | H | $NO_2$ | Br | 4-Methyl-imidazolyl-5 | |

| Nr. | $R^1$ | $R^2$ | $R^3$ | $R^4$ | $R^5$ | Fp. [°C] |
|---|---|---|---|---|---|---|
| 464 | H | H | $NO_2$ | Br | 1-Methyl-imidazolyl-5 | |
| 465 | H | H | $NO_2$ | Br | 4-Nitro-imidazolyl-1 | |
| 466 | H | H | $NO_2$ | Br | 2-Methyl-4-nitro-imidazolyl-1 | |
| 467 | H | H | $NO_2$ | Br | 4,5-Dichlorimidazolyl-1 | |
| 468 | H | H | $NO_2$ | Br | 1-Methyl-pyrryl-2 | |
| 469 | H | H | $NO_2$ | Br | Isoxazolyl-3 | 143-147 |
| 470 | H | H | $NO_2$ | Br | Isoxazolyl-4 | |
| 471 | H | H | $NO_2$ | Br | Isoxazolyl-5 | 138-141 |
| 472 | H | H | $NO_2$ | Br | 5-Chlormethyl-isoxazolyl-3 | |
| 473 | H | H | $NO_2$ | Br | Isothiazolyl-3 | |
| 474 | H | H | $NO_2$ | Br | Isothiazolyl-4 | |
| 475 | H | H | $NO_2$ | Br | Isothiazolyl-5 | |
| 476 | H | H | $NO_2$ | Br | 4-Methyl-isothiazolyl-5 | |
| 477 | H | H | $NO_2$ | Br | Pyrazolyl-4 | |
| 478 | H | H | $NO_2$ | Br | Pyrazolyl-5 | |
| 479 | H | H | $NO_2$ | Br | 4-Chlor-pyrazolyl-5 | |
| 480 | H | H | $NO_2$ | Br | 1-Methyl-pyrazolyl-5 | |
| 481 | H | H | $NO_2$ | Br | 1,2,3-Thiadiazolyl-5 | |
| 482 | H | H | $NO_2$ | Br | 1,2,3-Oxadiazolyl-5 | |
| 483 | H | H | $NO_2$ | Br | 1,2,3-Oxadiazolyl-4 | |
| 484 | H | H | $NO_2$ | Br | 5-Methyl-1,2,3-oxadiazolyl-4 | |
| 485 | H | H | $NO_2$ | Br | 4-Methyl-1,2,3-oxadiazolyl-5 | |
| 486 | H | H | $NO_2$ | Br | 1,3,4-Thiadiazolyl-2 | |
| 487 | H | H | $NO_2$ | Br | 2-Methyl-1,3,4-thiadiazolyl-5 | |
| 488 | H | H | $NO_2$ | Br | 1,2,4-Thiadiazolyl-3 | |
| 489 | H | H | $NO_2$ | Br | 1,2,4-Thiadiazolyl-5 | |
| 490 | H | H | $NO_2$ | Br | 3-Methyl-1,2,4-thiadiazolyl-5 | |
| 491 | H | H | $NO_2$ | Br | 1,2,4-Oxadiazolyl-3 | |
| 492 | H | H | $NO_2$ | Br | 1,2,4-Oxadiazolyl-5 | |
| 493 | H | H | $NO_2$ | Br | 1,2,4-Triazolyl-5 | |
| 494 | H | H | $NO_2$ | Br | 1,2,4-Triazolyl-3 | |
| 495 | H | H | $NO_2$ | Br | 1,3,4-Oxadiazolyl-5 | |
| 496 | H | H | $NO_2$ | Br | 2-Methyl-1,3,4-oxadiazolyl-5 | |
| 497 | H | H | $NO_2$ | Br | 2-Chlor-1,3,4-oxadiazolyl-5 | |
| 498 | H | H | $NO_2$ | Br | 1,2,5-Oxadiazolyl-3 | |
| 499 | H | H | $NO_2$ | Br | 3-Methyl-1,2,5-oxadiazolyl-4 | |
| 500 | H | H | $NO_2$ | Br | 1,2,5-Thiadiazolyl-3 | |
| 501 | H | H | $NO_2$ | Br | 3-Methyl-1,2,5-thiadiazolyl-4 | |
| 502 | H | H | $NO_2$ | Br | 2-Methyl-furyl-5 | |
| 503 | H | H | $NO_2$ | Br | 2,5-Dimethylfuryl | 121-123 |
| 504 | H | H | $NO_2$ | Br | 2-Chlor-furyl-5 | |
| 505 | H | H | $NO_2$ | Br | 5-Methyl-oxazolyl-4 | |

| Nr. | $R^1$ | $R^2$ | $R^3$ | $R^4$ | $R^5$ | Fp. [°C] |
|---|---|---|---|---|---|---|
| 506 | H | H | $NO_2$ | Br | 4-Methyl-oxazolyl-2 | |
| 507 | H | H | $NO_2$ | Br | 2-Bromfuryl-5 | |
| 508 | H | H | $NO_2$ | Br | 2-Methyl-furyl-4 | |
| 509 | H | H | $NO_2$ | Br | 5-Methyl-isoxazolyl-3 | |
| 510 | H | H | $NO_2$ | Br | 4-Methyl-isoxazolyl-3 | |
| 511 | H | H | $NO_2$ | Br | 5-Methyl-isothiazolyl-3 | |
| 512 | H | H | $NO_2$ | Br | 4-Methyl-isothiazolyl-3 | |
| 513 | H | H | $NO_2$ | Br | 4-Methyl-1,2,3-thiadiazolyl-5 | |
| 514 | H | H | $NO_2$ | Br | 5-Methyl-1,2,3-thiadiazolyl-4 | |
| 515 | H | H | $NO_2$ | Br | Pyrryl-1 | |
| 516 | H | H | $NO_2$ | Br | Imidazolyl-1 | |
| 517 | H | H | $NO_2$ | Br | Pyrazolyl-1 | |
| 518 | H | H | $NO_2$ | Br | 1,3,4-Triazolyl-1 | |
| 519 | H | H | $NO_2$ | Br | 1,2,4-Triazolyl-1 | |
| 520 | H | H | $NO_2$ | J | Thienyl-2 | |
| 521 | H | H | $NO_2$ | J | Thienyl-3 | |
| 522 | H | H | $NO_2$ | J | 2-Chlor-thienyl-3 | |
| 523 | H | H | $NO_2$ | J | 5-Chlor-thienyl-2 | |
| 524 | H | H | $NO_2$ | J | 5-Nitro-thienyl-2 | |
| 525 | H | H | $NO_2$ | J | 4-Methyl-thienyl-2 | |
| 526 | H | H | $NO_2$ | J | 3-Methyl-thienyl-2 | |
| 527 | H | H | $NO_2$ | J | 2-Methyl-thienyl-3 | |
| 528 | H | H | $NO_2$ | J | 3-Methyl-thienyl-4 | |
| 529 | H | H | $NO_2$ | J | 2,5-Dimethyl-thienyl-4 | |
| 530 | H | H | $NO_2$ | J | 2,3-Dichlor- thienyl-4 | |
| 531 | H | H | $NO_2$ | J | 5-Methyl- thienyl-2 | |
| 532 | H | H | $NO_2$ | J | Pyrryl-2 | |
| 533 | H | H | $NO_2$ | J | Pyrryl-3 | |
| 534 | H | H | $NO_2$ | J | 3-Methyl-pyrryl-2 | |
| 535 | H | H | $NO_2$ | J | 2-Methyl-pyrryl-3 | |
| 536 | H | H | $NO_2$ | J | 5-Chlor-pyrryl-2 | |
| 537 | H | H | $NO_2$ | J | Oxazolyl-2 | |
| 538 | H | H | $NO_2$ | J | Oxazolyl-4 | |
| 539 | H | H | $NO_2$ | J | Oxazolyl-5 | |
| 540 | H | H | $NO_2$ | J | 1,2,3-Thiadiazolyl-4 | |
| 541 | H | H | $NO_2$ | J | 4-Methyl-oxazolyl-5 | |
| 542 | H | H | $NO_2$ | J | 2-Methyl-oxazolyl-5 | |
| 543 | H | H | $NO_2$ | J | Thiazolyl-2 | |
| 544 | H | H | $NO_2$ | J | Thiazolyl-4 | |
| 545 | H | H | $NO_2$ | J | Thiazolyl-5 | |
| 546 | H | H | $NO_2$ | J | 4-Methyl-thiazolyl-5 | |
| 547 | H | H | $NO_2$ | J | 2-Methyl-thiazolyl-5 | |

| Nr. | $R^1$ | $R^2$ | $R^3$ | $R^4$ | $R^5$ | Fp. [°C] |
|---|---|---|---|---|---|---|
| 548 | H | H | $NO_2$ | J | Imidazolyl-4 | |
| 549 | H | H | $NO_2$ | J | Imidazolyl-5 | |
| 550 | H | H | $NO_2$ | J | 4-Methyl-imidazolyl-5 | |
| 551 | H | H | $NO_2$ | J | 1-Methyl-imidazolyl-5 | |
| 552 | H | H | $NO_2$ | J | 4-Nitro-imidazolyl-1 | |
| 553 | H | H | $NO_2$ | J | 2-Methyl-4-nitro-imidazolyl-1 | |
| 554 | H | H | $NO_2$ | J | 4,5-Dichlorimidazolyl-1 | |
| 555 | H | H | $NO_2$ | J | 1-Methyl-pyrryl-2 | |
| 556 | H | H | $NO_2$ | J | Isoxazolyl-3 | |
| 557 | H | H | $NO_2$ | J | Isoxazolyl-4 | |
| 558 | H | H | $NO_2$ | J | Isoxazolyl-5 | |
| 559 | H | H | $NO_2$ | J | 5-Chlormethyl-isoxazolyl-3 | |
| 560 | H | H | $NO_2$ | J | Isothiazolyl-3 | |
| 561 | H | H | $NO_2$ | J | Isothiazolyl-4 | |
| 562 | H | H | $NO_2$ | J | Isothiazolyl-5 | |
| 563 | H | H | $NO_2$ | J | 4-Methyl-isothiazolyl-5 | |
| 564 | H | H | $NO_2$ | J | Pyrazolyl-4 | |
| 565 | H | H | $NO_2$ | J | Pyrazolyl-5 | |
| 566 | H | H | $NO_2$ | J | 4-Chlor-pyrazolyl-5 | |
| 567 | H | H | $NO_2$ | J | 1-Methyl-pyrazolyl-5 | |
| 568 | H | H | $NO_2$ | J | 1,2,3-Thiadiazolyl-5 | |
| 569 | H | H | $NO_2$ | J | 1,2,3-Oxadiazolyl-5 | |
| 570 | H | H | $NO_2$ | J | 1,2,3-Oxadiazolyl-4 | |
| 571 | H | H | $NO_2$ | J | 5-Methyl-1,2,3-oxadiazolyl-4 | |
| 572 | H | H | $NO_2$ | J | 4-Methyl-1,2,3-oxadiazolyl-5 | |
| 573 | H | H | $NO_2$ | J | 1,3,4-Thiadiazolyl-2 | |
| 574 | H | H | $NO_2$ | J | 2-Methyl-1,3,4-thiadiazolyl-5 | |
| 575 | H | H | $NO_2$ | J | 1,2,4-Thiadiazolyl-3 | |
| 576 | H | H | $NO_2$ | J | 1,2,4-Thiadiazolyl-5 | |
| 577 | H | H | $NO_2$ | J | 3-Methyl-1,2,4-thiadiazolyl-5 | |
| 578 | H | H | $NO_2$ | J | 1,2,4-Oxadiazolyl-3 | |
| 579 | H | H | $NO_2$ | J | 1,2,4-Oxadiazolyl-5 | |
| 580 | H | H | $NO_2$ | J | 1,2,4-Triazolyl-5 | |
| 581 | H | H | $NO_2$ | J | 1,2,4-Triazolyl-3 | |
| 582 | H | H | $NO_2$ | J | 1,3,4-Oxadiazolyl-5 | |
| 583 | H | H | $NO_2$ | J | 2-Methyl-1,3,4-oxadiazolyl-5 | |
| 584 | H | H | $NO_2$ | J | 2-Chlor-1,3,4-oxadiazolyl-5 | |
| 585 | H | H | $NO_2$ | J | 1,2,5-Oxadiazolyl-3 | |
| 586 | H | H | $NO_2$ | J | 3-Methyl-1,2,5-oxadiazolyl-4 | |
| 587 | H | H | $NO_2$ | J | 1,2,5-Thiadiazolyl-3 | |
| 588 | H | H | $NO_2$ | J | 3-Methyl-1,2,5-thiadiazolyl-4 | |
| 589 | H | H | $NO_2$ | J | 2-Methyl-furyl-5 | |

| Nr. | $R^1$ | $R^2$ | $R^3$ | $R^4$ | $R^5$ | Fp. [°C] |
|---|---|---|---|---|---|---|
| 590 | H | H | $NO_2$ | J | 2,5-Dimethylfuryl-4 | |
| 591 | H | H | $NO_2$ | J | 2-Chlor-furyl-5 | |
| 592 | H | H | $NO_2$ | J | 5-Methyl-oxazolyl-4 | |
| 593 | H | H | $NO_2$ | J | 4-Methyl-oxazolyl-2 | |
| 594 | H | H | $NO_2$ | J | 2-Bromfuryl-5 | |
| 595 | H | H | $NO_2$ | J | 2-Methyl-furyl-4 | |
| 596 | H | H | $NO_2$ | J | 5-Methyl-isoxazolyl-3 | |
| 597 | H | H | $NO_2$ | J | 4-Methyl-isoxazolyl-3 | |
| 598 | H | H | $NO_2$ | J | 5-Methyl-isothiazolyl-3 | |
| 599 | H | H | $NO_2$ | J | 4-Methyl-isothiazolyl-3 | |
| 600 | H | H | $NO_2$ | J | 4-Methyl-1,2,3-thiadiazolyl-5 | |
| 601 | H | H | $NO_2$ | J | 5-Methyl-1,2,3-thiadiazolyl-4 | |
| 602 | H | H | $NO_2$ | J | Pyrryl-1 | |
| 603 | H | H | $NO_2$ | J | Imidazolyl-1 | |
| 604 | H | H | $NO_2$ | J | Pyrazolyl-1 | |
| 605 | H | H | $NO_2$ | J | 1,3,4-Triazolyl-1 | |
| 606 | H | H | $NO_2$ | J | 1,2,4-Triazolyl-1 | |
| 607 | H | H | $NO_2$ | $NO_2$ | Thienyl-2 | |
| 608 | H | H | $NO_2$ | $NO_2$ | Thienyl-3 | |
| 609 | H | H | $NO_2$ | $NO_2$ | 2-Chlor-thienyl-3 | |
| 610 | H | H | $NO_2$ | $NO_2$ | 5-Chlor-thienyl-2 | |
| 611 | H | H | $NO_2$ | $NO_2$ | 5-Nitro-thienyl-2 | |
| 612 | H | H | $NO_2$ | $NO_2$ | 4-Methyl-thienyl-2 | |
| 613 | H | H | $NO_2$ | $NO_2$ | 3-Methyl-thienyl-2 | |
| 614 | H | H | $NO_2$ | $NO_2$ | 2-Methyl-thienyl-3 | |
| 615 | H | H | $NO_2$ | $NO_2$ | 3-Methyl-thienyl-4 | |
| 616 | H | H | $NO_2$ | $NO_2$ | 2,5-Dimethyl-thienyl-4 | |
| 617 | H | H | $NO_2$ | $NO_2$ | 2,3-Dichlor-thienyl-4 | |
| 618 | H | H | $NO_2$ | $NO_2$ | 5-Methyl- thienyl-2 | |
| 619 | H | H | $NO_2$ | $NO_2$ | Pyrryl-2 | |
| 620 | H | H | $NO_2$ | $NO_2$ | Pyrryl-3 | |
| 621 | H | H | $NO_2$ | $NO_2$ | 3-Methyl-pyrryl-2 | |
| 622 | H | H | $NO_2$ | $NO_2$ | 2-Methyl-pyrryl-3 | |
| 623 | H | H | $NO_2$ | $NO_2$ | 5-Chlor-pyrryl-2 | |
| 624 | H | H | $NO_2$ | $NO_2$ | Oxazolyl-2 | |
| 625 | H | H | $NO_2$ | $NO_2$ | Oxazolyl-4 | |
| 626 | H | H | $NO_2$ | $NO_2$ | Oxazolyl-5 | |
| 627 | H | H | $NO_2$ | $NO_2$ | 1,2,3-Thiadiazolyl-4 | 172-174 |
| 628 | H | H | $NO_2$ | $NO_2$ | 4-Methyl-oxazolyl-5 | |
| 629 | H | H | $NO_2$ | $NO_2$ | 2-Methyl-oxazolyl-5 | |
| 630 | H | H | $NO_2$ | $NO_2$ | Thiazolyl-2 | |
| 631 | H | H | $NO_2$ | $NO_2$ | Thiazolyl-4 | |

| Nr. | $R^1$ | $R^2$ | $R^3$ | $R^4$ | $R^5$ | Fp. [°C] |
|---|---|---|---|---|---|---|
| 632 | H | H | $NO_2$ | $NO_2$ | Thiazolyl-5 | |
| 633 | H | H | $NO_2$ | $NO_2$ | 4-Methyl-thiazolyl-5 | |
| 634 | H | H | $NO_2$ | $NO_2$ | 2-Methyl-thiazolyl-5 | |
| 635 | H | H | $NO_2$ | $NO_2$ | Imidazolyl-4 | |
| 636 | H | H | $NO_2$ | $NO_2$ | Imidazolyl-5 | |
| 637 | H | H | $NO_2$ | $NO_2$ | 4-Methyl-imidazolyl-5 | |
| 638 | H | H | $NO_2$ | $NO_2$ | 1-Methyl-imidazolyl-5 | |
| 639 | H | H | $NO_2$ | $NO_2$ | 4-Nitro-imidazolyl-1 | |
| 640 | H | H | $NO_2$ | $NO_2$ | 2-Methyl-4-nitro-imidazolyl-1 | |
| 641 | H | H | $NO_2$ | $NO_2$ | 4,5-Dichlorimidazolyl-1 | |
| 642 | H | H | $NO_2$ | $NO_2$ | 1-Methyl-pyrryl-2 | |
| 643 | H | H | $NO_2$ | $NO_2$ | Isoxazolyl-3 | 171-174 |
| 644 | H | H | $NO_2$ | $NO_2$ | Isoxazolyl-4 | |
| 645 | H | H | $NO_2$ | $NO_2$ | Isoxazolyl-5 | |
| 646 | H | H | $NO_2$ | $NO_2$ | 5-Chlormethyl-isoxazolyl-3 | |
| 647 | H | H | $NO_2$ | $NO_2$ | Isothiazolyl-3 | |
| 648 | H | H | $NO_2$ | $NO_2$ | Isothiazolyl-4 | |
| 649 | H | H | $NO_2$ | $NO_2$ | Isothiazolyl-5 | |
| 650 | H | H | $NO_2$ | $NO_2$ | 4-Methyl-isothiazolyl-5 | |
| 651 | H | H | $NO_2$ | $NO_2$ | Pyrazolyl-4 | |
| 652 | H | H | $NO_2$ | $NO_2$ | Pyrazolyl-5 | |
| 653 | H | H | $NO_2$ | $NO_2$ | 4-Chlor-pyrazolyl-5 | |
| 654 | H | H | $NO_2$ | $NO_2$ | 1-Methyl-pyrazolyl-5 | |
| 655 | H | H | $NO_2$ | $NO_2$ | 1,2,3-Thiadiazolyl-5 | |
| 656 | H | H | $NO_2$ | $NO_2$ | 1,2,3-Oxadiazolyl-5 | |
| 657 | H | H | $NO_2$ | $NO_2$ | 1,2,3-Oxadiazolyl-4 | |
| 658 | H | H | $NO_2$ | $NO_2$ | 5-Methyl-1,2,3-oxadiazolyl-4 | |
| 659 | H | H | $NO_2$ | $NO_2$ | 4-Methyl-1,2,3-oxadiazolyl-5 | |
| 660 | H | H | $NO_2$ | $NO_2$ | 1,3,4-Thiadiazolyl-2 | |
| 661 | H | H | $NO_2$ | $NO_2$ | 2-Methyl-1,3,4-thiadiazolyl-5 | |
| 662 | H | H | $NO_2$ | $NO_2$ | 1,2,4-Thiadiazolyl-3 | |
| 663 | H | H | $NO_2$ | $NO_2$ | 1,2,4-Thiadiazolyl-5 | |
| 664 | H | H | $NO_2$ | $NO_2$ | 3-Methyl-1,2,4-thiadiazolyl-5 | |
| 665 | H | H | $NO_2$ | $NO_2$ | 1,2,4-Oxadiazolyl-3 | |
| 666 | H | H | $NO_2$ | $NO_2$ | 1,2,4-Oxadiazolyl-5 | |
| 667 | H | H | $NO_2$ | $NO_2$ | 1,2,4-Triazolyl-5 | |
| 668 | H | H | $NO_2$ | $NO_2$ | 1,2,4-Triazolyl-3 | |
| 669 | H | H | $NO_2$ | $NO_2$ | 1,3,4-Oxadiazolyl-5 | |
| 670 | H | H | $NO_2$ | $NO_2$ | 2-Methyl-1,3,4-oxadiazolyl-5 | |
| 671 | H | H | $NO_2$ | $NO_2$ | 2-Chlor-1,3,4-oxadiazolyl-5 | |
| 672 | H | H | $NO_2$ | $NO_2$ | 1,2,5-Oxadiazolyl-3 | |
| 673 | H | H | $NO_2$ | $NO_2$ | 3-Methyl-1,2,5-oxadiazolyl-4 | |

| Nr. | $R^1$ | $R^2$ | $R^3$ | $R^4$ | $R^5$ | Fp. [°C] |
|---|---|---|---|---|---|---|
| 674 | H | H | $NO_2$ | $NO_2$ | 1,2,5-Thiadiazolyl-3 | |
| 675 | H | H | $NO_2$ | $NO_2$ | 3-Methyl-1,2,5-thiadiazolyl-4 | |
| 676 | H | H | $NO_2$ | $NO_2$ | 2-Methyl-furyl-5 | |
| 677 | H | H | $NO_2$ | $NO_2$ | 2,5-Dimethylfuryl | 145-150 |
| 678 | H | H | $NO_2$ | $NO_2$ | 2-Chlor-furyl-5 | |
| 679 | H | H | $NO_2$ | $NO_2$ | 5-Methyl-oxazolyl-4 | |
| 680 | H | H | $NO_2$ | $NO_2$ | 4-Methyl-oxazolyl-2 | |
| 681 | H | H | $NO_2$ | $NO_2$ | 2-Bromfuryl-5 | |
| 682 | H | H | $NO_2$ | $NO_2$ | 2-Methyl-furyl-4 | |
| 683 | H | H | $NO_2$ | $NO_2$ | 5-Methyl-isoxazolyl-3 | |
| 684 | H | H | $NO_2$ | $NO_2$ | 4-Methyl-isoxazolyl-3 | |
| 685 | H | H | $NO_2$ | $NO_2$ | 5-Methyl-isothiazolyl-3 | |
| 686 | H | H | $NO_2$ | $NO_2$ | 4-Methyl-isothiazolyl-3 | |
| 687 | H | H | $NO_2$ | $NO_2$ | 4-Methyl-1,2,3-thiadiazolyl-5 | |
| 688 | H | H | $NO_2$ | $NO_2$ | 5-Methyl-1,2,3-thiadiazolyl-4 | |
| 689 | H | H | $NO_2$ | $NO_2$ | Pyrryl-1 | |
| 690 | H | H | $NO_2$ | $NO_2$ | Imidazolyl-1 | |
| 691 | H | H | $NO_2$ | $NO_2$ | Pyrazolyl-1 | |
| 692 | H | H | $NO_2$ | $NO_2$ | 1,3,4-Triazolyl-1 | |
| 693 | H | H | $NO_2$ | $NO_2$ | 1,2,4-Triazolyl-1 | |
| 694 | H | H | Cl | H | Thienyl-2 | |
| 695 | H | H | Cl | H | Thienyl-3 | |
| 696 | H | H | Cl | H | 2-Chlor-thienyl-3 | |
| 697 | H | H | Cl | H | 5-Chlor-thienyl-2 | |
| 698 | H | H | Cl | H | 5-Nitro-thienyl-2 | |
| 699 | H | H | Cl | H | 4-Methyl-thienyl-2 | |
| 700 | H | H | Cl | H | 3-Methyl-thienyl-2 | |
| 701 | H | H | Cl | H | 2-Methyl-thienyl-3 | |
| 702 | H | H | Cl | H | 3-Methyl-thienyl-4 | |
| 703 | H | H | Cl | H | 2,5-Dimethyl-thienyl-4 | |
| 704 | H | H | Cl | H | 2,3-Dichlor-thienyl-4 | |
| 705 | H | H | Cl | H | 5-Methyl-thienyl-2 | |
| 706 | H | H | Cl | H | Pyrryl-2 | |
| 707 | H | H | Cl | H | Pyrryl-3 | |
| 708 | H | H | Cl | H | 3-Methyl-pyrryl-2 | |
| 709 | H | H | Cl | H | 2-Methyl-pyrryl-3 | |
| 710 | H | H | Cl | H | 5-Chlor-pyrryl-2 | |
| 711 | H | H | Cl | H | Oxazolyl-2 | |
| 712 | H | H | Cl | H | Oxazolyl-4 | |
| 713 | H | H | Cl | H | Oxazolyl-5 | |
| 714 | H | H | Cl | H | 4-Methyl-oxazolyl-5 | |
| 715 | H | H | Cl | H | 2-Methyl-oxazolyl-5 | |

| Nr. | $R^1$ | $R^2$ | $R^3$ | $R^4$ | $R^5$ | Fp. [°C] |
|---|---|---|---|---|---|---|
| 716 | H | H | Cl | H | Thiazolyl-2 | |
| 717 | H | H | Cl | H | Thiazolyl-4 | |
| 718 | H | H | Cl | H | Thiazolyl-5 | |
| 719 | H | H | Cl | H | 4-Methyl-thiazolyl-5 | |
| 720 | H | H | Cl | H | 2-Methyl-thiazolyl-5 | |
| 721 | H | H | Cl | H | Imidazolyl-4 | |
| 722 | H | H | Cl | H | Imidazolyl-5 | |
| 723 | H | H | Cl | H | 4-Methyl-imidazolyl-5 | |
| 724 | H | H | Cl | H | 1-Methyl-imidazolyl-5 | |
| 725 | H | H | Cl | H | 4-Nitro-imidazolyl-1 | |
| 726 | H | H | Cl | H | 2-Methyl-4-nitro-imidazolyl-1 | |
| 727 | H | H | Cl | H | 4,5-Dichlorimidazolyl-1 | |
| 728 | H | H | Cl | H | 1-Methyl-pyrryl-2 | |
| 729 | H | H | Cl | H | Isoxazolyl-3 | 135-137 |
| 730 | H | H | Cl | H | Isoxazolyl-4 | |
| 731 | H | H | Cl | H | Isoxazolyl-5 | 125-128 |
| 732 | H | H | Cl | H | 5-Chlormethyl-isoxazolyl-3 | 130-133 |
| 733 | H | H | Cl | H | Isothiazolyl-3 | |
| 734 | H | H | Cl | H | Isothiazolyl-4 | |
| 735 | H | H | Cl | H | Isothiazolyl-5 | |
| 736 | H | H | Cl | H | 4-Methyl-isothiazolyl-5 | |
| 737 | H | H | Cl | H | Pyrazolyl-4 | |
| 738 | H | H | Cl | H | Pyrazolyl-5 | |
| 739 | H | H | Cl | H | 4-Chlor-pyrazolyl-5 | |
| 740 | H | H | Cl | H | 1-Methyl-pyrazolyl-5 | |
| 741 | H | H | Cl | H | 1,2,3-Thiadiazolyl-5 | |
| 742 | H | H | Cl | H | 1,2,3-Oxadiazolyl-5 | |
| 743 | H | H | Cl | H | 1,2,3-Oxadiazolyl-4 | |
| 744 | H | H | Cl | H | 5-Methyl-1,2,3-oxadiazolyl-4 | |
| 745 | H | H | Cl | H | 4-Methyl-1,2,3-oxadiazolyl-5 | |
| 746 | H | H | Cl | H | 1,3,4-Thiadiazolyl-2 | |
| 747 | H | H | Cl | H | 2-Methyl-1,3,4-thiadiazolyl-5 | |
| 748 | H | H | Cl | H | 1,2,4-Thiadiazolyl-3 | |
| 749 | H | H | Cl | H | 1,2,4-Thiadiazolyl-5 | |
| 750 | H | H | Cl | H | 3-Methyl-1,2,4-thiadiazolyl-5 | |
| 751 | H | H | Cl | H | 1,2,4-Oxadiazolyl-3 | |
| 752 | H | H | Cl | H | 1,2,4-Oxadiazolyl-5 | |
| 753 | H | H | Cl | H | 1,2,4-Triazolyl-5 | |
| 754 | H | H | Cl | H | 1,2,4-Triazolyl-3 | |
| 755 | H | H | Cl | H | 1,3,4-Oxadiazolyl-5 | |
| 756 | H | H | Cl | H | 2-Methyl-1,3,4-oxadiazolyl-5 | |
| 757 | H | H | Cl | H | 2-Chlor-1,3,4-oxadiazolyl-5 | |

| Nr. | $R^1$ | $R^2$ | $R^3$ | $R^4$ | $R^5$ | Fp. [°C] |
|---|---|---|---|---|---|---|
| 758 | H | H | Cl | H | 1,2,5-Oxadiazolyl-3 | |
| 759 | H | H | Cl | H | 3-Methyl-1,2,5-oxadiazolyl-4 | |
| 760 | H | H | Cl | H | 1,2,5-Thiadiazolyl-3 | |
| 761 | H | H | Cl | H | 3-Methyl-1,2,5-thiadiazolyl-4 | |
| 762 | H | H | Cl | H | 2-Methyl-furyl-5 | |
| 763 | H | H | Cl | H | 2,5-Dimethylfuryl-4 | 105-106 |
| 764 | H | H | Cl | H | 2-Chlor-furyl-5 | |
| 765 | H | H | Cl | H | 5-Methyl-oxazolyl-4 | |
| 766 | H | H | Cl | H | 4-Methyl-oxazolyl-2 | |
| 767 | H | H | Cl | H | 2-Bromfuryl-5 | |
| 768 | H | H | Cl | H | 2-Methyl-furyl-4 | |
| 769 | H | H | Cl | H | 5-Methyl-isoxazolyl-3 | |
| 770 | H | H | Cl | H | 4-Methyl-isoxazolyl-3 | |
| 771 | H | H | Cl | H | 5-Methyl-isothiazolyl-3 | |
| 772 | H | H | Cl | H | 4-Methyl-isothiazolyl-3 | |
| 773 | H | H | Cl | H | 5-Methyl-1,2,3-thiadiazolyl-4 | |
| 774 | H | H | Cl | H | Pyrryl-1 | |
| 775 | H | H | Cl | H | Imidazolyl-1 | |
| 776 | H | H | Cl | H | Pyrazolyl-1 | |
| 777 | H | H | Cl | H | 1,3,4-Triazolyl-1 | |
| 778 | H | H | Cl | H | 1,2,4-Triazolyl-1 | |
| 779 | H | H | Cl | $NO_2$ | Thienyl-2 | |
| 780 | H | H | Cl | $NO_2$ | Thienyl-3 | |
| 781 | H | H | Cl | $NO_2$ | 2-Chlor-thienyl-3 | |
| 782 | H | H | Cl | $NO_2$ | 5-Chlor-thienyl-2 | |
| 783 | H | H | Cl | $NO_2$ | 5-Nitro-thienyl-2 | |
| 784 | H | H | Cl | $NO_2$ | 4-Methyl-thienyl-2 | |
| 785 | H | H | Cl | $NO_2$ | 3-Methyl-thienyl-2 | |
| 786 | H | H | Cl | $NO_2$ | 2-Methyl-thienyl-3 | |
| 787 | H | H | Cl | $NO_2$ | 3-Methyl-thienyl-4 | |
| 788 | H | H | Cl | $NO_2$ | 2,5-Dimethyl-thienyl-4 | |
| 789 | H | H | Cl | $NO_2$ | 2,3-Dichlor-thienyl-4 | |
| 790 | H | H | Cl | $NO_2$ | 5-Methyl-thienyl-2 | |
| 791 | H | H | Cl | $NO_2$ | Pyrryl-2 | |
| 792 | H | H | Cl | $NO_2$ | Pyrryl-3 | |
| 793 | H | H | Cl | $NO_2$ | 3-Methyl-pyrryl-2 | |
| 794 | H | H | Cl | $NO_2$ | 2-Methyl-pyrryl-3 | |
| 795 | H | H | Cl | $NO_2$ | 5-Chlor-pyrryl-2 | |
| 796 | H | H | Cl | $NO_2$ | Oxazolyl-2 | |
| 797 | H | H | Cl | $NO_2$ | Oxazolyl-4 | |
| 798 | H | H | Cl | $NO_2$ | Oxazolyl-5 | |
| 799 | H | H | Cl | $NO_2$ | 1,2,3-Thiadiazolyl-4 | |

| Nr. | $R^1$ | $R^2$ | $R^3$ | $R^4$ | $R^5$ | Fp. [°C] |
|---|---|---|---|---|---|---|
| 800 | H | H | Cl | $NO_2$ | 4-Methyl-oxazolyl-5 | |
| 801 | H | H | Cl | $NO_2$ | 2-Methyl-oxazolyl-5 | |
| 802 | H | H | Cl | $NO_2$ | Thiazolyl-2 | |
| 803 | H | H | Cl | $NO_2$ | Thiazolyl-4 | |
| 804 | H | H | Cl | $NO_2$ | Thiazolyl-5 | |
| 805 | H | H | Cl | $NO_2$ | 4-Methyl-thiazolyl-5 | |
| 806 | H | H | Cl | $NO_2$ | 2-Methyl-thiazolyl-5 | |
| 807 | H | H | Cl | $NO_2$ | Imidazolyl-4 | |
| 808 | H | H | Cl | $NO_2$ | Imidazolyl-5 | |
| 809 | H | H | Cl | $NO_2$ | 4-Methyl-imidazolyl-5 | |
| 810 | H | H | Cl | $NO_2$ | 1-Methyl-imidazolyl-5 | |
| 811 | H | H | Cl | $NO_2$ | 4-Nitro-imidazolyl-5 | |
| 812 | H | H | Cl | $NO_2$ | 2-Methyl-4-nitro-imidazolyl-1 | |
| 813 | H | H | Cl | $NO_2$ | 4,5-Dichlorimidazolyl-1 | |
| 814 | H | H | Cl | $NO_2$ | 1-Methyl-pyrryl-2 | |
| 815 | H | H | Cl | $NO_2$ | Isoxazolyl-3 | 128-130 |
| 816 | H | H | Cl | $NO_2$ | Isoxazolyl-4 | |
| 817 | H | H | Cl | $NO_2$ | Isoxazolyl-5 | |
| 818 | H | H | Cl | $NO_2$ | 5-Chlormethyl-isoxazolyl-3 | 122-125 |
| 819 | H | H | Cl | $NO_2$ | Isothiazolyl-3 | |
| 820 | H | H | Cl | $NO_2$ | Isothiazolyl-4 | |
| 821 | H | H | Cl | $NO_2$ | Isothiazolyl-5 | |
| 822 | H | H | Cl | $NO_2$ | 4-Methyl-isothiazolyl-5 | |
| 823 | H | H | Cl | $NO_2$ | Pyrazolyl-4 | |
| 824 | H | H | Cl | $NO_2$ | Pyrazolyl-5 | |
| 825 | H | H | Cl | $NO_2$ | 4-Chlor-pyrazolyl-5 | |
| 826 | H | H | Cl | $NO_2$ | 1-Methyl-pyrazolyl-5 | |
| 827 | H | H | Cl | $NO_2$ | 1,2,3-Thiadiazolyl-5 | |
| 828 | H | H | Cl | $NO_2$ | 1,2,3-Oxadiazolyl-5 | |
| 829 | H | H | Cl | $NO_2$ | 1,2,3-Oxadiazolyl-4 | |
| 830 | H | H | Cl | $NO_2$ | 5-Methyl-1,2,3-oxadiazolyl-4 | |
| 831 | H | H | Cl | $NO_2$ | 4-Methyl-1,2,3-oxadiazolyl-5 | |
| 832 | H | H | Cl | $NO_2$ | 1,3,4-Thiadiazolyl-2 | |
| 833 | H | H | Cl | $NO_2$ | 2-Methyl-1,3,4-thiadiazolyl-5 | |
| 834 | H | H | Cl | $NO_2$ | 1,2,4-Thiadiazolyl-3 | |
| 835 | H | H | Cl | $NO_2$ | 1,2,4-Thiadiazolyl-5 | |
| 836 | H | H | Cl | $NO_2$ | 3-Methyl-1,2,4-thiadiazolyl-5 | |
| 837 | H | H | Cl | $NO_2$ | 1,2,4-Oxadiazolyl-3 | |
| 838 | H | H | Cl | $NO_2$ | 1,2,4-Oxadiazolyl-5 | |
| 839 | H | H | Cl | $NO_2$ | 1,2,4-Triazolyl-5 | |
| 840 | H | H | Cl | $NO_2$ | 1,2,4-Triazolyl-3 | |
| 841 | H | H | Cl | $NO_2$ | 1,3,4-Oxadiazolyl-5 | |

| Nr. | $R^1$ | $R^2$ | $R^3$ | $R^4$ | $R^5$ | Fp. [°C] |
|---|---|---|---|---|---|---|
| 842 | H | H | Cl | $NO_2$ | 2-Methyl-1,3,4-oxadiazolyl-5 | |
| 843 | H | H | Cl | $NO_2$ | 2-Chlor-1,3,4-oxadiazolyl-5 | |
| 844 | H | H | Cl | $NO_2$ | 1,2,5-Oxadiazolyl-3 | |
| 845 | H | H | Cl | $NO_2$ | 3-Methyl-1,2,5-oxadiazolyl-4 | |
| 846 | H | H | Cl | $NO_2$ | 1,2,5-Thiadiazolyl-3 | |
| 847 | H | H | Cl | $NO_2$ | 3-Methyl-1,2,5-thiadiazolyl-4 | |
| 848 | H | H | Cl | $NO_2$ | 2-Methyl-furyl-5 | |
| 849 | H | H | Cl | $NO_2$ | 2,5-Dimethylfuryl-4 | 141-144 |
| 850 | H | H | Cl | $NO_2$ | 2-Chlor-furyl-5 | |
| 851 | H | H | Cl | $NO_2$ | 5-Methyl-oxazolyl-4 | |
| 852 | H | H | Cl | $NO_2$ | 4-Methyl-oxazolyl-2 | |
| 853 | H | H | Cl | $NO_2$ | 2-Bromfuryl-5 | |
| 854 | H | H | Cl | $NO_2$ | 2-Methyl-furyl-4 | |
| 855 | H | H | Cl | $NO_2$ | 5-Methyl-isoxazolyl-3 | |
| 856 | H | H | Cl | $NO_2$ | 4-Methyl-isoxazolyl-3 | |
| 857 | H | H | Cl | $NO_2$ | 5-Methyl-isothiazolyl-3 | |
| 858 | H | H | Cl | $NO_2$ | 4-Methyl-isothiazolyl-3 | |
| 859 | H | H | Cl | $NO_2$ | 4-Methyl-1,2,3-thiadiazolyl-5 | 137-138 |
| 860 | H | H | Cl | $NO_2$ | 5-Methyl-1,2,3-thiadiazolyl-4 | |
| 861 | H | H | Cl | $NO_2$ | Pyrryl-1 | |
| 862 | H | H | Cl | $NO_2$ | Imidazolyl-1 | |
| 863 | H | H | Cl | $NO_2$ | Pyrazolyl-1 | |
| 864 | H | H | Cl | $NO_2$ | 1,3,4-Triazolyl-1 | |
| 865 | H | H | Cl | $NO_2$ | 1,2,4-Triazolyl-1 | |
| 866 | H | H | Br | $NO_2$ | Thienyl-2 | |
| 867 | H | H | Br | $NO_2$ | Thienyl-3 | |
| 868 | H | H | Br | $NO_2$ | 2-Chlor-thienyl-3 | |
| 869 | H | H | Br | $NO_2$ | 5-Chlor-thienyl-2 | |
| 870 | H | H | Br | $NO_2$ | 5-Nitro-thienyl-2 | |
| 871 | H | H | Br | $NO_2$ | 4-Methyl-thienyl-2 | |
| 872 | H | H | Br | $NO_2$ | 3-Methyl-thienyl-2 | |
| 873 | H | H | Br | $NO_2$ | 2-Methyl-thienyl-3 | |
| 874 | H | H | Br | $NO_2$ | 3-Methyl-thienyl-4 | |
| 875 | H | H | Br | $NO_2$ | 2,5-Dimethyl-thienyl-4 | |
| 876 | H | H | Br | $NO_2$ | 2,3-Dichlor-thienyl-4 | |
| 877 | H | H | Br | $NO_2$ | 5-Methyl-thienyl-2 | |
| 878 | H | H | Br | $NO_2$ | Pyrryl-2 | |
| 879 | H | H | Br | $NO_2$ | Pyrryl-3 | |
| 880 | H | H | Br | $NO_2$ | 3-Methyl-pyrryl-2 | |
| 881 | H | H | Br | $NO_2$ | 2-Methyl-pyrryl-3 | |
| 882 | H | H | Br | $NO_2$ | 5-Chlor-pyrryl-2 | |
| 883 | H | H | Br | $NO_2$ | Oxazolyl-2 | |

| Nr. | $R^1$ | $R^2$ | $R^3$ | $R^4$ | $R^5$ | Fp. [°C] |
|---|---|---|---|---|---|---|
| 884 | H | H | Br | $NO_2$ | Oxazolyl-4 | |
| 885 | H | H | Br | $NO_2$ | Oxazolyl-5 | |
| 886 | H | H | Br | $NO_2$ | 1,2,3-Thiadiazolyl | 172 Zers. |
| 887 | H | H | Br | $NO_2$ | 4-Methyl-oxazolyl-5 | |
| 888 | H | H | Br | $NO_2$ | 2-Methyl-oxazolyl-5 | |
| 889 | H | H | Br | $NO_2$ | Thiazolyl-2 | |
| 890 | H | H | Br | $NO_2$ | Thiazolyl-4 | |
| 891 | H | H | Br | $NO_2$ | Thiazolyl-5 | |
| 892 | H | H | Br | $NO_2$ | 4-Methyl-thiazolyl-5 | |
| 893 | H | H | Br | $NO_2$ | 2-Metyhl-thiazolyl-5 | |
| 894 | H | H | Br | $NO_2$ | Imidazolyl-4 | |
| 895 | H | H | Br | $NO_2$ | Imidazolyl-5 | |
| 896 | H | H | Br | $NO_2$ | 4-Methyl-imidazolyl-5 | |
| 897 | H | H | Br | $NO_2$ | 1-Methyl-imidazolyl-5 | |
| 898 | H | H | Br | $NO_2$ | 4-Nitro-imidazolyl-5 | |
| 899 | H | H | Br | $NO_2$ | 2-Methyl-4-nitro-imidazolyl-1 | |
| 900 | H | H | Br | $NO_2$ | 4,5-Dichlorimidazolyl-1 | |
| 901 | H | H | Br | $NO_2$ | 1-Methyl-pyrryl-2 | |
| 902 | H | H | Br | $NO_2$ | Isoxazolyl-3 | 159-162 |
| 903 | H | H | Br | $NO_2$ | Isoxazolyl-4 | |
| 904 | H | H | Br | $NO_2$ | Isoxazolyl-5 | 159-160 |
| 905 | H | H | Br | $NO_2$ | 5-Chlormethyl-isoxazolyl-3 | |
| 906 | H | H | Br | $NO_2$ | Isothiazolyl-3 | |
| 907 | H | H | Br | $NO_2$ | Isothiazolyl-4 | |
| 908 | H | H | Br | $NO_2$ | Isothiazolyl-5 | |
| 909 | H | H | Br | $NO_2$ | 4-Methyl-isothiazolyl-5 | |
| 910 | H | H | Br | $NO_2$ | Pyrazolyl-4 | |
| 911 | H | H | Br | $NO_2$ | Pyrazolyl-5 | |
| 912 | H | H | Br | $NO_2$ | 4-Chlor-pyrazolyl-5 | |
| 913 | H | H | Br | $NO_2$ | 1-Methyl-pyrazolyl-5 | |
| 914 | H | H | Br | $NO_2$ | 1,2,3-Thiadiazolyl-5 | |
| 915 | H | H | Br | $NO_2$ | 1,2,3-Oxadiazolyl-5 | |
| 916 | H | H | Br | $NO_2$ | 1,2,3-Oxadiazolyl-4 | |
| 917 | H | H | Br | $NO_2$ | 5-Methyl-1,2,3-oxadiazolyl-4 | |
| 918 | H | H | Br | $NO_2$ | 4-Methyl-1,2,3-oxadiazolyl-5 | |
| 919 | H | H | Br | $NO_2$ | 1,3,4-Thiadiazolyl-2 | |
| 920 | H | H | Br | $NO_2$ | 2-Methyl-1,3,4-thiadiazolyl-5 | |
| 921 | H | H | Br | $NO_2$ | 1,2,4-Thiadiazolyl-3 | |
| 922 | H | H | Br | $NO_2$ | 1,2,4-Thiadiazolyl-5 | |
| 923 | H | H | Br | $NO_2$ | 3-Methyl-1,2,4-thiadiazolyl-5 | |
| 924 | H | H | Br | $NO_2$ | 1,2,4-Oxadiazolyl-3 | |
| 925 | H | H | Br | $NO_2$ | 1,2,4-Oxadiazolyl-5 | |

| Nr. | $R^1$ | $R^2$ | $R^3$ | $R^4$ | $R^5$ | Fp. [°C] |
|---|---|---|---|---|---|---|
| 926 | H | H | Br | $NO_2$ | 1,2,4-Triazolyl-5 | |
| 927 | H | H | Br | $NO_2$ | 1,2,4-Triazolyl-3 | |
| 928 | H | H | Br | $NO_2$ | 1,3,4-Oxadiazolyl-5 | |
| 929 | H | H | Br | $NO_2$ | 2-Methyl-1,3,4-oxadiazolyl-5 | |
| 930 | H | H | Br | $NO_2$ | 2-Chlor-1,3,4-oxadiazolyl-5 | |
| 931 | H | H | Br | $NO_2$ | 1,2,5-Oxadiazolyl-3 | |
| 932 | H | H | Br | $NO_2$ | 3-Methyl-1,2,5-oxadiazolyl-4 | |
| 933 | H | H | Br | $NO_2$ | 1,2,5-Thiadiazolyl-3 | |
| 934 | H | H | Br | $NO_2$ | 3-Methyl-1,2,5-thiadiazolyl-4 | |
| 935 | H | H | Br | $NO_2$ | 2-Methyl-furyl-5 | |
| 936 | H | H | Br | $NO_2$ | 2,5-Dimethylfuryl-4 | 144-146 |
| 937 | H | H | Br | $NO_2$ | 2-Chlor-furyl-5 | |
| 938 | H | H | Br | $NO_2$ | 5-Methyl-oxazolyl-4 | |
| 939 | H | H | Br | $NO_2$ | 4-Methyl-oxazolyl-2 | |
| 940 | H | H | Br | $NO_2$ | 2-Bromfuryl-5 | |
| 941 | H | H | Br | $NO_2$ | 2-Methyl-furyl-4 | |
| 942 | H | H | Br | $NO_2$ | 5-Methyl-isoxazolyl-3 | |
| 943 | H | H | Br | $NO_2$ | 4-Methyl-isoxazolyl-3 | |
| 944 | H | H | Br | $NO_2$ | 5-Methyl-isothiazolyl-3 | |
| 945 | H | H | Br | $NO_2$ | 4-Methyl-isothiazolyl-3 | |
| 946 | H | H | Br | $NO_2$ | 4-Methyl-1,2,3-thiadiazolyl-5 | 148-150 |
| 947 | H | H | Br | $NO_2$ | 5-Methyl-1,2,3-thiadiazolyl-4 | |
| 948 | H | H | Br | $NO_2$ | Pyrryl-1 | |
| 949 | H | H | Br | $NO_2$ | Imidazolyl-1 | |
| 950 | H | H | Br | $NO_2$ | Pyrazolyl-1 | |
| 951 | H | H | Br | $NO_2$ | 1,3,4-Triazolyl-1 | |
| 952 | H | H | Br | $NO_2$ | 1,2,4-Triazolyl-1 | |
| 953 | H | H | Br | H | Thienyl-2 | |
| 954 | H | H | Br | H | Thienyl-3 | |
| 955 | H | H | Br | H | 2-Chlor-thienyl-3 | |
| 956 | H | H | Br | H | 5-Chlor-thienyl-2 | |
| 957 | H | H | Br | H | 5-Nitro-thienyl-2 | |
| 958 | H | H | Br | H | 4-Methyl-thienyl-2 | |
| 959 | H | H | Br | H | 3-Methyl-thienyl-2 | |
| 960 | H | H | Br | H | 2-Methyl-thienyl-3 | |
| 961 | H | H | Br | H | 3-Methyl-thienyl-4 | |
| 962 | H | H | Br | H | 2,5-Dimethyl-thienyl-4 | |
| 963 | H | H | Br | H | 2,3-Dichlor-thienyl-4 | |
| 964 | H | H | Br | H | 5-Methyl-thienyl-2 | |
| 965 | H | H | Br | H | Pyrryl-2 | |
| 966 | H | H | Br | H | Pyrryl-3 | |
| 967 | H | H | Br | H | 3-Methyl-pyrryl-2 | |

| Nr. | $R^1$ | $R^2$ | $R^3$ | $R^4$ | $R^5$ | Fp. [°C] |
|---|---|---|---|---|---|---|
| 968 | H | H | Br | H | 2-Methyl-pyrryl-3 | |
| 969 | H | H | Br | H | 5-Chlor-pyrryl-2 | |
| 970 | H | H | Br | H | Oxazolyl-2 | |
| 971 | H | H | Br | H | Oxazolyl-4 | |
| 972 | H | H | Br | H | Oxazolyl-5 | |
| 973 | H | H | Br | H | 1,2,3-Thiadiazolyl-4 | 158-159 |
| 974 | H | H | Br | H | 4-Methyl-oxazolyl-5 | |
| 975 | H | H | Br | H | 2-Methyl-oxazolyl-5 | |
| 976 | H | H | Br | H | Thiazolyl-2 | |
| 977 | H | H | Br | H | Thiazolyl-4 | |
| 978 | H | H | Br | H | Thiazolyl-5 | |
| 979 | H | H | Br | H | 4-Methyl-thiazolyl-5 | |
| 980 | H | H | Br | H | 2-Methyl-thiazolyl-5 | |
| 981 | H | H | Br | H | Imidazolyl-4 | |
| 982 | H | H | Br | H | Imidazolyl-5 | |
| 983 | H | H | Br | H | 4-Methyl-imidazolyl-5 | |
| 984 | H | H | Br | H | 1-Methyl-imidazolyl-5 | |
| 985 | H | H | Br | H | 4-Nitro-imidazolyl-1 | |
| 986 | H | H | Br | H | 2-Methyl-4-nitro-imidazolyl-1 | |
| 987 | H | H | Br | H | 4,5-Dichlorimidazolyl-1 | |
| 988 | H | H | Br | H | 1-Methyl-pyrryl-2 | |
| 989 | H | H | Br | H | Isoxazolyl-3 | 128-129 |
| 990 | H | H | Br | H | Isoxazolyl-4 | |
| 991 | H | H | Br | H | Isoxazolyl-5 | 135-137 |
| 992 | H | H | Br | H | 5-Chlormethyl-isoxazolyl-3 | 134-136 |
| 993 | H | H | Br | H | Isothiazolyl-3 | |
| 994 | H | H | Br | H | Isothiazolyl-4 | |
| 995 | H | H | Br | H | Isothiazolyl-5 | |
| 996 | H | H | Br | H | 4-Methyl-isothiazolyl-5 | |
| 997 | H | H | Br | H | Pyrazolyl-4 | |
| 998 | H | H | Br | H | Pyrazolyl-5 | |
| 999 | H | H | Br | H | 4-Chlor-pyrazolyl-5 | |
| 1000 | H | H | Br | H | 1-Methyl-pyrazolyl-5 | |
| 1001 | H | H | Br | H | 1,2,3-Thiadiazolyl-5 | |
| 1002 | H | H | Br | H | 1,2,3-Oxadiazolyl-5 | |
| 1003 | H | H | Br | H | 1,2,3-Oxadiazolyl-4 | |
| 1004 | H | H | Br | H | 5-Methyl-1,2,3-oxadiazolyl-4 | |
| 1005 | H | H | Br | H | 4-Methyl-1,2,3-oxadiazolyl-5 | |
| 1006 | H | H | Br | H | 1,3,4-Thiadiazolyl-2 | |
| 1007 | H | H | Br | H | 2-Methyl-1,3,4-thiadiazolyl-5 | |
| 1008 | H | H | Br | H | 1,2,4-Thiadiazolyl-3 | |
| 1009 | H | H | Br | H | 1,2,4-Thiadiazolyl-5 | |

| Nr. | $R^1$ | $R^2$ | $R^3$ | $R^4$ | $R^5$ | Fp. [°C] |
|---|---|---|---|---|---|---|
| 1010 | H | H | Br | H | 3-Methyl-1,2,4-thiadiazolyl-5 | |
| 1011 | H | H | Br | H | 1,2,4-Oxadiazolyl-3 | |
| 1012 | H | H | Br | H | 1,2,4-Oxadiazolyl-5 | |
| 1013 | H | H | Br | H | 1,2,4-Triazolyl-5 | |
| 1014 | H | H | Br | H | 1,2,4-Triazolyl-3 | |
| 1015 | H | H | Br | H | 1,3,4-Oxadiazolyl-5 | |
| 1016 | H | H | Br | H | 2-Methyl-1,3,4-oxadiazolyl-5 | |
| 1017 | H | H | Br | H | 2-Chlor-1,3,4-oxadiazolyl-5 | |
| 1018 | H | H | Br | H | 1,2,5-Oxadiazolyl-3 | |
| 1019 | H | H | Br | H | 3-Methyl-1,2,5-oxadiazolyl-4 | |
| 1020 | H | H | Br | H | 1,2,5-Thiadiazolyl-3 | |
| 1021 | H | H | Br | H | 3-Methyl-1,2,5-thiadiazolyl-4 | |
| 1022 | H | H | Br | H | 2-Methyl-furyl-5 | |
| 1023 | H | H | Br | H | 2,5-Dimethylfuryl-4 | 116-117 |
| 1024 | H | H | Br | H | 2-Chlor-furyl-5 | |
| 1025 | H | H | Br | H | 5-Methyl-oxazolyl-4 | |
| 1026 | H | H | Br | H | 4-Methyl-oxazolyl-2 | |
| 1027 | H | H | Br | H | 2-Bromfuryl-5 | |
| 1028 | H | H | Br | H | 2-Methyl-furyl-4 | |
| 1029 | H | H | Br | H | 5-Methyl-isoxazolyl-3 | |
| 1030 | H | H | Br | H | 4-Methyl-isoxazolyl-3 | |
| 1031 | H | H | Br | H | 5-Methyl-isothiazolyl-3 | |
| 1032 | H | H | Br | H | 4-Methyl-isothiazolyl-3 | |
| 1033 | H | H | Br | H | 4-Methyl-1,2,3-thiadiazolyl-5 | 120-121 |
| 1034 | H | H | Br | H | 5-Methyl-1,2,3-thiadiazolyl-4 | |
| 1035 | H | H | Br | H | Pyrryl-1 | |
| 1036 | H | H | Br | H | Imidazolyl-1 | |
| 1037 | H | H | Br | H | Pyrazolyl-1 | |
| 1038 | H | H | Br | H | 1,3,4-Triazolyl-1 | |
| 1039 | H | H | Br | H | 1,2,4-Triazolyl-1 | |
| 1040 | $CH_3$ | H | $NO_2$ | $NO_2$ | Thienyl-2 | |
| 1041 | $CH_3$ | H | $NO_2$ | $NO_2$ | Thienyl-3 | |
| 1042 | $CH_3$ | H | $NO_2$ | $NO_2$ | 2,5-Dimethyl-thienyl-4 | |
| 1043 | $CH_3$ | H | $NO_2$ | $NO_2$ | 5-Methyl-thienyl-2 | |
| 1044 | $CH_3$ | H | $NO_2$ | $NO_2$ | Pyrryl-2 | |
| 1045 | $CH_3$ | H | $NO_2$ | $NO_2$ | Pyrryl-3 | |
| 1046 | $CH_3$ | H | $NO_2$ | $NO_2$ | Oxazolyl-2 | |
| 1047 | $CH_3$ | H | $NO_2$ | $NO_2$ | Oxazolyl-4 | |
| 1048 | $CH_3$ | H | $NO_2$ | $NO_2$ | Oxazolyl-5 | |
| 1049 | $CH_3$ | H | $NO_2$ | $NO_2$ | 1,2,3-Thiadiazolyl-4 | |
| 1050 | $CH_3$ | H | $NO_2$ | $NO_2$ | 4-Methyl-oxazolyl-5 | |
| 1051 | $CH_3$ | H | $NO_2$ | $NO_2$ | Thiazolyl-2 | |

| Nr. | $R^1$ | $R^2$ | $R^3$ | $R^4$ | $R^5$ | Fp. [°C] |
|---|---|---|---|---|---|---|
| 1052 | $CH_3$ | H | $NO_2$ | $NO_2$ | Thiazolyl-4 | |
| 1053 | $CH_3$ | H | $NO_2$ | $NO_2$ | Thiazolyl-5 | |
| 1054 | $CH_3$ | H | $NO_2$ | $NO_2$ | 4-Methyl-thiazolyl-5 | |
| 1055 | $CH_3$ | H | $NO_2$ | $NO_2$ | Imidazolyl-4 | |
| 1056 | $CH_3$ | H | $NO_2$ | $NO_2$ | Imidazolyl-5 | |
| 1057 | $CH_3$ | H | $NO_2$ | $NO_2$ | 4-Methyl-imidazolyl-5 | |
| 1058 | $CH_3$ | H | $NO_2$ | $NO_2$ | 1-Methyl-imidazolyl-5 | |
| 1059 | $CH_3$ | H | $NO_2$ | $NO_2$ | 4-Nitro-imidazolyl-1 | |
| 1060 | $CH_3$ | H | $NO_2$ | $NO_2$ | 2-Methyl-4-nitro-imidazolyl-1 | |
| 1061 | $CH_3$ | H | $NO_2$ | $NO_2$ | 4,5-Dichlorimidazolyl-1 | |
| 1062 | $CH_3$ | H | $NO_2$ | $NO_2$ | 1-Methyl-pyrryl-2 | |
| 1063 | $CH_3$ | H | $NO_2$ | $NO_2$ | Isoxazolyl-3 | |
| 1064 | $CH_3$ | H | $NO_2$ | $NO_2$ | Isoxazolyl-4 | |
| 1065 | $CH_3$ | H | $NO_2$ | $NO_2$ | Isoxazolyl-5 | |
| 1066 | $CH_3$ | H | $NO_2$ | $NO_2$ | 5-Chlormethyl-isoxazolyl-3 | |
| 1067 | $CH_3$ | H | $NO_2$ | $NO_2$ | Isothiazolyl-3 | |
| 1068 | $CH_3$ | H | $NO_2$ | $NO_2$ | Isothiazolyl-4 | |
| 1069 | $CH_3$ | H | $NO_2$ | $NO_2$ | Isothiazolyl-5 | |
| 1070 | $CH_3$ | H | $NO_2$ | $NO_2$ | 4-Methyl-isothiazolyl-5 | |
| 1071 | $CH_3$ | H | $NO_2$ | $NO_2$ | Pyrazolyl-4 | |
| 1072 | $CH_3$ | H | $NO_2$ | $NO_2$ | Pyrazolyl-5 | |
| 1073 | $CH_3$ | H | $NO_2$ | $NO_2$ | 1-Methyl-pyrazolyl-5 | |
| 1074 | $CH_3$ | H | $NO_2$ | $NO_2$ | 1,2,3-Thiadiazolyl-5 | |
| 1075 | $CH_3$ | H | $NO_2$ | $NO_2$ | 1,2,3-Oxadiazolyl-5 | |
| 1076 | $CH_3$ | H | $NO_2$ | $NO_2$ | 1,2,3-Oxadiazolyl-4 | |
| 1077 | $CH_3$ | H | $NO_2$ | $NO_2$ | 5-Methyl-1,2,3-oxadiazolyl-4 | |
| 1078 | $CH_3$ | H | $NO_2$ | $NO_2$ | 4-Methyl-1,2,3-oxadiazolyl-5 | |
| 1079 | $CH_3$ | H | $NO_2$ | $NO_2$ | 1,3,4-Thiadiazolyl-2 | |
| 1080 | $CH_3$ | H | $NO_2$ | $NO_2$ | 2-Methyl-1,3,4-thiadiazolyl-5 | |
| 1081 | $CH_3$ | H | $NO_2$ | $NO_2$ | 1,2,4-Thiadiazolyl-3 | |
| 1082 | $CH_3$ | H | $NO_2$ | $NO_2$ | 1,2,4-Thiadiazolyl-5 | |
| 1083 | $CH_3$ | H | $NO_2$ | $NO_2$ | 3-Methyl-1,2,4-thiadiazolyl-5 | |
| 1084 | $CH_3$ | H | $NO_2$ | $NO_2$ | 1,2,4-Oxadiazolyl-3 | |
| 1085 | $CH_3$ | H | $NO_2$ | $NO_2$ | 1,2,4-Oxadiazolyl-5 | |
| 1086 | $CH_3$ | H | $NO_2$ | $NO_2$ | 1,2,4-Triazolyl-5 | |
| 1087 | $CH_3$ | H | $NO_2$ | $NO_2$ | 1,2,4-Triazolyl-3 | |
| 1088 | $CH_3$ | H | $NO_2$ | $NO_2$ | 1,3,4-Oxadiazolyl-5 | |
| 1089 | $CH_3$ | H | $NO_2$ | $NO_2$ | 2-Methyl-1,3,4-oxadiazolyl-5 | |
| 1090 | $CH_3$ | H | $NO_2$ | $NO_2$ | 1,2,5-Oxadiazolyl-3 | |
| 1091 | $CH_3$ | H | $NO_2$ | $NO_2$ | 3-Methyl-1,2,5-oxadiazolyl-4 | |
| 1092 | $CH_3$ | H | $NO_2$ | $NO_2$ | 1,2,5-Thiadiazolyl-3 | |
| 1093 | $CH_3$ | H | $NO_2$ | $NO_2$ | 3-Methyl-1,2,5-thiadiazolyl-4 | |

| Nr. | $R^1$ | $R^2$ | $R^3$ | $R^4$ | $R^5$ | Fp. [°C] |
|---|---|---|---|---|---|---|
| 1094 | $CH_3$ | H | $NO_2$ | $NO_2$ | 2-Methyl-furyl-5 | |
| 1095 | $CH_3$ | H | $NO_2$ | $NO_2$ | 2,5-Dimethyl-furyl-4 | 156-160 |
| 1096 | $CH_3$ | H | $NO_2$ | $NO_2$ | 2-Chlor-furyl-5 | |
| 1097 | $CH_3$ | H | $NO_2$ | $NO_2$ | 5-Methyl-oxazolyl-4 | |
| 1098 | $CH_3$ | H | $NO_2$ | $NO_2$ | 4-Methyl-oxazolyl-2 | |
| 1099 | $CH_3$ | H | $NO_2$ | $NO_2$ | 5-Methyl-isoxazolyl-3 | |
| 1100 | $CH_3$ | H | $NO_2$ | $NO_2$ | 4-Methyl-isoxazolyl-3 | |
| 1101 | $CH_3$ | H | $NO_2$ | $NO_2$ | 5-Methyl-isothiazolyl-3 | |
| 1102 | $CH_3$ | H | $NO_2$ | $NO_2$ | 4-Methyl-isothiazolyl-3 | |
| 1103 | $CH_3$ | H | $NO_2$ | $NO_2$ | 4-Methyl-1,2,3-thiadiazolyl-5 | |
| 1104 | $CH_3$ | H | $NO_2$ | $NO_2$ | 5-Methyl-1,2,3-thiadiazolyl-4 | |
| 1105 | $CH_3$ | H | $NO_2$ | $NO_2$ | Pyrryl-1 | |
| 1106 | $CH_3$ | H | $NO_2$ | $NO_2$ | Imidazolyl-1 | |
| 1107 | $CH_3$ | H | $NO_2$ | $NO_2$ | Pyrazolyl-1 | |
| 1108 | $CH_3$ | H | $NO_2$ | $NO_2$ | 1,3,4-Triazolyl-1 | |
| 1109 | $CH_3$ | H | $NO_2$ | $NO_2$ | 1,2,4-Triazolyl-1 | |
| 1110 | $CH_3$ | H | $NO_2$ | Cl | Thienyl-2 | |
| 1111 | $CH_3$ | H | $NO_2$ | Cl | Thienyl-3 | |
| 1112 | $CH_3$ | H | $NO_2$ | Cl | 2,5-Dimethyl-thienyl-4 | |
| 1113 | $CH_3$ | H | $NO_2$ | Cl | 5-Methyl-thienyl-2 | |
| 1114 | $CH_3$ | H | $NO_2$ | Cl | Pyrryl-2 | |
| 1115 | $CH_3$ | H | $NO_2$ | Cl | Pyrryl-3 | |
| 1116 | $CH_3$ | H | $NO_2$ | Cl | Oxazolyl-2 | |
| 1117 | $CH_3$ | H | $NO_2$ | Cl | Oxazolyl-4 | |
| 1118 | $CH_3$ | H | $NO_2$ | Cl | Oxazolyl-5 | |
| 1119 | $CH_3$ | H | $NO_2$ | Cl | 1,2,3-Thiadiazolyl-4 | |
| 1120 | $CH_3$ | H | $NO_2$ | Cl | 4-Methyl-oxazolyl-5 | |
| 1121 | $CH_3$ | H | $NO_2$ | Cl | Thiazolyl-2 | |
| 1122 | $CH_3$ | H | $NO_2$ | Cl | Thiazolyl-4 | |
| 1123 | $CH_3$ | H | $NO_2$ | Cl | Thiazolyl-5 | |
| 1124 | $CH_3$ | H | $NO_2$ | Cl | 4-Methyl-thiazolyl-5 | |
| 1125 | $CH_3$ | H | $NO_2$ | Cl | Imidazolyl-4 | |
| 1126 | $CH_3$ | H | $NO_2$ | Cl | Imidazolyl-5 | |
| 1127 | $CH_3$ | H | $NO_2$ | Cl | 4-Methyl-imidazolyl-5 | |
| 1128 | $CH_3$ | H | $NO_2$ | Cl | 1-Methyl-imidazolyl-5 | |
| 1129 | $CH_3$ | H | $NO_2$ | Cl | 4-Nitro-imidazolyl-1 | |
| 1130 | $CH_3$ | H | $NO_2$ | Cl | 2-Methyl-4-nitro-imidazolyl-1 | |
| 1131 | $CH_3$ | H | $NO_2$ | Cl | 4,5-Dichlorimidazolyl-1 | |
| 1132 | $CH_3$ | H | $NO_2$ | Cl | 1-Methyl-pyrryl-2 | |
| 1133 | $CH_3$ | H | $NO_2$ | Cl | Isoxazolyl-3 | |
| 1134 | $CH_3$ | H | $NO_2$ | Cl | Isoxazolyl-4 | |
| 1135 | $CH_3$ | H | $NO_2$ | Cl | Isoxazolyl-5 | |

| Nr. | $R^1$ | $R^2$ | $R^3$ | $R^4$ | $R^5$ | Fp. [°C] |
|---|---|---|---|---|---|---|
| 1136 | $CH_3$ | H | $NO_2$ | Cl | 5-Chlormethyl-isoxazolyl-3 | |
| 1137 | $CH_3$ | H | $NO_2$ | Cl | Isothiazolyl-3 | |
| 1138 | $CH_3$ | H | $NO_2$ | Cl | Isothiazolyl-4 | |
| 1139 | $CH_3$ | H | $NO_2$ | Cl | Isothiazolyl-5 | |
| 1140 | $CH_3$ | H | $NO_2$ | Cl | 4-Methyl-isothiazolyl-5 | |
| 1141 | $CH_3$ | H | $NO_2$ | Cl | Pyrazolyl-4 | |
| 1142 | $CH_3$ | H | $NO_2$ | Cl | Pyrazolyl-5 | |
| 1143 | $CH_3$ | H | $NO_2$ | Cl | 1-Methyl-pyrazolyl-5 | |
| 1144 | $CH_3$ | H | $NO_2$ | Cl | 1,2,3-Thiadiazolyl-5 | |
| 1145 | $CH_3$ | H | $NO_2$ | Cl | 1,2,3-Oxadiazolyl-5 | |
| 1146 | $CH_3$ | H | $NO_2$ | Cl | 1,2,3-Oxadiazolyl-4 | |
| 1147 | $CH_3$ | H | $NO_2$ | Cl | 5-Methyl-1,2,3-oxadiazolyl-4 | |
| 1148 | $CH_3$ | H | $NO_2$ | Cl | 4-Methyl-1,2,3-oxadiazolyl-5 | |
| 1149 | $CH_3$ | H | $NO_2$ | Cl | 1,3,4-Thiadiazolyl-2 | |
| 1150 | $CH_3$ | H | $NO_2$ | Cl | 2-Methyl-1,3,4-thiadiazolyl-5 | |
| 1151 | $CH_3$ | H | $NO_2$ | Cl | 1,2,4-Thiadiazolyl-3 | |
| 1152 | $CH_3$ | H | $NO_2$ | Cl | 1,2,4-Thiadiazolyl-5 | |
| 1153 | $CH_3$ | H | $NO_2$ | Cl | 3-Methyl-1,2,4-thiadiazolyl-5 | |
| 1154 | $CH_3$ | H | $NO_2$ | Cl | 1,2,4-Oxadiazolyl-3 | |
| 1155 | $CH_3$ | H | $NO_2$ | Cl | 1,2,4-Oxadiazolyl-5 | |
| 1156 | $CH_3$ | H | $NO_2$ | Cl | 1,2,4-Triazolyl-5 | |
| 1157 | $CH_3$ | H | $NO_2$ | Cl | 1,2,4-Triazolyl-3 | |
| 1158 | $CH_3$ | H | $NO_2$ | Cl | 1,3,4-Oxadiazolyl-5 | |
| 1159 | $CH_3$ | H | $NO_2$ | Cl | 2-Methyl-1,3,4-oxadiazolyl-5 | |
| 1160 | $CH_3$ | H | $NO_2$ | Cl | 1,2,5-Oxadiazolyl-3 | |
| 1161 | $CH_3$ | H | $NO_2$ | Cl | 3-Methyl-1,2,5-oxadiazolyl-4 | |
| 1162 | $CH_3$ | H | $NO_2$ | Cl | 1,2,5-Thiadiazolyl-3 | |
| 1163 | $CH_3$ | H | $NO_2$ | Cl | 3-Methyl-1,2,5-thiadiazolyl-4 | |
| 1164 | $CH_3$ | H | $NO_2$ | Cl | 2-Methyl-furyl-5 | |
| 1165 | $CH_3$ | H | $NO_2$ | Cl | 2,5-Dimethyl-furyl-4 | |
| 1166 | $CH_3$ | H | $NO_2$ | Cl | 2-Chlor-furyl-5 | |
| 1167 | $CH_3$ | H | $NO_2$ | Cl | 5-Methyl-oxazolyl-4 | |
| 1168 | $CH_3$ | H | $NO_2$ | Cl | 4-Methyl-oxazolyl-2 | |
| 1169 | $CH_3$ | H | $NO_2$ | Cl | 5-Methyl-isoxazolyl-3 | |
| 1170 | $CH_3$ | H | $NO_2$ | Cl | 4-Methyl-isoxazolyl-3 | |
| 1171 | $CH_3$ | H | $NO_2$ | Cl | 5-Methyl-isothiazolyl-3 | |
| 1172 | $CH_3$ | H | $NO_2$ | Cl | 4-Methyl-isothiazolyl-3 | |
| 1173 | $CH_3$ | H | $NO_2$ | Cl | 4-Methyl-1,2,3-thiadiazolyl-5 | |
| 1174 | $CH_3$ | H | $NO_2$ | Cl | 5-Methyl-1,2,3-thiadiazolyl-4 | |
| 1175 | $CH_3$ | H | $NO_2$ | Cl | Pyrryl-1 | |
| 1176 | $CH_3$ | H | $NO_2$ | Cl | Imidazolyl-1 | |
| 1177 | $CH_3$ | H | $NO_2$ | Cl | Pyrazolyl-1 | |

| Nr. | $R^1$ | $R^2$ | $R^3$ | $R^4$ | $R^5$ | Fp. [°C] |
|---|---|---|---|---|---|---|
| 1178 | $CH_3$ | H | $NO_2$ | Cl | 1,3,4-Triazolyl-1 | |
| 1179 | $CH_3$ | H | $NO_2$ | Cl | 1,2,4-Triazolyl-1 | |

| Nr. | $R^1$ | $R^2$ | $R^3$ | $R^4$ | $R^5$ | Fp. [°C] |
|---|---|---|---|---|---|---|
| 1247 | H | H | H | J | Thienyl-2 | |
| 1248 | H | H | H | J | Thienyl-3 | |
| 1249 | H | H | H | J | 2,5-Dimethyl-thienyl-4 | |
| 1250 | H | H | H | J | 5-Methyl-thienyl-2 | |
| 1251 | H | H | H | J | Pyrryl-2 | |
| 1252 | H | H | H | J | Pyrryl-3 | |
| 1253 | H | H | H | J | Oxazolyl-2 | |
| 1254 | H | H | H | J | Oxazolyl-4 | |
| 1255 | H | H | H | J | Oxazolyl-5 | |
| 1256 | H | H | H | J | 1,2,3-Thiadiazolyl-4 | 177-178 |
| 1257 | H | H | H | J | 4-Methyl-oxazolyl-5 | |
| 1258 | H | H | H | J | Thiazolyl-2 | |
| 1259 | H | H | H | J | Thiazolyl-4 | |
| 1260 | H | H | H | J | Thiazolyl-5 | |
| 1261 | H | H | H | J | 4-Methyl-thiazolyl-5 | |

| Nr. | $R^1$ | $R^2$ | $R^3$ | $R^4$ | $R^5$ | Fp. [°C] |
|---|---|---|---|---|---|---|
| 1262 | H | H | H | J | Imidazolyl-4 | |
| 1263 | H | H | H | J | Imidazolyl-5 | |
| 1264 | H | H | H | J | 4-Methyl-imidazolyl-5 | |
| 1265 | H | H | H | J | 1-Methyl-imidazolyl-5 | |
| 1266 | H | H | H | J | 4-Nitro-imidazolyl-1 | |
| 1267 | H | H | H | J | 2-Methyl-4-nitro-imidazolyl-1 | |
| 1268 | H | H | H | J | 4,5-Dichlorimidazolyl-1 | |
| 1269 | H | H | H | J | 1-Methyl-pyrryl-2 | |
| 1270 | H | H | H | J | Isoxazolyl-3 | 115-117 |
| 1271 | H | H | H | J | Isoxazolyl-4 | |
| 1272 | H | H | H | J | Isoxazolyl-5 | |
| 1273 | H | H | H | J | 5-Chlormethyl-isoxazolyl-3 | |
| 1274 | H | H | H | J | Isothiazolyl-3 | |
| 1275 | H | H | H | J | Isothiazolyl-4 | |
| 1276 | H | H | H | J | Isothiazolyl-5 | |
| 1277 | H | H | H | J | 4-Methyl-isothiazolyl-5 | |
| 1278 | H | H | H | J | Pyrazolyl-4 | |
| 1279 | H | H | H | J | Pyrazolyl-5 | |
| 1280 | H | H | H | J | 1-Methyl-pyrazolyl-5 | |
| 1281 | H | H | H | J | 1,2,3-Thiadiazolyl-5 | |
| 1282 | H | H | H | J | 1,2,3-Oxadiazolyl-5 | |
| 1283 | H | H | H | J | 1,2,3-Oxadiazolyl-4 | |
| 1284 | H | H | H | J | 5-Methyl-1,2,3-oxadiazolyl-4 | |
| 1285 | H | H | H | J | 4-Methyl-1,2,3-oxadiazolyl-5 | |
| 1286 | H | H | H | J | 1,3,4-Thiadiazolyl-2 | |
| 1287 | H | H | H | J | 2-Methyl-1,3,4-thiadiazolyl-5 | |
| 1288 | H | H | H | J | 1,2,4-Thiadiazolyl-3 | |
| 1289 | H | H | H | J | 1,2,4-Thiadiazolyl-5 | |
| 1290 | H | H | H | J | 3-Methyl-1,2,4-thiadiazolyl-5 | |
| 1291 | H | H | H | J | 1,2,4-Oxadiazolyl-3 | |
| 1292 | H | H | H | J | 1,2,4-Oxadiazolyl-5 | |
| 1293 | H | H | H | J | 1,2,4-Triazolyl-5 | |
| 1294 | H | H | H | J | 1,2,4-Triazolyl-3 | |
| 1295 | H | H | H | J | 1,3,4-Oxadiazolyl-5 | |
| 1296 | H | H | H | J | 2-Methyl-1,3,4-oxadiazolyl-5 | |
| 1297 | H | H | H | J | 1,2,5-Oxadiazolyl-3 | |
| 1298 | H | H | H | J | 3-Methyl-1,2,5-oxadiazolyl-4 | |
| 1299 | H | H | H | J | 1,2,5-Thiadiazolyl-3 | |
| 1300 | H | H | H | J | 3-Methyl-1,2,5-thiadiazolyl-4 | |
| 1301 | H | H | H | J | 2-Methyl-furyl-5 | |
| 1302 | H | H | H | J | 2,5-Dimethyl-furyl-4 | |
| 1303 | H | H | H | J | 2-Chlor-furyl-5 | |

| Nr. | $R^1$ | $R^2$ | $R^3$ | $R^4$ | $R^5$ | Fp. [°C] |
|---|---|---|---|---|---|---|
| 1304 | H | H | H | J | 5-Methyl-oxazolyl-4 | |
| 1305 | H | H | H | J | 4-Methyl-oxazolyl-2 | |
| 1306 | H | H | H | J | 5-Methyl-isoxazolyl-3 | |
| 1307 | H | H | H | J | 4-Methyl-isoxazolyl-3 | |
| 1308 | H | H | H | J | 5-Methyl-isothiazolyl-3 | |
| 1309 | H | H | H | J | 4-Methyl-isothiazolyl-3 | |
| 1310 | H | H | H | J | 4-Methyl-1,2,3-thiadiazolyl-5 | |
| 1311 | H | H | H | J | 5-Methyl-1,2,3-thiadiazolyl-4 | |
| 1312 | H | H | H | J | Pyrryl-1 | |
| 1313 | H | H | H | J | Imidazolyl-1 | |
| 1314 | H | H | H | J | Pyrazolyl-1 | |
| 1315 | H | H | H | J | 1,3,4-Triazolyl-1 | |
| 1316 | H | H | H | J | 1,2,4-Triazolyl-1 | |
| 1317 | H | H | H | F | Thienyl-2 | |
| 1318 | H | H | H | F | Thienyl-3 | |
| 1319 | H | H | H | F | 2,5-Dimethyl-thienyl-4 | |
| 1320 | H | H | H | F | 5-Methyl-thienyl-2 | |
| 1321 | H | H | H | F | Pyrryl-2 | |
| 1322 | H | H | H | F | Pyrryl-3 | |
| 1323 | H | H | H | F | Oxazolyl-2 | |
| 1324 | H | H | H | F | Oxazolyl-4 | |
| 1325 | H | H | H | F | Oxazolyl-5 | |
| 1326 | H | H | H | F | 1,2,3-Thiadiazolyl-4 | |
| 1327 | H | H | H | F | 4-Methyl-oxazolyl-5 | |
| 1328 | H | H | H | F | Thiazolyl-2 | |
| 1329 | H | H | H | F | Thiazolyl-4 | |
| 1330 | H | H | H | F | Thiazolyl-5 | |
| 1331 | H | H | H | F | 4-Methyl-thiazolyl-5 | |
| 1332 | H | H | H | F | Imidazolyl-4 | |
| 1333 | H | H | H | F | Imidazolyl-5 | |
| 1334 | H | H | H | F | 4-Methyl-imidazolyl-5 | |
| 1335 | H | H | H | F | 1-Methyl-imidazolyl-5 | |
| 1336 | H | H | H | F | 4-Nitro-imidazolyl-1 | |
| 1337 | H | H | H | F | 2-Methyl-4-nitro-imidazolyl-1 | |
| 1338 | H | H | H | F | 4,5-Dichlorimidazolyl-1 | |
| 1339 | H | H | H | F | 1-Methyl-pyrryl-2 | |
| 1340 | H | H | H | F | Isoxazolyl-3 | |
| 1341 | H | H | H | F | Isoxazolyl-4 | |
| 1342 | H | H | H | F | Isoxazolyl-5 | |
| 1343 | H | H | H | F | 5-Chlormethyl-isoxazolyl-3 | |
| 1344 | H | H | H | F | Isothiazolyl-3 | |
| 1345 | H | H | H | F | Isothiazolyl-4 | |

| Nr. | $R^1$ | $R^2$ | $R^3$ | $R^4$ | $R^5$ | Fp. [°C] |
|---|---|---|---|---|---|---|
| 1346 | H | H | H | F | Isothiazolyl-5 | |
| 1347 | H | H | H | F | 4-Methyl-isothiazolyl-5 | |
| 1348 | H | H | H | F | Pyrazolyl-4 | |
| 1349 | H | H | H | F | Pyrazolyl-5 | |
| 1350 | H | H | H | F | 1-Methyl-pyrazolyl-5 | |
| 1351 | H | H | H | F | 1,2,3-Thiadiazolyl-5 | |
| 1352 | H | H | H | F | 1,2,3-Oxadiazolyl-5 | |
| 1353 | H | H | H | F | 1,2,3-Oxadiazolyl-4 | |
| 1354 | H | H | H | F | 5-Methyl-1,2,3-oxadiazolyl-4 | |
| 1355 | H | H | H | F | 4-Methyl-1,2,3-oxadiazolyl-5 | |
| 1356 | H | H | H | F | 1,3,4-Thiadiazolyl-2 | |
| 1357 | H | H | H | F | 2-Methyl-1,3,4-thiadiazolyl-5 | |
| 1358 | H | H | H | F | 1,2,4-Thiadiazolyl-3 | |
| 1359 | H | H | H | F | 1,2,4-Thiadiazolyl-5 | |
| 1360 | H | H | H | F | 3-Methyl-1,2,4-thiadiazolyl-5 | |
| 1361 | H | H | H | F | 1,2,4-Oxadiazolyl-3 | |
| 1362 | H | H | H | F | 1,2,4-Oxadiazolyl-5 | |
| 1363 | H | H | H | F | 1,2,4-Triazolyl-5 | |
| 1364 | H | H | H | F | 1,2,4-Triazolyl-3 | |
| 1365 | H | H | H | F | 1,3,4-Oxadiazolyl-5 | |
| 1366 | H | H | H | F | 2-Methyl-1,3,4-oxadiazolyl-5 | |
| 1367 | H | H | H | F | 1,2,5-Oxadiazolyl-3 | |
| 1368 | H | H | H | F | 3-Methyl-1,2,5-oxadiazolyl-4 | |
| 1369 | H | H | H | F | 1,2,5-Thiadiazolyl-3 | |
| 1370 | H | H | H | F | 3-Methyl-1,2,5-thiadiazolyl-4 | |
| 1371 | H | H | H | F | 2-Methyl-furyl-5 | |
| 1372 | H | H | H | F | 2,5-Dimethyl-furyl-4 | |
| 1373 | H | H | H | F | 2-Chlor-furyl-5 | |
| 1374 | H | H | H | F | 5-Methyl-oxazolyl-4 | |
| 1375 | H | H | H | F | 4-Methyl-oxazolyl-2 | |
| 1376 | H | H | H | F | 5-Methyl-isoxazolyl-3 | |
| 1377 | H | H | H | F | 4-Methyl-isoxazolyl-3 | |
| 1378 | H | H | H | F | 5-Methyl-isothiazolyl-3 | |
| 1379 | H | H | H | F | 4-Methyl-isothiazolyl-3 | |
| 1380 | H | H | H | F | 4-Methyl-1,2,3-thiadiazolyl-5 | |
| 1381 | H | H | H | F | 5-Methyl-1,2,3-thiadiazolyl-4 | |
| 1382 | H | H | H | F | Pyrryl-1 | |
| 1383 | H | H | H | F | Imidazolyl-1 | |
| 1384 | H | H | H | F | Pyrazolyl-1 | |
| 1385 | H | H | H | F | 1,3,4-Triazolyl-1 | |
| 1386 | H | H | H | F | 1,2,4-Triazolyl-1 | |

| Nr. | $R^1$ | $R^2$ | $R^3$ | $R^4$ | $R^5$ | Fp. [°C] |
|---|---|---|---|---|---|---|
| 1387 | H | H | $NO_2$ | F | Thienyl-2 | |
| 1388 | H | H | $NO_2$ | F | Thienyl-3 | |
| 1389 | H | H | $NO_2$ | F | 2,5-Dimethyl-thienyl-4 | |
| 1390 | H | H | $NO_2$ | F | 5-Methyl-thienyl-2 | |
| 1391 | H | H | $NO_2$ | F | Pyrryl-2 | |
| 1392 | H | H | $NO_2$ | F | Pyrryl-3 | |
| 1393 | H | H | $NO_2$ | F | Oxazolyl-2 | |
| 1394 | H | H | $NO_2$ | F | Oxazolyl-4 | |
| 1395 | H | H | $NO_2$ | F | Oxazolyl-5 | |
| 1396 | H | H | $NO_2$ | F | 1,2,3-Thiadiazolyl-4 | |
| 1397 | H | H | $NO_2$ | F | 4-Methyl-oxazolyl-5 | |
| 1398 | H | H | $NO_2$ | F | Thiazolyl-2 | |
| 1399 | H | H | $NO_2$ | F | Thiazolyl-4 | |
| 1400 | H | H | $NO_2$ | F | Thiazolyl-5 | |
| 1401 | H | H | $NO_2$ | F | 4-Methyl-thiazolyl-5 | |
| 1402 | H | H | $NO_2$ | F | Imidazolyl-4 | |
| 1403 | H | H | $NO_2$ | F | Imidazolyl-5 | |
| 1404 | H | H | $NO_2$ | F | 4-Methyl-imidazolyl-5 | |
| 1405 | H | H | $NO_2$ | F | 1-Methyl-imidazolyl-5 | |
| 1406 | H | H | $NO_2$ | F | 4-Nitro-imidazolyl-5 | |
| 1407 | H | H | $NO_2$ | F | 2-Methyl-4-nitro-imidazolyl-1 | |
| 1408 | H | H | $NO_2$ | F | 4,5-Dichlorimidazolyl-1 | |
| 1409 | H | H | $NO_2$ | F | 1-Methyl-pyrryl-2 | |
| 1410 | H | H | $NO_2$ | F | Isoxazolyl-3 | |
| 1411 | H | H | $NO_2$ | F | Isoxazolyl-4 | |
| 1412 | H | H | $NO_2$ | F | Isoxazolyl-5 | |
| 1413 | H | H | $NO_2$ | F | 5-Chlormethyl-isoxazolyl-3 | |
| 1414 | H | H | $NO_2$ | F | Isothiazolyl-3 | |
| 1415 | H | H | $NO_2$ | F | Isothiazolyl-4 | |
| 1416 | H | H | $NO_2$ | F | Isothiazolyl-5 | |
| 1417 | H | H | $NO_2$ | F | 4-Methyl-isothiazolyl-5 | |
| 1418 | H | H | $NO_2$ | F | Pyrazolyl-4 | |
| 1419 | H | H | $NO_2$ | F | Pyrazolyl-5 | |
| 1420 | H | H | $NO_2$ | F | 1-Methyl-pyrazolyl-5 | |
| 1421 | H | H | $NO_2$ | F | 1,2,3-Thiadiazolyl-5 | |
| 1422 | H | H | $NO_2$ | F | 1,2,3-Oxadiazolyl-5 | |
| 1423 | H | H | $NO_2$ | F | 1,2,3-Oxadiazolyl-4 | |
| 1424 | H | H | $NO_2$ | F | 5-Methyl-1,2,3-oxadiazolyl-4 | |
| 1425 | H | H | $NO_2$ | F | 4-Methyl-1,2,3-oxadiazolyl-5 | |
| 1426 | H | H | $NO_2$ | F | 1,3,4-Thiadiazolyl-2 | |
| 1427 | H | H | $NO_2$ | F | 2-Methyl-1,3,4-thiadiazolyl-5 | |
| 1428 | H | H | $NO_2$ | F | 1,2,4-Thiadiazolyl-3 | |

| Nr. | $R^1$ | $R^2$ | $R^3$ | $R^4$ | $R^5$ | Fp. [°C] |
|---|---|---|---|---|---|---|
| 1429 | H | H | $NO_2$ | F | 1,2,4-Thiadiazolyl-5 | |
| 1430 | H | H | $NO_2$ | F | 3-Methyl-1,2,4-thiadiazolyl-5 | |
| 1431 | H | H | $NO_2$ | F | 1,2,4-Oxadiazolyl-3 | |
| 1432 | H | H | $NO_2$ | F | 1,2,4-Oxadiazolyl-5 | |
| 1433 | H | H | $NO_2$ | F | 1,2,4-Triazolyl-5 | |
| 1434 | H | H | $NO_2$ | F | 1,2,4-Triazolyl-3 | |
| 1435 | H | H | $NO_2$ | F | 1,3,4-Oxadiazolyl-5 | |
| 1436 | H | H | $NO_2$ | F | 2-Methyl-1,3,4-oxadiazolyl-5 | |
| 1437 | H | H | $NO_2$ | F | 1,2,5-Oxadiazolyl-3 | |
| 1438 | H | H | $NO_2$ | F | 3-Methyl-1,2,5-oxadiazolyl-4 | |
| 1439 | H | H | $NO_2$ | F | 1,2,5-Thiadiazolyl-3 | |
| 1440 | H | H | $NO_2$ | F | 3-Methyl-1,2,5-thiadiazolyl-4 | |
| 1441 | H | H | $NO_2$ | F | 2-Methyl-furyl-5 | |
| 1442 | H | H | $NO_2$ | F | 2,5-Dimethyl-furyl-4 | |
| 1443 | H | H | $NO_2$ | F | 2-Chlor-furyl-5 | |
| 1444 | H | H | $NO_2$ | F | 5-Methyl-oxazolyl-4 | |
| 1445 | H | H | $NO_2$ | F | 4-Methyl-oxazolyl-2 | |
| 1446 | H | H | $NO_2$ | F | 5-Methyl-isoxazolyl-3 | |
| 1447 | H | H | $NO_2$ | F | 4-Methyl-isoxazolyl-3 | |
| 1448 | H | H | $NO_2$ | F | 5-Methyl-isothiazolyl-3 | |
| 1449 | H | H | $NO_2$ | F | 4-Methyl-isothiazolyl-3 | |
| 1450 | H | H | $NO_2$ | F | 4-Methyl-1,2,3-thiadiazolyl-5 | |
| 1451 | H | H | $NO_2$ | F | 5-Methyl-1,2,3-thiadiazolyl-4 | |
| 1452 | H | H | $NO_2$ | F | Pyrryl-1 | |
| 1453 | H | H | $NO_2$ | F | Imidazolyl-1 | |
| 1454 | H | H | $NO_2$ | F | Pyrazolyl-1 | |
| 1455 | H | H | $NO_2$ | F | 1,3,4-Triazolyl-1 | |
| 1456 | H | H | $NO_2$ | F | 1,2,4-Triazolyl-1 | |

| Nr. | $R^1$ | $R^2$ | $R^3$ | $R^4$ | $R^5$ | Fp. [°C] |
|---|---|---|---|---|---|---|
| 1525 | H | H | J | H | Thienyl-2 | |
| 1526 | H | H | J | H | Thienyl-3 | |
| 1527 | H | H | J | H | 2,5-Dimethyl-thienyl-4 | |
| 1528 | H | H | J | H | 5-Methyl-thienyl-2 | |
| 1529 | H | H | J | H | Pyrryl-2 | |
| 1530 | H | H | J | H | Pyrryl-3 | |
| 1531 | H | H | J | H | Oxazolyl-2 | |
| 1532 | H | H | J | H | Oxazolyl-4 | |
| 1533 | H | H | J | H | Oxazolyl-5 | |
| 1534 | H | H | J | H | 1,2,3-Thiadiazolyl-4 | |
| 1535 | H | H | J | H | 4-Methyl-oxazolyl-5 | |
| 1536 | H | H | J | H | Thiazolyl-2 | |
| 1537 | H | H | J | H | Thiazolyl-4 | |
| 1538 | H | H | J | H | Thiazolyl-5 | |
| 1539 | H | H | J | H | 4-Methyl-thiazolyl-5 | |
| 1540 | H | H | J | H | Imidazolyl-4 | |
| 1541 | H | H | J | H | Imidazolyl-5 | |
| 1542 | H | H | J | H | 4-Methyl-imidazolyl-5 | |
| 1543 | H | H | J | H | 1-Methyl-imidazolyl-5 | |
| 1544 | H | H | J | H | 4-Nitro-imidazolyl-1 | |
| 1545 | H | H | J | H | 2-Methyl-4-nitro-imidazolyl-1 | |
| 1546 | H | H | J | H | 4,5-Dichlorimidazolyl-1 | |
| 1547 | H | H | J | H | 1-Methyl-pyrryl-2 | |
| 1548 | H | H | J | H | Isoxazolyl-3 | |
| 1549 | H | H | J | H | Isoxazolyl-4 | |
| 1550 | H | H | J | H | Isoxazolyl-5 | |
| 1551 | H | H | J | H | 5-Chlormethyl-isoxazolyl-3 | |
| 1552 | H | H | J | H | Isothiazolyl-3 | |
| 1553 | H | H | J | H | Isothiazolyl-4 | |
| 1554 | H | H | J | H | Isothiazolyl-5 | |

| Nr. | $R^1$ | $R^2$ | $R^3$ | $R^4$ | $R^5$ | Fp. [°C] |
|---|---|---|---|---|---|---|
| 1555 | H | H | J | H | 4-Methyl-isothiazolyl-5 | |
| 1556 | H | H | J | H | Pyrazolyl-4 | |
| 1557 | H | H | J | H | Pyrazolyl-5 | |
| 1558 | H | H | J | H | 1-Methyl-pyrazolyl-5 | |
| 1559 | H | H | J | H | 1,2,3-Thiadiazolyl-5 | |
| 1560 | H | H | J | H | 1,2,3-Oxadiazolyl-5 | |
| 1561 | H | H | J | H | 1,2,3-Oxadiazolyl-4 | |
| 1562 | H | H | J | H | 5-Methyl-1,2,3-oxadiazolyl-4 | |
| 1563 | H | H | J | H | 4-Methyl-1,2,3-oxadiazolyl-5 | |
| 1564 | H | H | J | H | 1,3,4-Thiadiazolyl-2 | |
| 1565 | H | H | J | H | 2-Methyl-1,3,4-thiadiazolyl-5 | |
| 1566 | H | H | J | H | 1,2,4-Thiadiazolyl-3 | |
| 1567 | H | H | J | H | 1,2,4-Thiadiazolyl-5 | |
| 1568 | H | H | J | H | 3-Methyl-1,2,4-thiadiazolyl-5 | |
| 1569 | H | H | J | H | 1,2,4-Oxadiazolyl-3 | |
| 1570 | H | H | J | H | 1,2,4-Oxadiazolyl-5 | |
| 1571 | H | H | J | H | 1,2,4-Triazolyl-5 | |
| 1572 | H | H | J | H | 1,2,4-Triazolyl-3 | |
| 1573 | H | H | J | H | 1,3,4-Oxadiazolyl-5 | |
| 1574 | H | H | J | H | 2-Methyl-1,3,4-oxadiazolyl-5 | |
| 1575 | H | H | J | H | 1,2,5-Oxadiazolyl-3 | |
| 1576 | H | H | J | H | 3-Methyl-1,2,5-oxadiazolyl-4 | |
| 1577 | H | H | J | H | 1,2,5-Thiadiazolyl-3 | |
| 1578 | H | H | J | H | 3-Methyl-1,2,5-thiadiazolyl-4 | |
| 1579 | H | H | J | H | 2-Methyl-furyl-5 | |
| 1580 | H | H | J | H | 2,5-Dimethyl-furyl-4 | |
| 1581 | H | H | J | H | 2-Chlor-furyl-5 | |
| 1582 | H | H | J | H | 5-Methyl-oxazolyl-4 | |
| 1583 | H | H | J | H | 4-Methyl-oxazolyl-2 | |
| 1584 | H | H | J | H | 5-Methyl-isoxazolyl-3 | |
| 1585 | H | H | J | H | 4-Methyl-isoxazolyl-3 | |
| 1586 | H | H | J | H | 5-Methyl-isothiazolyl-3 | |
| 1587 | H | H | J | H | 4-Methyl-isothiazolyl-3 | |
| 1588 | H | H | J | H | 4-Methyl-1,2,3-thiadiazolyl-5 | |
| 1589 | H | H | J | H | 5-Methyl-1,2,3-thiadiazolyl-4 | |
| 1590 | H | H | J | H | Pyrryl-1 | |
| 1591 | H | H | J | H | Imidazolyl-1 | |
| 1592 | H | H | J | H | Pyrazolyl-1 | |
| 1593 | H | H | J | H | 1,3,4-Triazolyl-1 | |
| 1594 | H | H | J | H | 1,2,4-Triazolyl-1 | |

| Nr. | $R^1$ | $R^2$ | $R^3$ | $R^4$ | $R^5$ | Fp. [°C] |
|---|---|---|---|---|---|---|
| 1595 | H | H | J | $NO_2$ | Thienyl-2 | |
| 1596 | H | H | J | $NO_2$ | Thienyl-3 | |
| 1597 | H | H | J | $NO_2$ | Pyrryl-2 | |
| 1598 | H | H | J | $NO_2$ | Pyrryl-3 | |
| 1599 | H | H | J | $NO_2$ | Oxazolyl-2 | |
| 1600 | H | H | J | $NO_2$ | Oxazolyl-4 | |
| 1601 | H | H | J | $NO_2$ | 1,2,3-Thiadiazolyl-4 | |
| 1602 | H | H | J | $NO_2$ | Thiazolyl-2 | |
| 1603 | H | H | J | $NO_2$ | Thiazolyl-4 | |
| 1604 | H | H | J | $NO_2$ | Thiazolyl-5 | |
| 1605 | H | H | J | $NO_2$ | Imidazolyl-4 | |
| 1606 | H | H | J | $NO_2$ | Imidazolyl-5 | |
| 1607 | H | H | J | $NO_2$ | Isoxazolyl-3 | |
| 1608 | H | H | J | $NO_2$ | Isoxazolyl-4 | |
| 1609 | H | H | J | $NO_2$ | Isoxazolyl-5 | |
| 1610 | H | H | J | $NO_2$ | 5-Chlormethyl-isoxazolyl-3 | |
| 1611 | H | H | J | $NO_2$ | Isothiazolyl-3 | |
| 1612 | H | H | J | $NO_2$ | Isothiazolyl-4 | |
| 1613 | H | H | J | $NO_2$ | Pyrazolyl-4 | |
| 1614 | H | H | J | $NO_2$ | Pyrazolyl-5 | |
| 1615 | H | H | J | $NO_2$ | 1,2,3-Thiadiazolyl-5 | |
| 1616 | H | H | J | $NO_2$ | 1,2,3-Oxadiazolyl-5 | |
| 1617 | H | H | J | $NO_2$ | 1,2,3-Oxadiazolyl-4 | |
| 1618 | H | H | J | $NO_2$ | 1,3,4-Thiadiazolyl-2 | |
| 1619 | H | H | J | $NO_2$ | 2-Methyl-1,3,4-thiadiazolyl-5 | |
| 1620 | H | H | J | $NO_2$ | 2-Methyl-1,3,4-oxadiazolyl-5 | |
| 1621 | H | H | J | $NO_2$ | 2-Methyl-furyl-5 | |
| 1622 | H | H | J | $NO_2$ | 2,5-Dimethyl-furyl-4 | |
| 1623 | H | H | J | $NO_2$ | 4-Methyl-1,2,3-thiadiazolyl-5 | |
| 1624 | H | H | J | $NO_2$ | 5-Methyl-1,2,3-thiadiazolyl-4 | |
| 1625 | H | H | J | $NO_2$ | Pyrryl-1 | |
| 1626 | H | H | J | $NO_2$ | Imidazolyl-1 | |
| 1627 | H | H | J | $NO_2$ | Pyrazolyl-1 | |
| 1628 | H | H | J | $NO_2$ | 1,3,4-Triazolyl-1 | |
| 1629 | H | H | J | $NO_2$ | 1,2,4-Triazolyl-1 | |
| 1630 | H | H | J | $NO_2$ | Isothiazolyl-5 | |
| 1631 | H | H | F | $NO_2$ | Thienyl-2 | |
| 1632 | H | H | F | $NO_2$ | Thienyl-3 | |
| 1633 | H | H | F | $NO_2$ | Pyrryl-2 | |
| 1634 | H | H | F | $NO_2$ | Pyrryl-3 | |
| 1635 | H | H | F | $NO_2$ | Oxazolyl-2 | |
| 1636 | H | H | F | $NO_2$ | Oxazolyl-4 | |

| Nr. | $R^1$ | $R^2$ | $R^3$ | $R^4$ | $R^5$ | Fp. [°C] |
|---|---|---|---|---|---|---|
| 1637 | H | H | F | $NO_2$ | 1,2,3-Thiadiazolyl-4 | |
| 1638 | H | H | F | $NO_2$ | Thiazolyl-2 | |
| 1639 | H | H | F | $NO_2$ | Thiazolyl-4 | |
| 1640 | H | H | F | $NO_2$ | Thiazolyl-5 | |
| 1641 | H | H | F | $NO_2$ | Imidazolyl-4 | |
| 1642 | H | H | F | $NO_2$ | Imidazolyl-5 | |
| 1643 | H | H | F | $NO_2$ | Isoxazolyl-3 | |
| 1644 | H | H | F | $NO_2$ | Isoxazolyl-4 | |
| 1645 | H | H | F | $NO_2$ | Isoxazolyl-5 | |
| 1646 | H | H | F | $NO_2$ | 5-Chlormethyl-isoxazolyl-3 | |
| 1647 | H | H | F | $NO_2$ | Isothiazolyl-3 | |
| 1648 | H | H | F | $NO_2$ | Isothiazolyl-4 | |
| 1649 | H | H | F | $NO_2$ | Pyrazolyl-4 | |
| 1650 | H | H | F | $NO_2$ | Pyrazolyl-5 | |
| 1651 | H | H | F | $NO_2$ | 1,2,3-Thiadiazolyl-5 | |
| 1652 | H | H | F | $NO_2$ | 1,2,3-Oxadiazolyl-5 | |
| 1653 | H | H | F | $NO_2$ | 1,2,3-Oxadiazolyl-4 | |
| 1654 | H | H | F | $NO_2$ | 1,3,4-Thiadiazolyl-2 | |
| 1655 | H | H | F | $NO_2$ | 2-Methyl-1,3,4-thiadiazolyl-5 | |
| 1656 | H | H | F | $NO_2$ | 2-Methyl-1,3,4-oxadiazolyl-5 | |
| 1657 | H | H | F | $NO_2$ | 2-Methyl-furyl-5 | |
| 1658 | H | H | F | $NO_2$ | 2,5-Dimethyl-furyl-4 | |
| 1659 | H | H | F | $NO_2$ | 4-Methyl-1,2,3-thiadiazolyl-5 | |
| 1660 | H | H | F | $NO_2$ | 5-Methyl-1,2,3-thiadiazolyl-4 | |
| 1661 | H | H | F | $NO_2$ | Pyrryl-1 | |
| 1662 | H | H | F | $NO_2$ | Imidazolyl-1 | |
| 1663 | H | H | F | $NO_2$ | Pyrazolyl-1 | |
| 1664 | H | H | F | $NO_2$ | 1,3,4-Triazolyl-1 | |
| 1665 | H | H | F | $NO_2$ | 1,2,4-Triazolyl-1 | |
| 1666 | H | H | F | $NO_2$ | Isothiazolyl-5 | |

| Nr. | $R^1$ | $R^2$ | $R^3$ | $R^4$ | $R^5$ | Fp. [°C] |
|---|---|---|---|---|---|---|
| 1701 | $CH_3$ | H | H | Cl | Thienyl-2 | |
| 1702 | $CH_3$ | H | H | Cl | Thienyl-3 | |
| 1703 | $CH_3$ | H | H | Cl | Pyrryl-2 | |
| 1704 | $CH_3$ | H | H | Cl | Pyrryl-3 | |
| 1705 | $CH_3$ | H | H | Cl | Oxazolyl-2 | |
| 1706 | $CH_3$ | H | H | Cl | Oxazolyl-4 | |
| 1707 | $CH_3$ | H | H | Cl | 1,2,3-Thiadiazolyl-4 | 127-130 |
| 1708 | $CH_3$ | H | H | Cl | Thiazolyl-2 | |
| 1709 | $CH_3$ | H | H | Cl | Thiazolyl-4 | |
| 1710 | $CH_3$ | H | H | Cl | Thiazolyl-5 | |
| 1711 | $CH_3$ | H | H | Cl | Imidazolyl-4 | |
| 1712 | $CH_3$ | H | H | Cl | Imidazolyl-5 | |
| 1713 | $CH_3$ | H | H | Cl | Isoxazolyl-3 | 113-117 |
| 1714 | $CH_3$ | H | H | Cl | Isoxazolyl-4 | |
| 1715 | $CH_3$ | H | H | Cl | Isoxazolyl-5 | 106-110 |
| 1716 | $CH_3$ | H | H | Cl | 5-Chlormethyl-isoxazolyl-3 | |
| 1717 | $CH_3$ | H | H | Cl | Isothiazolyl-3 | |
| 1718 | $CH_3$ | H | H | Cl | Isothiazolyl-4 | |
| 1719 | $CH_3$ | H | H | Cl | Pyrazolyl-4 | |
| 1720 | $CH_3$ | H | H | Cl | Pyrazolyl-5 | |

| Nr. | $R^1$ | $R^2$ | $R^3$ | $R^4$ | $R^5$ | Fp. [°C] |
|---|---|---|---|---|---|---|
| 1721 | $CH_3$ | H | H | Cl | 1,2,3-Thiadiazolyl-5 | |
| 1722 | $CH_3$ | H | H | Cl | 1,2,3-Oxadiazolyl-5 | |
| 1723 | $CH_3$ | H | H | Cl | 1,2,3-Oxadiazolyl-4 | |
| 1724 | $CH_3$ | H | H | Cl | 1,3,4-Thiadiazolyl-2 | |
| 1725 | $CH_3$ | H | H | Cl | 2-Methyl-1,3,4-thiadiazolyl-5 | |
| 1726 | $CH_3$ | H | H | Cl | 2-Methyl-1,3,4-oxadiazolyl-5 | |
| 1727 | $CH_3$ | H | H | Cl | 2-Methyl-furyl-5 | |
| 1728 | $CH_3$ | H | H | Cl | 2,5-Dimethyl-furyl-4 | |
| 1729 | $CH_3$ | H | H | Cl | 4-Methyl-1,2,3-thiadiazolyl-5 | 140-142 |
| 1730 | $CH_3$ | H | H | Cl | 5-Methyl-1,2,3-thiadiazolyl-4 | |
| 1731 | $CH_3$ | H | H | Cl | Pyrryl-1 | |
| 1732 | $CH_3$ | H | H | Cl | Imidazolyl-1 | |
| 1733 | $CH_3$ | H | H | Cl | Pyrazolyl-1 | |
| 1734 | $CH_3$ | H | H | Cl | 1,3,4-Triazolyl-1 | |
| 1735 | $CH_3$ | H | H | Cl | 1,2,4-Triazolyl-1 | |
| 1736 | $CH_3$ | H | H | Cl | Isothiazolyl-5 | |
| 1737 | $CH_3$ | H | Cl | H | Thienyl-2 | |
| 1738 | $CH_3$ | H | Cl | H | Thienyl-3 | |
| 1739 | $CH_3$ | H | Cl | H | Pyrryl-2 | |
| 1740 | $CH_3$ | H | Cl | H | Pyrryl-3 | |
| 1741 | $CH_3$ | H | Cl | H | Oxazolyl-2 | |
| 1742 | $CH_3$ | H | Cl | H | Oxazolyl-4 | |
| 1743 | $CH_3$ | H | Cl | H | 1,2,3-Thiadiazolyl-4 | 155-157 |
| 1744 | $CH_3$ | H | Cl | H | Thiazolyl-2 | |
| 1745 | $CH_3$ | H | Cl | H | Thiazolyl-4 | |
| 1746 | $CH_3$ | H | Cl | H | Thiazolyl-5 | |
| 1747 | $CH_3$ | H | Cl | H | Imidazolyl-4 | |
| 1748 | $CH_3$ | H | Cl | H | Imidazolyl-5 | |
| 1749 | $CH_3$ | H | Cl | H | Isoxazolyl-3 | 128-130 |
| 1750 | $CH_3$ | H | Cl | H | Isoxazolyl-4 | |
| 1751 | $CH_3$ | H | Cl | H | Isoxazolyl-5 | |
| 1752 | $CH_3$ | H | Cl | H | 5-Chlormethyl-isoxazolyl-3 | |
| 1753 | $CH_3$ | H | Cl | H | Isothiazolyl-3 | |
| 1754 | $CH_3$ | H | Cl | H | Isothiazolyl-4 | |
| 1755 | $CH_3$ | H | Cl | H | Pyrazolyl-4 | |
| 1756 | $CH_3$ | H | Cl | H | Pyrazolyl-5 | |
| 1757 | $CH_3$ | H | Cl | H | 1,2,3-Thiadiazolyl-5 | |
| 1758 | $CH_3$ | H | Cl | H | 1,2,3-Oxadiazolyl-5 | |
| 1759 | $CH_3$ | H | Cl | H | 1,2,3-Oxadiazolyl-4 | |
| 1760 | $CH_3$ | H | Cl | H | 1,3,4-Thiadiazolyl-2 | |
| 1761 | $CH_3$ | H | Cl | H | 2-Methyl-1,3,4-thiadiazolyl-5 | |
| 1762 | $CH_3$ | H | Cl | H | 2-Methyl-1,3,4-oxadiazolyl-5 | |

| Nr. | $R^1$ | $R^2$ | $R^3$ | $R^4$ | $R^5$ | Fp. [°C] |
|---|---|---|---|---|---|---|
| 1763 | $CH_3$ | H | Cl | H | 2-Methyl-furyl-5 | |
| 1764 | $CH_3$ | H | Cl | H | 2,5-Dimethyl-furyl-4 | 100-102 |
| 1765 | $CH_3$ | H | Cl | H | 4-Methyl-1,2,3-thiadiazolyl-5 | 161-163 |
| 1766 | $CH_3$ | H | Cl | H | 5-Methyl-1,2,3-thiadiazolyl-4 | |
| 1767 | $CH_3$ | H | Cl | H | Pyrryl-1 | |
| 1768 | $CH_3$ | H | Cl | H | Imidazolyl-1 | |
| 1769 | $CH_3$ | H | Cl | H | Pyrazolyl-1 | |
| 1770 | $CH_3$ | H | Cl | H | 1,3,4-Triazolyl-1 | |
| 1771 | $CH_3$ | H | Cl | H | 1,2,4-Triazolyl-1 | |
| 1772 | $CH_3$ | H | Cl | H | Isothiazolyl-5 | |
| 1773 | $CH_3$ | H | $NO_2$ | H | Thienyl-2 | |
| 1774 | $CH_3$ | H | $NO_2$ | H | Thienyl-3 | |
| 1775 | $CH_3$ | H | $NO_2$ | H | Pyrryl-2 | |
| 1776 | $CH_3$ | H | $NO_2$ | H | Pyrryl-3 | |
| 1777 | $CH_3$ | H | $NO_2$ | H | Oxazolyl-2 | |
| 1778 | $CH_3$ | H | $NO_2$ | H | Oxazolyl-4 | |
| 1779 | $CH_3$ | H | $NO_2$ | H | 1,2,3-Thiadiazolyl-4 | |
| 1780 | $CH_3$ | H | $NO_2$ | H | Thiazolyl-2 | |
| 1781 | $CH_3$ | H | $NO_2$ | H | Thiazolyl-4 | |
| 1782 | $CH_3$ | H | $NO_2$ | H | Thiazolyl-5 | |
| 1783 | $CH_3$ | H | $NO_2$ | H | Imidazolyl-4 | |
| 1784 | $CH_3$ | H | $NO_2$ | H | Imidazolyl-5 | |
| 1785 | $CH_3$ | H | $NO_2$ | H | Isoxazolyl-3 | |
| 1786 | $CH_3$ | H | $NO_2$ | H | Isoxazolyl-4 | |
| 1787 | $CH_3$ | H | $NO_2$ | H | Isoxazolyl-5 | |
| 1788 | $CH_3$ | H | $NO_2$ | H | 5-Chlormethyl-isoxazolyl-3 | |
| 1789 | $CH_3$ | H | $NO_2$ | H | Isothiazolyl-3 | |
| 1790 | $CH_3$ | H | $NO_2$ | H | Isothiazolyl-4 | |
| 1791 | $CH_3$ | H | $NO_2$ | H | Pyrazolyl-4 | |
| 1792 | $CH_3$ | H | $NO_2$ | H | Pyrazolyl-5 | |
| 1793 | $CH_3$ | H | $NO_2$ | H | 1,2,3-Thiadiazolyl-5 | |
| 1794 | $CH_3$ | H | $NO_2$ | H | 1,2,3-Oxadiazolyl-5 | |
| 1795 | $CH_3$ | H | $NO_2$ | H | 1,2,3-Oxadiazolyl-4 | |
| 1796 | $CH_3$ | H | $NO_2$ | H | 1,3,4-Thiadiazolyl-2 | |
| 1797 | $CH_3$ | H | $NO_2$ | H | 2-Methyl-1,3,4-thiadiazolyl-5 | |
| 1798 | $CH_3$ | H | $NO_2$ | H | 2-Methyl-1,3,4-oxadiazolyl-5 | |
| 1799 | $CH_3$ | H | $NO_2$ | H | 2-Methyl-furyl-5 | |
| 1800 | $CH_3$ | H | $NO_2$ | H | 2,5-Dimethyl-furyl-4 | |
| 1801 | $CH_3$ | H | $NO_2$ | H | 4-Methyl-1,2,3-thiadiazolyl-5 | |
| 1802 | $CH_3$ | H | $NO_2$ | H | 5-Methyl-1,2,3-thiadiazolyl-4 | |
| 1803 | $CH_3$ | H | $NO_2$ | H | Pyrryl-1 | |
| 1804 | $CH_3$ | H | $NO_2$ | H | Imidazolyl-1 | |

| Nr. | $R^1$ | $R^2$ | $R^3$ | $R^4$ | $R^5$ | Fp. [°C] |
|---|---|---|---|---|---|---|
| 1805 | $CH_3$ | H | $NO_2$ | H | Pyrazolyl-1 | |
| 1806 | $CH_3$ | H | $NO_2$ | H | 1,3,4-Triazolyl-1 | |
| 1807 | $CH_3$ | H | $NO_2$ | H | 1,2,4-Triazolyl-1 | |
| 1808 | $CH_3$ | H | $NO_2$ | H | Isothiazolyl-5 | |
| 1809 | H | Cl | $NO_2$ | $NO_2$ | Thienyl-2 | |
| 1810 | H | Cl | $NO_2$ | $NO_2$ | Thienyl-3 | |
| 1811 | H | Cl | $NO_2$ | $NO_2$ | Pyrryl-2 | |
| 1812 | H | Cl | $NO_2$ | $NO_2$ | Pyrryl-3 | |
| 1813 | H | Cl | $NO_2$ | $NO_2$ | Oxazolyl-2 | |
| 1814 | H | Cl | $NO_2$ | $NO_2$ | Oxazolyl-4 | |
| 1815 | H | Cl | $NO_2$ | $NO_2$ | 1,2,3-Thiadiazolyl-4 | |
| 1816 | H | Cl | $NO_2$ | $NO_2$ | Thiazolyl-2 | |
| 1817 | H | Cl | $NO_2$ | $NO_2$ | Thiazolyl-4 | |
| 1818 | H | Cl | $NO_2$ | $NO_2$ | Thiazolyl-5 | |
| 1819 | H | Cl | $NO_2$ | $NO_2$ | Imidazolyl-4 | |
| 1820 | H | Cl | $NO_2$ | $NO_2$ | Imidazolyl-5 | |
| 1821 | H | Cl | $NO_2$ | $NO_2$ | Isoxazolyl-3 | |
| 1822 | H | Cl | $NO_2$ | $NO_2$ | Isoxazolyl-4 | |
| 1823 | H | Cl | $NO_2$ | $NO_2$ | Isoxazolyl-5 | |
| 1824 | H | Cl | $NO_2$ | $NO_2$ | 5-Chlormethyl-isoxazolyl-3 | |
| 1825 | H | Cl | $NO_2$ | $NO_2$ | Isothiazolyl-3 | |
| 1826 | H | Cl | $NO_2$ | $NO_2$ | Isothiazolyl-4 | |
| 1827 | H | Cl | $NO_2$ | $NO_2$ | Pyrazolyl-4 | |
| 1828 | H | Cl | $NO_2$ | $NO_2$ | Pyrazolyl-5 | |
| 1829 | H | Cl | $NO_2$ | $NO_2$ | 1,2,3-Thiadiazolyl-5 | |
| 1830 | H | Cl | $NO_2$ | $NO_2$ | 1,2,3-Oxadiazolyl-5 | |
| 1831 | H | Cl | $NO_2$ | $NO_2$ | 1,2,3-Oxadiazolyl-4 | |
| 1832 | H | Cl | $NO_2$ | $NO_2$ | 1,3,4-Thiadiazolyl-2 | |
| 1833 | H | Cl | $NO_2$ | $NO_2$ | 2-Methyl-1,3,4-thiadiazolyl-5 | |
| 1834 | H | Cl | $NO_2$ | $NO_2$ | 2-Methyl-1,3,4-oxadiazolyl-5 | |
| 1835 | H | Cl | $NO_2$ | $NO_2$ | 2-Methyl-furyl-5 | |
| 1836 | H | Cl | $NO_2$ | $NO_2$ | 2,5-Dimethyl-furyl-4 | |
| 1837 | H | Cl | $NO_2$ | $NO_2$ | 4-Methyl-1,2,3-thiadiazolyl-5 | |
| 1838 | H | Cl | $NO_2$ | $NO_2$ | 5-Methyl-1,2,3-thiadiazolyl-4 | |
| 1839 | H | Cl | $NO_2$ | $NO_2$ | Pyrryl-1 | |
| 1840 | H | Cl | $NO_2$ | $NO_2$ | Imidazolyl-1 | |
| 1841 | H | Cl | $NO_2$ | $NO_2$ | Pyrazolyl-1 | |
| 1842 | H | Cl | $NO_2$ | $NO_2$ | 1,3,4-Triazolyl-1 | |
| 1843 | H | Cl | $NO_2$ | $NO_2$ | 1,2,4-Triazolyl-1 | |
| 1844 | H | Cl | $NO_2$ | $NO_2$ | Isothiazolyl-5 | |

| Nr. | $R^1$ | $R^2$ | $R^3$ | $R^4$ | $R^5$ | Fp. [°C] |
|---|---|---|---|---|---|---|
| 1846 | H | H | $NO_2$ | H | 3,5-Dimethyl-isoxazolyl-4 | 205-207 |
| 1847 | H | H | H | Cl | 3,5-Dimethyl-isoxazolyl-4 | |
| 1848 | H | H | H | Br | 3,5-Dimethyl-isoxazolyl-4 | |
| 1849 | H | H | H | $NO_2$ | 3,5-Dimethyl-isoxazolyl-4 | |
| 1850 | H | H | $NO_2$ | Cl | 3,5-Dimethyl-isoxazolyl-4 | |
| 1851 | H | H | $NO_2$ | Br | 3,5-Dimethyl-isoxazolyl-4 | |
| 1852 | H | H | $NO_2$ | J | 3,5-Dimethyl-isoxazolyl-4 | |
| 1853 | H | H | Cl | H | 3,5-Dimethyl-isoxazolyl-4 | |
| 1854 | H | H | Cl | $NO_2$ | 3,5-Dimethyl-isoxazolyl-4 | |
| 1855 | H | H | Br | $NO_2$ | 3,5-Dimethyl-isoxazolyl-4 | |
| 1856 | H | H | Br | H | 3,5-Dimethyl-isoxazolyl-4 | |
| 1857 | H | H | $NO_2$ | $NO_2$ | 3,5-Dimethyl-isoxazolyl-4 | |
| 1858 | $CH_3$ | H | $NO_2$ | $NO_2$ | 3,5-Dimethyl-isoxazolyl-4 | |
| 1859 | $CH_3$ | H | $NO_2$ | Cl | 3,5-Dimethyl-isoxazolyl-4 | |
| 1862 | H | H | J | H | 3,5-Dimethyl-isoxazolyl-4 | |
| 1863 | H | H | J | $NO_2$ | 3,5-Dimethyl-isoxazolyl-4 | |
| 1865 | $CH_3$ | H | H | Cl | 3,5-Dimethyl-isoxazolyl-4 | |
| 1866 | $CH_3$ | H | Cl | H | 3,5-Dimethyl-isoxazolyl-4 | |
| 1867 | $CH_3$ | H | $NO_2$ | H | 3,5-Dimethyl-isoxazolyl-4 | |

Die Wirkstoffe zeigen eine starke Wirksamkeit gegen Mikroorganismen. Sie dienen zur Verhütung und Heilung von Pflanzenkrankheiten, die durch Pilze verursacht werden, wie z.B. Botrytis cinerea an Reben und Erdbeeren, Monilia fructigena an Äpfeln, Cercospora arachidicola an Erdnüssen, Phytophthora infestans an Kartoffeln und Tomaten, Plasmopara viticola an Reben, Alternaria solani an Tomaten und Helminthosporium teres sowie Septoria nodorum an Getreide.

Weiterhin sind die Verbindungen auch gegenüber phytopathogenen Bakterien wie Erwinia amylovora an Birnen und Äpfeln, Erwinia carotovora an Kartoffeln, Pseudomonas lachrymans an Gurken, Pseudomonas phaseolicola an Bohnen, Xanthomonas oryzae an Reis wirksam.

Die Wirkstoffe werden beispielsweise in Form von direkt versprühbaren Lösungen, von Pulvern, Suspensionen, auch hochprozentigen wäßrigen, öligen oder sonstigen Suspensionen oder Dispersionen, Emulsionen, Öldispersionen, Pasten, Stäubemitteln, Streumitteln, Granulaten, durch Versprühen, Zumischen unter Rühren, Spachteln, Streichen, Tränken, Imprägnieren, Vernebeln, Verstäuben, Verstreuen oder Gießen angewandt. Die Anwendungsformen richten sich nach den Verwendungszwecken ; sie sollten in jedem Fall möglichst die feinste Verteilung der neuen Wirkstoffe gewährleisten.

Zur Herstellung von direkt versprühbaren Lösungen, Emulsionen und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate z.B. Methanol, Ethanol, Propanol, Butanol, Chloroform, Tetrechlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, vor allem aber Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur verdünnung mit Wasser geeignet sind.

Als Netz-, Dispergier- und Emulgiermittel kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze von Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Talkum, Bolus, Löß, Ton, Dolomit, Dietomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nuß- schalenmehl, Cellulosepulver und andere feste Trägerstoffe.

Beispiele für solche Zubereitungen sind :

I. Man vermischt 90 Gewichtsteile der Verbindung 1 (der Verbindung von Beispiel 1) mit 10 Gewichtsteilen N-methyl-alpha-pyrrolidon und erhält eine Lösung, die zur Anwendung in Form kleinster Tropfen geeignet ist.

II. 20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 mol Ethylenoxid an 1 mol Ricinusöl besteht. Durch Ausgießen und feines Verteilen der Lösung in Wasser erhält men eine wäßrige Dispersion.

III. 20 Gewichtsteile der Verbindung Nr. 24 werden in einer Mischung gelöst, die aus 40 Gewichtsteilen Cyclohexanon, 30 Gewichtsteilen Isobutanol, 20 Gewichtsteilen des Anlagerungsproduktes von 40 mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 43 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 mol Ethylenoxid an 1 mol Ricinusöl besteht. Durch Eingießen und feines Verteilen der Lösung in Wasser erhält man eine wäßrige Dispersion.

V. 80 Gewichtsteile der Verbindung Nr. 111 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung Nr. 196 werden mit 95 Gewichtsteilen feinteiligem Kaolin innig vermischt. Man erhält auf diese weise ein Stäubemittel, das 5 Gew.% des Wirkstoffs enthält.

VII. 30 Gewichtsteile der Verbindung Nr. 214 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 503 werden mit 30 Teilen Natriumsalz eines Phenolsulfonsäureharnstoff-formaldehyd-Kondensates, 2 Teilen Kieselgel und 48 Teilen Wasser innig vermischt. Man erhält eine stabile wäßrige Dispersion.

IX. 20 Teile der Verbindung Nr. 904 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die Wirkstoffe können auch zusammen mit anderen Wirkstoffen, z.B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden eine Vergröberung des fungiziden Wirkungsspektrums; bei einer Anzahl dieser Fungizidmischungen treten auch synergistische Effekte auf, d.h. die fungizide Wirksamkeit des Kombinationsproduktes ist gröber als die Summe der Wirksamkeiten der Einzelkomponenten.

Die neuen Wirkstoffe eignen sich nicht nur zum Pflanzenschutz, sondern auch zum Schutz von verschiedenen Materialien gegen den Abbau bzw. die Zerstörung durch Bakterien und Pilze. Materialien, die mit den neuen Wirkstoffen konserviert bzw. mikrozid ausgerüstet werden können, sind beispielsweise Leime und Klebstoffe, Kunststoffdispersionen, Dispersionsfarben, Anstrichfilme in Feuchträumen, Textilien, Leder, Rohhäute, Holz und Kunststoffe, insbesondere Weich-PVC.

Die Zubereitungen enthalten im allgemeinen 0,1 bis 95 Gew.% Wirkstoff, vorzugsweise 0,5 bis 90%. Die Aufwandmengen betragen je nach Art des gewünschten Effektes 0,001 bis 5 Gew.% Wirkstoff, bezogen auf das Gewicht des zu schützenden Materials, vorzugsweise zwischen 0,01 und 5%, bzw. in der Landwirtschaft 0,1 bis 5 kg je ha.

Folgende Mikroorganismen lassen sich beispielsweise mit den erfindungsgemäßen Verbindungen zum Schutz von Material bekämpfen :

Chaetomium globosum, Chaetomium alba, Aspergillus terreus, Aspergillus niger, Aspergillus versicolor, Penicillium glaucum, Penicillium funiculosum, Trichoderma viride, Aureobasidium pullulans, Cladosporium herbarum, Cladosporium resinae, Humicola grisea, Glenospora graphii, Phoma violacea ; Streptomyces albus ; Staphylococcus aureus, Escherichia coli, Pseudomonas aeruginosa, Aerobacter aerogenes, Serratia marcescens.

Die folgende Liete von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielweise:

Schwefel,

Dithiocarbamate und deren Derivate, wie Ferridimethyl-, Zinkdimethyl-, Zinkethylenbis-, Manganethylenbis-, Mangan-Zink-ethylendiamin-bis- oder Zink-(N,N'-propylen-bis-dithiocarbamat), Ammoniakkomplexe von Zink-(N,N''-ethylen-bis-dithiocarbamat) und von Zink-(N,N''-propylen-bis-dithiocarbamat), Tetramethylthiuramdisulfid oder N,N''-Poly-(propylen-bis-thiocarbamoyl-disulfid), Nitroverbindungen, wie Dinitro-(1-methylheptyl)-phenylcrotonat, (2-sec.-Butyl-4,6-dinitrophenyl)-3,3-dimethylacrylat, 2-sec.-Butyl-4,6-dinitrophenyl-isopropylcarbonat oder 5-Nitro-isophthalsäure-di-isopropylester,

Heterocyclen, wie

N-Cyclohexyl-N-methoxy-2,5-dimethyl-furan-3-carbonsäureamid,
2,5-Dimethyl-furan-3-carbonsäurecyclohexylamid,
2-Methyl-furan-3-carbonsäureanilid,
2,5-Dimethyl-furan-3-carbonsäureanilid,
2,4,5-Trimethyl-furen-3-carbonsäureanilid,
DL-Methyl-N-(2,6-dimethyl-phenyl)-N-furoyl(2)-alaninat,
0,0-Diethyl-N-phthalimidothiophoshat,
N-Trichlormethylthio-phthalimid,
N-(1,1,2,2-Tetrachlorethylthio)-tetrahydrophthalimid,
N-Trichlormethylthio-tetrahydrophthalimid,
Heptadecyl-2-imidazolin-acetat,
2-Furyl-2''-benzimidazol,
2-Methoxycarbonylamino-benzimidazol,
1-(N-butylcarbamoyl)-benzimidazol-2-carbaminsäuremethylester,
2-(Thiazol-4-yl)-benzimidazol,
N-(n-Propyl)-N-(2,4,6-trichlorphenoxyethyl)-N''-imidazolyl-harnstoff,
5-Methyl-5-vinyl-3-(3,5-dichlorphenyl)-2,4-dioxo-1,3-oxazolidin,
3-(3,5-Dichlorphenyl)-5-methyl-5-methoxymethyl-1,3-oxazolidin-2,4-dion,
4-(2-Chlorphenylhydrazono)-3-methyl-5-isoxazolon,
2-Thio-1,3-dithiolo-(4,5-b)-chinoxalin,
2,3-Dihydro-5-carboxanilido-6-methyl-1,4-oxathiin,
2,3-Dihydro-5-carboxanilido-6-methyl-1,4-oxathiin-4,4-dioxid,
2-Thiocyanatomethylthio-benzthiazol,
1-[2-(2,4-Dichlorphenyl)-4-ethyl-1,3-dioxolan-2-yl-ethyl]-1H-1,2,4-triazol,
1-[2-(2,4-Dichlorphenyl)-4-n-propyl-1,3-dioxolan-2-yl-ethyl]-1H-1,2,4-triazol,
1-(2-(2,4-Dichlorphenyl)-1H-1,2,4-triazol,
5-Amino-1-[bis-(dimethylamino)-phosphinyl]-3-phenyl-1,2,4-triazol,
1-[bis-(4-Fluorphenyl)-methylsilyl]-methyl-1H-1,2,4-triazol,
1-(4-Chlorphenoxy)-3,3-dimethyl-1-(1H-1,2,4-triazol-1-yl)-2-butanon,
1-(4-Phenylphenoxy)-3,3-dimethyl-1-(1H-1,2,4-triazol-1-yl)-2-butanol,

2,4"-Difluor-alpha-(1H-1,2,4-triazolyl-1-methyl)-benzhydrylalkohol,
1-(4-Chlorphenoxy)-3,3-dimethyl-1-(1H-1,2,4-triazol-1-yl)-2-butanol,
5-Ethoxy-3-trichlormethyl-1,2,4-thiadiazol, 2-Methyl-5,6-dihydro-4-H-pyran-3-carbonsäure-anilid,
N-[3-(p-tert.-Butylphenyl)-2-methylpropyl]-piperidin,
3-(3,5-Dimethyl-2-oxycyclohexyl)-3-(2-hydroxyethyl)-glutarimid,
2,6-Dimethyl-N-cyclododecyl-morpholin bzw. 2,6-Dimethyl-N-tridecyl-morpholin und deren Salze,
N-Formyl-N-morpholin-2,2,2-trichlorethyl-N,N-acetal,
N-[3-(p-tert.-Butylphenyl)-2-methylpropyl]-cis-2,6-dimethylmorpholin,
1,4-Di-(2,2,2-trichlor-1-formylaminoethyl)-piperazin, Pyridin-2-mercapto-oxid,
Bis-(p-chlorphenyl)-pyridin-3-yl-methanol,
2-[N-(3-Chlor-2,6-dinitro-4-trifluormethylphenyl-amino]-5-trifluormethyl-3-chlorpyridin,
8-Hydroxychinolin bzw. dessen Kupfersalz,
5-Butyl-2-dimethylamino-4-hydroxy-6-methyl-pyrimidin,
alpha-(2-Chlorphenyl)-alpha-(4-chlorphenyl)-pyrimidin-5-yl-methanol,
2,4-Dichlor-6-(o-chloranilino)-s-triazin oder N-(2,6-Dimethylphenyl)-N-chloracetyl-D,L-2-aminobutyrolacton,

sowie andere Fungizide, wie

1-(3,4-Dichloranilino)-1-formylamino-2,2,2-trichlorethan, Dodecylguanidinacetat,
3-(3,5-Dichlorphenyl)-1-isopropylcarbamoylhydantoin, Hexachlorbenzol,
1,4-Dichlor-2,5-dimethoxybenzol,
1,2-Bis-(3-methoxycarbonyl-2-thioureido)-benzol,
1,2-Bis-(3-ethoxycarbonyl-2-thioureido)-benzol
2-Methyl-benzoesäure-anilid,
2-Iod-benzoesäure-anilid,
2,3-Dicyano-1,4-dithiaanthrachinon,
DL-N-(2,6-Dimethylphenyl)-N-(phenylacetyl)-alaninmethylester,
2-Cyano-N-(ethylaminocarbonyl)-2-methoximino-acetamid,
N-(3,5-Dichlorphenyl)-1,2-dimethylcyclopropan-1,2-dicarbonsäureimid, N-Dichlorfluormethylthio-N',N'-dimethyl-N-phenyl-schwefelsäurediamid oder DL-N-(2,6-Dimethyl-phenyl)-N-(2'-methoxyacetyl)-alaninmethylester.

In den Anwendungsbeispielen wurden folgende Substanzen als Vergleichsmittel eingesetzt:
A : 5-Chlor-8-(4'-methyl-1',2',3'-thiadiazol-5'-carbonyloxy)-chinolin (EP 98 486, Bsp. 128) ;
B : 8-alpha-Furoyloxy-5-methylchinolin (GB 1 141 697, Bsp. 1) ;
C : 5-Acetyl-7-nitro-8-(1',2',3'-thiadiazol-4-carbonyloxy)-chinolin (DE 3 225 169, Bsp. 24) ;
D : 7-Nitro-8-(alpha-thenoyloxy)-chinolin (DE 2 005 959, Bsp. 38).

Anwendungsbeispiele :

Beispiel I

Wirksamkeit gegen Botrytis cinerea an Paprika

Paprikasämlinge der Sorte "Neusiedler Ideal Elite" wurden, nach dem sich 4 bis 5 Blätter gut entwickelt hatten, mit wäßrigen Suspensionen, die 80% Wirkstoff und 20% Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die krankheit auf den unbehandelten Kontrollpflanzen so stark entwickelt, daß die entstandenen Blattnekrosen den überwiegenden Teil der Blätter bedeckten.

Die Verbindungen der Beispiele 1, 111, 298, 300, 301, 332, 342, 368, 384, 386, 427, 627 und 815 erwiesen sich dabei als sehr wirksam und als den Vergleichsmitteln A und D weit überlegen.

Beispiel II

Wirksamkeit gegen Phytophthora infestans an Tomaten

Blätter von Topfpflanzen der Sorte "Große Fleischtomate" wurden mit wäßriger Spritzbrühe, die 80% Wirkstoff und 20% Emulgiermittel in der Trockensubstanz enthielt, besprüht. Nach 24 Stunden wurden die Blätter mit einer Zoosporenaufschwemmung des Pilzes Phytophthore infestans infiziert. Die Pflanzen wurden dann in einer wasserdampfgesättigten Kammer bei Temperaturen zwischen 16 und 18°C aufgestellt. Nach 6 Tagen hatte sich die Krankheit auf den unbehandelten, jedoch infizierten Kontrollpflanzen so stark entwickelt, daß die fungizide Wirksamkeit der Substanzen beurteilt werden konnte.

Die Verbindungen der Beispiele 1, 2, 85, 256, 300, 301, 368, 384, 427, 469, 471 und 818 zeigten dabei eine ausgezeichnete Wirksamkeit, und zwar eine wesentlich bessere als die Vergleichsmittel A und B.

Beispiel III

Wirksamkeit gegen Pyrenophora teres

Gerstenkeimlinge der Sorte "Asse" wurden im Zweiblattstadium ait wäßrigen Suspensionen, die 80% Wirkstoff und 20% Emulgator in der Trockensubstanz enthielt, tropfnaß gespritzt. Nach 24 Stunden wurden die Pflanzen mit einer Sporensuspension des Pilzes Pyrenophora teres inoculiert und für 48 Stunden in eine Klimakammer mit hoher Luftfeuchtigkeit bei 18°C gestellt. Anschließend wurden die Pflanzen im Gewächshaus bei 20 bis 22°C und 70% relativer Luftfeuchtigkeit für weitere 5 Tage kultiviert. Dann wurde das Ausmaß der Symptomentwicklung ermittelt.

Die Verbindungen der Beispiele 85, 300, 386, 427, 973 und 1256 erwiesen sich hierbei nicht nur als hochwirksam, sondern waren auch dem Vergleichsmittel B deutlich überlegen.

Beispiel IV

Fungizide Wirksamkeit gegenüber Aspergillus niger

Die Wirkstoffe wurden einer für das Wachstum des Pilzes Aspergillus niger optimal geeigneten Nährlösung in Mengen von 100, 50, 25, 10, 5 und 1 Gewichtsteilen pro Million Teile Nährlösung zugesetzt. Es wurden jeweils 20 ml der so behandelten Nährlösung in 100 ml Glaskolben mit 0,3 mg Aspergillus-Pilzsporen beimpft. Die Kolben wurden 120 Stunden lang auf 36°C erwärmt und anschließend das Ausmaß der Pilzentwicklung, das bevorzugt auf der Nährlösungsoberfläche erfolgt, beurteilt.

Hierbei zeigten die Verbindungen der Beispiele 1, 2, 24, 43, 127, 196, 212, 214, 215, 298, 300, 342, 384, 427, 469, 471, 729, 731 und 732 eine hervorragende Wirkung. Sie war wesentlich besser als die des Vergleichsmittels C.

Beispiel V

Fungizide Wirksamkeit gegenüber Aureobasidium pullulans

Zur Prüfung der Wirksamkeit gegenüber Aureobasidium pullulans wurden die Wirkstoffe einer für das Wachstum des Pilzes optimalen Nährlösung in Mengen von 100, 50, 25, 12, 6, 3 und 1,5 Gewichtsteilen pro million Teile Nährlösung zugesetzt. Jeweils 10 ml Nährlösungs-Wirkstoffgemisch wurden in sterile Reagenzgläser gegeben und mit einem Tropfen einer Sporensuspension beimpft, die $10^6$ Konidien bzw. Zellen enthielt. Nach 120-stündiger Bebrütung wurden aus denjenigen Röhren, die kein sichtbares Pilzwachstum zeigten, Proben entnommen und auf Pilznährböden übertragen. In dieser Weise wurden die Wirkstoffmengen ermittelt, bei welcher nach dem Übertragen einer Probe auf den Nährboden kein Wachstum der Pilze mehr erfolgte.

Hierbei erwiesen sich die Verbindungen der Beispiele 24, 43, 74, 127, 196 und 732 als gut bis sehr gut, die der Beispiele 212, 214, 215, 298, 300, 342, 427, 729 und 731 als hervorragend wirksam.

Beispiel VI

Bakterizide Wirksamkeit gegenüber Staphylococcus aureus und Escherichia coli

Die Abtötungswerte gegenüber Bakterien wurden wie folgt ermittelt :

Zu je 5 ml einer Verdünnung der mittel in Wasser wurden 5 ml doppeltkonzentrierter Nährbouillon in sterilen Reagenzgläsern gegeben und vermischt. Durch Zugabe von einem Tropfen 1 : 10 verdünnter 16 Stunden alter Bouillon-Kulturen der Bakterienarten Staphylococcus aureus bzw. Escherichia coli wurden dann die Röhren beimpft und 24 Stunden lang bei 37°C bebrütet. Nach dieser Zeit wurden Proben aus den Röhren auf Bakteriennährböden übertragen und diese ebenfalls 24 Stunden lang bei 37°C bebrütet. Diejenige Verdünnungsstufe, bei welcher nach dem Übertragen einer Probe auf den Nährboden keine Bakterienentwicklung mehr erfolgte, wurde als Abtötungswert angesehen. Die Verbindungen der Beispiele 2, 298, 300 und 427 erwiesen sich dabei als hochwirksam.

## Ansprüche

1. Chinolinderivate der Formel I

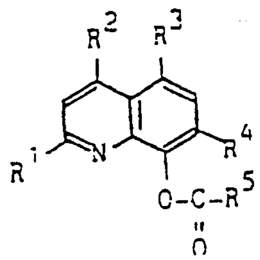

I,

in der

$R^1$ Wasserstoff oder Methyl,
$R^2$ Wasserstoff oder Halogen,
$R^3$ Wasserstoff, Halogen oder Nitro,
$R^4$ Wasserstoff, Halogen oder Nitro und
$R^5$ einen gegebenenfalls durch einen $C_1$-$C_4$-Alkylrest, einen $C_1$-$C_4$-Halogenalkylrest, eine Nitrogruppe oder ein Halogenatom einfach oder unabhängig voneinander zweifach substituierten Thiophen-, Pyrrol-, Oxazol-, Thiazol-, Imidazol-, Isoxazol-, Isothiazol-, Pyrazol-, 1,2,3-, 1,2,4-, 1,2,5- oder 1,3,4-Thiadiazol-, 1,2,3-, 1,2,4-, 1,2,5- oder 1,3,4-Oxadiazol-, 1,2,3- oder 1,2,4-Triazol- oder einen halogen- oder mono- oder dimethyl-substituierten Furan-Rest bedeuten, mit der Maßgabe, daß
$R^3$ und $R^4$ nicht gleichzeitig für Wasserstoff oder Halogen stehen und das 5-Chlor-, das 2-Methyl- und das 5-Nitro-8-(1′,2′,3′-thiadiazol-4′-carbonyloxy)-chinolin, das 5-Chlor-8-(4′-methyl-1′,2′,3′-thiadiazol-5′-carbonyl-oxy)-chinolin, das 7-Nitro-8-(alpha-thienoyl-oxy)-chinolin, das 5-Nitro-8-(2′-halogenfuroyl-5′-oxy)-chinolin und das 2-Methyl-8-(isoxazol-5′-carbonyl-oxy)-chinolin ausgeschlossen bleiben.

2. Chinolinderivate der Formel I gemäß Anspruch 1, in der $R^1$ Wasserstoff bedeutet, $R^2$, $R^3$ und $R^4$ die im Anspruch 1 angegebene Bedeutung haben und $R^5$ einen gegebenenfalls durch Chlormethyl substituierten Isoxazol-(3)- oder (5)-Rest, einen gegebenenfalls durch Methyl substituierten Oxazol(5)-Rest, einen Imidazol-(1)-Rest oder einen gegebenenfalls durch Methyl substituierten 1,2,3-Thiadiazol-(4)- oder -(5)-Rest bedeutet.

3. Chinolinderivate der Formel I nach Anspruch 1, in der $R^1$ und $R^2$ Wasserstoff, $R^3$ und $R^4$ unabhängig voneinander Wasserstoff, Halogen oder die Nitrogruppe bedeuten und $R^5$ die in Anspruch 1 genannten Bedeutungen hat, mit der Maßgabe, daß $R^3$ und $R^4$ gleichzeitig weder Wasserstoff noch Halogen bedeuten.

4. Chinolinderivate der Formel I nach Anspruch 1, in der $R^1$ und $R^2$ Wasserstoff, $R^3$ und $R^4$ unabhängig voneinander Wasserstoff, Halogen oder die Nitrogruppe bedeuten, wobei $R^5$ die in Anspruch 2 genannten Bedeutungen hat.

5. Chinolinderivate der Formel I nach Anspruch 3, wobei $R^4$ Chlor, Brom oder die Nitrogruppe bedeutet.

6. Chinolinderivate der Formel I nach Anspruch 4, wobei $R^4$ Chlor, Brom oder die Nitrogruppe bedeutet.

7. Mikrobizides Mittel enthaltend einen Trägerstoff und ein Chinolinderivat der Formel I gemäß Anspruch 1.

8. Verfahren zur Bekämpfung von Bakterien oder Pilzen, dadurch gekennzeichnet, daß man ein Chinolinderivat der Formel I gemäß Anspruch 1 auf Bakterien oder Pilze oder auf durch Bakterien- oder Pilzbefall bedrohte Materialien, Boden, Pflanzen oder Saatgüter einwirken läßt.

9. Verfahren zur Herstellung der Verbindungen der Formel I gemäß Anspruch 1, dadurch gekennzeichnet, daß man eine Verbindung der Formel II

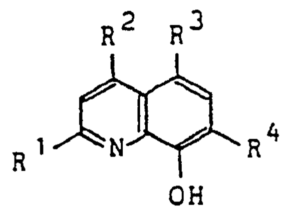

II,

in der $R^1$, $R^2$, $R^3$ und $R^4$ die im Anspruch 1 angegebene Bedeutung haben oder eines ihrer Alkalimetall- oder Erdalkalimetallsalze mit einem Carbonsäurederivat der Formel III

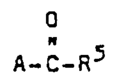

III,

in der
$R^5$ die im Anspruch 1 angegebene Bedeutung hat und
A für eine nucleophil verdrängbare Abgangsgruppe steht, umsetzt.

## Claims

1. A quinoline derivative of the formula

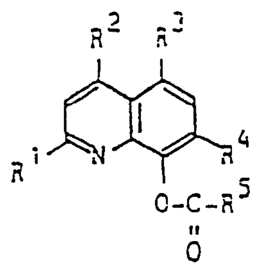

I,

where $R^1$ is hydrogen or methyl, $R^2$ is hydrogen or halogen, $R^3$ is hydrogen, halogen or nitro, $R^4$ is hydrogen, halogen or nitro, and $R^5$ is a thiophene, pyrrole, oxazole, thiazole, imidazole, isoxazole, isothiazole, pyrazole, 1,2,3-, 1,2,4-, 1,2,5- or 1,3,4-thiadiazole, 1,2,3-, 1,2,4-, 1,2,5- or 1,3,4-oxadiazole or 1,2,3- or 1,2,4-triazole radical which is unsubstituted or monosubstituted or disubstituted by $C_1$-$C_4$-alkyl, $C_1$-$C_4$-haloalkyl, nitro or halogen, the substituents being independent of one another in the case of disubstitution, or is furan which is substituted by halogen or monosubstituted or disubstituted by methyl, with the proviso that $R^3$ and $R^4$ are not simultaneously hydrogen or halogen, and that 5-chloro-, 2-methyl- and 5-nitro-8-(1′,2′,3′,-thiadiazole-4′-carbonyloxy)-quinoline, 5-chloro-8-(4′-methyl-1′,2′,3′-thiadiazole-5′-cabonyloxy)-quinoline, 7-nitro-8-(α-thienoyloxy)-quinoline, 5-nitro-8-(2′-halofuroyl-5′-oxy)-quinoline and 2-methyl-8-(isoxazole-5′-carbonyloxy)-quinoline are excluded.

2. A quinoline derivative of the formula I as claimed in claim 1, where $R^1$ is hydrogen, $R^2$, $R^3$ and $R^4$ have the meanings given in claim 1, and $R^5$ is an unsubstitiuted or chloromethyl-substitued isoxazol-3-yl or isoxazol-5-yl radical, an unsubstituted or methyl-substituted oxazol-5-yl radical, an imidazol-1-yl radical or an unsubstituted or methyl-substituted 1,2,3-thiadiazol-4-yl or 1,2,3-thiadiazol-5-yl radical.

3. A quinoline derivative of the formula I as claimed in claim 1, where $R^1$ and $R^2$ are hydrogen, $R^3$ and $R^4$ are independent of each other and are hydrogen, halogen or nitro, and $R^5$ has the meanings given in claim 1, with the proviso that $R^3$ and $R^4$ are simultaneously neither hydrogen nor halogen.

4. A quinoline derivative of the formula I as claimed in claim 1, where $R^1$ and $R^2$ are hydrogen, $R^3$ and $R^4$ are independent of each other and are hydrogen, halogen or nitro, and $R^5$ has the meanings given in claim 2.

5. A quinoline derivative of the formula I as claimed in claim 3, where $R^4$ is chlorine, bromine or nitro.

6. A quinoline derivative of the formula I as claimed in claim 4, where $R^4$ is chlorine, bromine or nitro.

7. A microbicidal agent containing a carrier and a quinoline derivative of the formula I as claimed in claim 1.

8. A process for combating bacteria or fungi, wherein a quinoline derivative of the formula I as claimed in claim 1 is allowed to act on bacteria or fungi, or on materials, soil, plants or seed threatened by bacterial or

fungal attack.

9. A process for preparing a compound of the formula I as claimed in claim 1, which comprises reacting a compound of the formula II

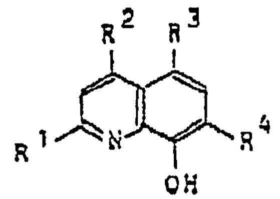

II,

where $R^1$, $R^2$, $R^3$ and $R^4$ have the meanings given in claim 1, or one of its alkate metal or alkaline earth metal with a carboxylic and derivative of the formula III

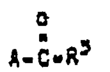

III,

where $R^5$ has the meaning given in claim 1, and A is a nucleophilically displaceable leaving group.

**Revendications**

1. Dérivés de quinoléine de formule I

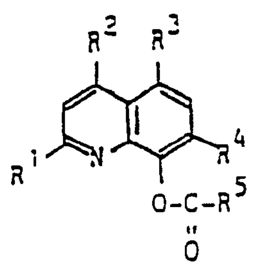

I,

dans laquelle

$R^1$ représente hydrogène ou méthyle,

$R^2$, hydrogène ou halogène,

$R^3$, hydrogène, halogène ou nitro,

$R^4$, hydrogène, halogène ou nitro et

$R^5$, un reste thiophène, pyrrole, oxazole, thiazole, imidazole, isoxazole, isothiazole, pyrazole, 1,2,3-, 1,2,4-, 1,2,5- ou 1,3,4-thiadiazole, 1,2,3-, 1,2,4-, 1,2,5- ou 1,3,4-oxadiazole, 1,2,3- ou 1,2,4-triazole, portant éventuellement un substituant ou deux substituants, indépendants l'un de l'autre, à savoir reste alkyle en $C_1$-$C_4$, halogéno-alkyle en $C_1$-$C_4$, groupe nitro ou atome d'halogène, ou un reste furanne substitué par halogène ou mono- ou diméthyle,

étant entendu que

$R^3$ et $R^4$ ne sont pas mis simultanément pour hydrogène ou halogène et que 5-chloro, 2-méthyl- et 5-nitro-8-(1′,2′,3′-thiadiazol-4′-carbonyloxy)-quinoléine, 5-chloro-8-(4′-méthyl-1′,2′,3′-thiadiazol-5′-carbonyloxy)-quinoléine, 7-nitro-8-(alpha-thiénoyl-oxy)-quinoléine, 5-nitro-8-(2′-halogénofuroyl-5′-oxy)-quinoléine et 2-méthyl-8-(isoxazol-5′-carbonyl-oxy)-quinoléine sont exclues.

2. Dérivés de quinoléine de formule I, selon la revendication 1, dans laquelle $R^1$ représente hydrogène, $R^2$, $R^3$, $R^4$ ont les significations données dans la revendication 1 et $R^5$ représente un reste isoxazole-(3) ou -(5), éventuellement substitué par chlorométhyle, un reste oxazole-(5) éventuellement substitué par méthyle, un reste imidazole-(1) ou un reste 1,2,3-thiadiazole-(4) ou -(5), éventuellement substitué par méthyle.

3. Dérivés de quinoléine de formule I, selon la revendication 1, dans laquelle $R^1$ et $R^2$ représentent hydrogène, $R^3$ et $R^4$, indépendamment l'un et l'autre, hydrogène, halogène ou le groupe nitro, $R^5$ ayant les significations données dans la revendication 1, étant entendu que $R^3$ et $R^4$ ne représentent pas simultanément

hydrogène ni halogène.

4. Dérivés de quinoléine de formule I, selon la revendication 1, dans laquelle $R^1$ et $R^2$ représentent, hydrogène, $R^3$ et $R^4$, indépendamment l'un de l'autre, hydrogène, halogène ou le groupe nitro, $R^5$ ayant les significations données dans la revendication 2.

5. Dérivés de quinoléine de formule I, selon la revendication 3, dans laquelle $R^4$ représente chlore, brome ou le groupe nitro.

6. Dérivés de quinoléine de formule I, selon la revendication 4, dans laquelle $R^4$ représente chlore, brome ou le groupe nitro.

7. Agent microbicide, contenant un véhicule et un dérivé de quinoléine de formule I, selon la revendication 1.

8. Procédé de lutte contre les bactéries ou les champignons, caractérisé par le fait que l'on fait agir un dérivé de quinoléine de formule I, selon la revendication 1, sur les bactéries ou champignons ou sur le sol, les plantes, les semences ou les matériaux menacés par l'attaque par les bactéries ou les champignons.

9. Procédé de préparation des composés de la formule I selon la revendication 1, caractérisé par le fait que l'on fait réagir un composé de la formule II

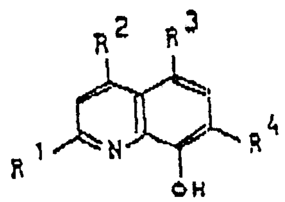

II,

dans laquelle $R^1$, $R^2$, $R^3$ et $R^4$ ont la signification donnée dans la revendication 1, ou un de leurs sels de métal alcalin ou de métal alcalinoterreux, avec un dérivé d'acide carboxylique de la formule III

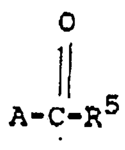

III,

dans laquelle
$R^5$ a la signification donnée dans la revendication 1 et
A est mis pour groupe éliminable nucléophile.